(19) Europäisches Patentamt
European Patent Office
Office européen des brevets

(11) EP 4 763 866 A1

(12) **EUROPEAN PATENT APPLICATION**

(43) Date of publication:
**24.06.2026 Bulletin 2026/26**

(21) Application number: **25222544.6**

(22) Date of filing: **25.11.2020**

(51) International Patent Classification (IPC):
***C07K 16/46*** *(2006.01)* ***C12N 15/13*** *(2006.01)*
***A61K 39/395*** *(2006.01)* ***A61P 35/00*** *(2006.01)*
***C07K 16/22*** *(2006.01)* ***A61P 35/02*** *(2006.01)*
***C07K 16/28*** *(2006.01)*

(52) Cooperative Patent Classification (CPC):
**A61P 35/00; A61P 35/02; C07K 16/22; C07K 16/2818; G01N 33/57557;** C07K 2317/24; C07K 2317/31; C07K 2317/52; C07K 2317/524; C07K 2317/732; C07K 2317/734; C07K 2317/92; G01N 2333/475; G01N 2333/70532; G01N 2800/7014

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.11.2019 CN 201911164156**

(62) Document number(s) of the earlier application(s) in accordance with Art. 76 EPC:
**20892544.6 / 4 067 387**

(71) Applicant: **Akeso Biopharma Co., Ltd.**
**Zhongshan, Guangdong 528437 (CN)**

(72) Inventors:
- **ZHANG, Peng**
  **Zhongshan, 528437 (CN)**
- **LI, Baiyong**
  **Zhongshan, 528437 (CN)**
- **XIA, Yu**
  **Zhongshan, 528437 (CN)**
- **WANG, Zhongmin**
  **Zhongshan, 528437 (CN)**

(74) Representative: **Gill Jennings & Every LLP**
**The Broadgate Tower**
**20 Primrose Street**
**London EC2A 2ES (GB)**

Remarks:
•The complete document including Reference Table(s) and the Sequence Listing(s) can be downloaded from the EPO website
•This application was filed on 11-12-2025 as a divisional application to the application mentioned under INID code 62.
•Claims filed after the date of filing of the application / after the date of receipt of the divisional application (Rule 68(4) EPC).

# (54) ANTI-PD-1-ANTI-VEGFA BISPECIFIC ANTIBODY, PHARMACEUTICAL COMPOSITION AND USE THEREOF

(57) Provided are an anti-VEGFA-anti-PD-1 bispecific antibody and a use thereof. Specifically, the anti-VEGFA-anti-PD-1 bispecific antibody comprises: a PD-1-targeted first protein functional region and a VEGFA-targeted second protein functional region. According to an EU numbering system, mutation occurs at two positions of positions 234 and 235 of a heavy chain constant region of the immunoglobulin contained in the bispecific antibody, and after the mutation, an affinity constant of the bispecific antibody with FcγRI, FcγRIIa, FcγRIIIa, and/or Clq is decreased compared to that before the mutation. The bispecific antibody can specifically bind to VEGFA and PD-1, specifically relieve immunosuppression of VEGFA and PD-1 on an organism, and inhibit tumor-induced angiogenesis, and thus has good application prospects.

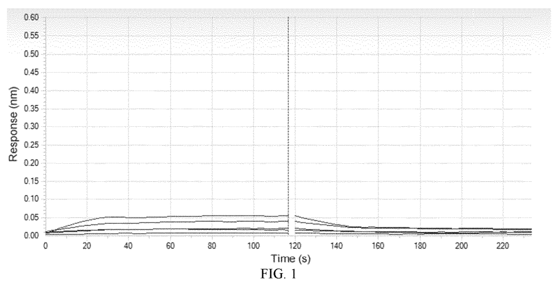

FIG. 1

EP 4 763 866 A1

SEQUENCE LISTING

<110> AKESO BIOPHARMA, INC

<120> ANTI-PD-1-ANTI-VEGFA BISPECIFIC ANTIBODY, PHARMACEUTICAL COMPOSITION AND USE THEREOF

<130> RMC00405EP1

<150> 20892544.6
<151> 2020-11-25
<150> CN201911164156.2
<151> 2019-11-25

<160> 42

<170> PatentIn version 3.5

<210> 1
<211> 123
<212> PRT
<213> Artificial Sequence

<220>
<223> Bevacizumab-Hv

<400> 1

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Phe Thr Asn Tyr
            20                  25                  30

Gly Met Asn Trp Val Arg Gln Ala Pro Gly Lys Gly Leu Glu Trp Val
        35                  40                  45

Gly Trp Ile Asn Thr Tyr Thr Gly Glu Pro Thr Tyr Ala Ala Asp Phe
    50                  55                  60

Lys Arg Arg Phe Thr Phe Ser Leu Asp Thr Ser Lys Ser Thr Ala Tyr
65                  70                  75                  80

Leu Gln Met Asn Ser Leu Arg Ala Glu Asp Thr Ala Val Tyr Tyr Cys
                85                  90                  95
```

Ala Lys Tyr Pro His Tyr Tyr Gly Ser Ser His Trp Tyr Phe Asp Val
100 105 110

Trp Gly Gln Gly Thr Leu Val Thr Val Ser Ser
115 120

<210> 2
<211> 369
<212> DNA
<213> Artificial Sequence

<220>
<223> nucleic acid encoding Bevacizumab-Hv

<400> 2
gaggtgcagc tggtcgagtc cggggggggg ctggtgcagc caggcgggtc tctgaggctg 60

agttgcgccg cttcagggta caccttcaca aactatggaa tgaattgggt gcgccaggca 120

ccaggaaagg gactggagtg ggtcggctgg atcaacactt acaccgggga acctacctat 180

gcagccgact ttaagcggcg gttcaccttc agcctggata caagcaaatc cactgcctac 240

ctgcagatga acagcctgcg agctgaggac accgcagtct actattgtgc taaatatccc 300

cactactatg ggagcagcca ttggtatttt gacgtgtggg ggcaggggac tctggtgaca 360

gtgagcagc 369

<210> 3
<211> 107
<212> PRT
<213> Artificial Sequence

<220>
<223> Bevacizumab-Lv

<400> 3

Asp Ile Gln Met Thr Gln Ser Pro Ser Ser Leu Ser Ala Ser Val Gly
1 5 10 15

Asp Arg Val Thr Ile Thr Cys Ser Ala Ser Gln Asp Ile Ser Asn Tyr
20 25 30

```
Leu Asn Trp Tyr Gln Gln Lys Pro Gly Lys Ala Pro Lys Val Leu Ile
        35                  40                  45

Tyr Phe Thr Ser Ser Leu His Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Val Pro Trp
                85                  90                  95

Thr Phe Gly Gln Gly Thr Lys Val Glu Ile Lys
            100                 105


<210>  4
<211>  321
<212>  DNA
<213>  Artificial Sequence

<220>
<223>  nucleic acid encoding Bevacizumab-Lv

<400>  4
gatattcaga tgactcagag cccctcctcc ctgtccgcct ctgtgggcga cagggtcacc       60

atcacatgca gtgcttcaca ggatatttcc aactacctga attggtatca gcagaagcca      120

ggaaaagcac ccaaggtgct gatctacttc actagctccc tgcactcagg agtgccaagc      180

cggttcagcg gatccggatc tggaaccgac tttactctga ccatttctag tctgcagcct      240

gaggatttcg ctacatacta ttgccagcag tattctaccg tgccatggac atttggccag      300

gggactaaag tcgagatcaa g                                                321


<210>  5
<211>  118
<212>  PRT
<213>  Artificial Sequence

<220>
<223>  VH of 14C12

<400>  5
```

```
Glu Val Lys Leu Val Glu Ser Gly Gly Gly Leu Val Lys Pro Gly Gly
1               5                   10                  15

Ser Leu Lys Leu Ser Cys Ala Ala Ser Gly Phe Ala Phe Ser Ser Tyr
            20                  25                  30

Asp Met Ser Trp Val Arg Gln Thr Pro Glu Lys Arg Leu Glu Trp Val
        35                  40                  45

Ala Thr Ile Ser Gly Gly Gly Arg Tyr Thr Tyr Tyr Pro Asp Ser Val
    50                  55                  60

Lys Gly Arg Phe Thr Ile Ser Arg Asp Asn Ala Arg Asn Thr Leu Tyr
65                  70                  75                  80

Leu Gln Met Ser Ser Leu Arg Ser Glu Asp Thr Ala Leu Tyr Tyr Cys
                85                  90                  95

Ala Asn Arg Tyr Gly Glu Ala Trp Phe Ala Tyr Trp Gly Gln Gly Thr
            100                 105                 110

Leu Val Thr Val Ser Ala
        115
```

<210> 6
<211> 354
<212> DNA
<213> Artificial Sequence

<220>
<223> nucleic acid encoding VH of 14C12

<400> 6

```
gaggtcaaac tggtggagag cggcggcggg ctggtgaagc ccggcgggtc actgaaactg        60

agctgcgccg cttccggctt cgcctttagc tcctacgaca tgtcatgggt gaggcagacc       120

cctgagaagc gcctggaatg ggtcgctact atcagcggag gcgggcgata cacctactat       180

cctgactctg tcaaagggag attcacaatt agtcgggata acgccagaaa tactctgtat       240

ctgcagatgt ctagtctgcg gtccgaggat acagctctgt actattgtgc aaaccggtac       300
```

```
ggcgaagcat ggtttgccta ttggggacag ggcaccctgg tgacagtctc tgcc          354


<210>  7
<211>  107
<212>  PRT
<213>  Artificial Sequence

<220>
<223>  VL of 14C12

<400>  7

Asp Ile Lys Met Thr Gln Ser Pro Ser Ser Met Tyr Ala Ser Leu Gly
1               5                   10                  15


Glu Arg Val Thr Phe Thr Cys Lys Ala Ser Gln Asp Ile Asn Thr Tyr
            20                  25                  30


Leu Ser Trp Phe Gln Gln Lys Pro Gly Lys Ser Pro Lys Thr Leu Ile
        35                  40                  45


Tyr Arg Ala Asn Arg Leu Val Asp Gly Val Pro Ser Arg Phe Ser Gly
    50                  55                  60


Ser Gly Ser Gly Gln Asp Tyr Ser Leu Thr Ile Ser Ser Leu Glu Tyr
65                  70                  75                  80


Glu Asp Met Gly Ile Tyr Tyr Cys Leu Gln Tyr Asp Glu Phe Pro Leu
                85                  90                  95


Thr Phe Gly Ala Gly Thr Lys Leu Glu Leu Lys
            100                 105


<210>  8
<211>  321
<212>  DNA
<213>  Artificial Sequence

<220>
<223>  nucleic acid encoding VL of 14C12

<400>  8
```

```
gacattaaga tgacacagtc cccttcctca atgtacgcta gcctgggcga gcgagtgacc        60

ttcacatgca aagcatccca ggacatcaac acatacctgt cttggtttca gcagaagcca       120

ggcaaaagcc ccaagaccct gatctaccgg gccaatagac tggtggacgg ggtccccagc       180

agattctccg gatctggcag tgggcaggat tactccctga ccatcagctc cctggagtat       240

gaagacatgg gcatctacta ttgcctgcag tatgatgagt tccctctgac ctttggagca       300

ggcacaaaac tggaactgaa g                                                 321


<210>  9
<211>  118
<212>  PRT
<213>  Artificial Sequence

<220>
<223>  VH of 14C12H1L1

<400>  9

Glu Val Gln Leu Val Glu Ser Gly Gly Gly Leu Val Gln Pro Gly Gly
1               5                   10                  15


Ser Leu Arg Leu Ser Cys Ala Ala Ser Gly Phe Ala Phe Ser Ser Tyr
            20                  25                  30


Asp Met Ser Trp Val Arg Gln Ala Pro Gly Lys Gly Leu Asp Trp Val
        35                  40                  45


Ala Thr Ile Ser Gly Gly Gly Arg Tyr Thr Tyr Tyr Pro Asp Ser Val
    50                  55                  60


Lys Gly Arg Phe Thr Ile Ser Arg Asp Asn Ser Lys Asn Asn Leu Tyr
65                  70                  75                  80


Leu Gln Met Asn Ser Leu Arg Ala Glu Asp Thr Ala Leu Tyr Tyr Cys
                85                  90                  95


Ala Asn Arg Tyr Gly Glu Ala Trp Phe Ala Tyr Trp Gly Gln Gly Thr
            100                 105                 110
```

Leu Val Thr Val Ser Ser
        115

<210>  10
<211>  354
<212>  DNA
<213>  Artificial Sequence

<220>
<223>  nucleic acid encoding VH of 14C12H1L1

<400>  10
gaagtgcagc tggtcgagtc tgggggaggg ctggtgcagc ccggcgggtc actgcgactg    60

agctgcgcag cttccggatt cgcctttagc tcctacgaca tgtcctgggt gcgacaggca    120

ccaggaaagg gactggattg ggtcgctact atctcaggag gcgggagata cacctactat    180

cctgacagcg tcaagggccg gttcacaatc tctagagata acagtaagaa caatctgtat    240

ctgcagatga acagcctgag ggctgaggac accgcactgt actattgtgc caaccgctac    300

ggggaagcat ggtttgccta ttgggggcag ggaaccctgg tgacagtctc tagt    354

<210>  11
<211>  107
<212>  PRT
<213>  Artificial Sequence

<220>
<223>  VL of 14C12H1L1

<400>  11

Asp Ile Gln Met Thr Gln Ser Pro Ser Ser Met Ser Ala Ser Val Gly
1               5                   10                  15

Asp Arg Val Thr Phe Thr Cys Arg Ala Ser Gln Asp Ile Asn Thr Tyr
            20                  25                  30

Leu Ser Trp Phe Gln Gln Lys Pro Gly Lys Ser Pro Lys Thr Leu Ile
        35                  40                  45

Tyr Arg Ala Asn Arg Leu Val Ser Gly Val Pro Ser Arg Phe Ser Gly
    50                  55                  60

Ser Gly Ser Gly Gln Asp Tyr Thr Leu Thr Ile Ser Ser Leu Gln Pro
65 70 75 80

Glu Asp Met Ala Thr Tyr Tyr Cys Leu Gln Tyr Asp Glu Phe Pro Leu
85 90 95

Thr Phe Gly Ala Gly Thr Lys Leu Glu Leu Lys
100 105

<210> 12
<211> 321
<212> DNA
<213> Artificial Sequence

<220>
<223> nucleic acid encoding VL of 14C12H1L1

<400> 12
gacattcaga tgactcagag cccctcctcc atgtccgcct ctgtgggcga cagggtcacc 60

ttcacatgcc gcgctagtca ggatatcaac acctacctga gctggtttca gcagaagcca 120

gggaaaagcc ccaagacact gatctaccgg gctaatagac tggtgtctgg agtcccaagt 180

cggttcagtg gctcagggag cggacaggac tacactctga ccatcagctc cctgcagcct 240

gaggacatgg caacctacta ttgcctgcag tatgatgagt tcccactgac ctttggcgcc 300

gggacaaaac tggagctgaa g 321

<210> 13
<211> 448
<212> PRT
<213> Artificial Sequence

<220>
<223> heavy chain of 14C12H1L1

<400> 13

Glu Val Gln Leu Val Glu Ser Gly Gly Gly Leu Val Gln Pro Gly Gly
1 5 10 15

Ser Leu Arg Leu Ser Cys Ala Ala Ser Gly Phe Ala Phe Ser Ser Tyr
20 25 30

```
Asp Met Ser Trp Val Arg Gln Ala Pro Gly Lys Gly Leu Asp Trp Val
        35                  40                  45

Ala Thr Ile Ser Gly Gly Gly Arg Tyr Thr Tyr Tyr Pro Asp Ser Val
    50                  55                  60

Lys Gly Arg Phe Thr Ile Ser Arg Asp Asn Ser Lys Asn Asn Leu Tyr
65                  70                  75                  80

Leu Gln Met Asn Ser Leu Arg Ala Glu Asp Thr Ala Leu Tyr Tyr Cys
                85                  90                  95

Ala Asn Arg Tyr Gly Glu Ala Trp Phe Ala Tyr Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Glu Leu Leu Gly Gly Pro Ser
```

```
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Asp Glu Leu Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430
```

Leu His Asn His Tyr Thr Gln Lys Ser Leu Ser Leu Ser Pro Gly Lys
435 440 445

<210> 14
<211> 1344
<212> DNA
<213> Artificial Sequence

<220>
<223> nucleic acid encoding heavy chain of 14C12H1L1

<400> 14
gaagtgcagc tggtcgagtc tgggggaggg ctggtgcagc ccggcgggtc actgcgactg 60

agctgcgcag cttccggatt cgcctttagc tcctacgaca tgtcctgggt gcgacaggca 120

ccaggaaagg gactggattg ggtcgctact atctcaggag gcgggagata cacctactat 180

cctgacagcg tcaagggccg gttcacaatc tctagagata acagtaagaa caatctgtat 240

ctgcagatga acagcctgag ggctgaggac accgcactgt actattgtgc caaccgctac 300

ggggaagcat ggtttgccta ttgggggcag ggaaccctgg tgacagtctc tagtgccagc 360

accaaaggac ctagcgtgtt tcctctcgcc ccctcctcca aaagcaccag cggaggaacc 420

gctgctctcg gatgtctggt gaaggactac ttccctgaac ccgtcaccgt gagctggaat 480

agcggcgctc tgacaagcgg agtccataca ttccctgctg tgctgcaaag cagcggactc 540

tattccctgt ccagcgtcgt cacagtgccc agcagcagcc tgggcaccca gacctacatc 600

tgtaacgtca accacaagcc ctccaacacc aaggtggaca agaaagtgga gcccaaatcc 660

tgcgacaaga cacacacctg tccccctgt cctgctcccg aactcctcgg aggccctagc 720

gtcttcctct ttcctcccaa acccaaggac accctcatga tcagcagaac ccctgaagtc 780

acctgtgtcg tcgtggatgt cagccatgag gaccccgagg tgaaattcaa ctggtatgtc 840

gatggcgtcg aggtgcacaa cgccaaaacc aagcccaggg aggaacagta caactccacc 900

tacagggtgg tgtccgtgct gacagtcctc caccaggact ggctgaacgg caaggagtac 960

aagtgcaagg tgtccaacaa ggctctccct gcccccattg agaagaccat cagcaaggcc 1020

aaaggccaac ccagggagcc ccaggtctat acactgcctc cctccaggga cgaactcacc 1080

aagaaccagg tgtccctgac ctgcctggtc aagggctttt atcccagcga catcgccgtc 1140

gagtgggagt ccaacggaca gcccgagaat aactacaaga ccacccctcc tgtcctcgac 1200

tccgacggct ccttcttcct gtacagcaag ctgaccgtgg acaaaagcag gtggcagcag 1260

ggaaacgtgt tctcctgcag cgtgatgcac gaagccctcc acaaccacta cacccagaaa 1320

agcctgtccc tgagccccgg caaa 1344

<210> 15
<211> 214
<212> PRT
<213> Artificial Sequence

<220>
<223> light chain of 14C12H1L1

<400> 15

```
Asp Ile Gln Met Thr Gln Ser Pro Ser Ser Met Ser Ala Ser Val Gly
1               5                   10                  15

Asp Arg Val Thr Phe Thr Cys Arg Ala Ser Gln Asp Ile Asn Thr Tyr
            20                  25                  30

Leu Ser Trp Phe Gln Gln Lys Pro Gly Lys Ser Pro Lys Thr Leu Ile
        35                  40                  45

Tyr Arg Ala Asn Arg Leu Val Ser Gly Val Pro Ser Arg Phe Ser Gly
    50                  55                  60

Ser Gly Ser Gly Gln Asp Tyr Thr Leu Thr Ile Ser Ser Leu Gln Pro
65                  70                  75                  80

Glu Asp Met Ala Thr Tyr Tyr Cys Leu Gln Tyr Asp Glu Phe Pro Leu
                85                  90                  95

Thr Phe Gly Ala Gly Thr Lys Leu Glu Leu Lys Arg Thr Val Ala Ala
            100                 105                 110

Pro Ser Val Phe Ile Phe Pro Pro Ser Asp Glu Gln Leu Lys Ser Gly
        115                 120                 125
```

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 16
<211> 642
<212> DNA
<213> Artificial Sequence

<220>
<223> lnucleic acid encoding ight chain of 14C12H1L1

<400> 16
gacattcaga tgactcagag cccctcctcc atgtccgcct ctgtgggcga cagggtcacc 60

ttcacatgcc gcgctagtca ggatatcaac acctacctga gctggtttca gcagaagcca 120

gggaaaagcc ccaagacact gatctaccgg gctaatagac tggtgtctgg agtcccaagt 180

cggttcagtg gctcagggag cggacaggac tacactctga ccatcagctc cctgcagcct 240

gaggacatgg caacctacta ttgcctgcag tatgatgagt tcccactgac ctttggcgcc 300

gggacaaaac tggagctgaa gcgaactgtg gccgctccct ccgtcttcat tttccccct 360

tctgacgaac agctgaaatc aggcacagcc agcgtggtct gtctgctgaa caatttctac 420

cctagagagg caaaagtgca gtggaaggtc gataacgccc tgcagtccgg caacagccag 480

gagagtgtga ctgaacagga ctcaaaagat agcacctatt ccctgtctag tacactgact 540

ctgtccaagg ctgattacga gaagcacaaa gtgtatgcat gcgaagtgac acatcaggga 600

ctgtcaagcc ccgtgactaa gtcttttaac cggggcgaat gt 642

<210> 17
<211> 108
<212> PRT
<213> Artificial Sequence

<220>
<223> light chain of 14C12H1L1(M)1

<400> 17

```
Asp Ile Gln Met Thr Gln Ser Pro Ser Ser Met Ser Ala Ser Val Gly
1               5                   10                  15

Asp Arg Val Thr Phe Thr Cys Arg Ala Ser Gln Asp Ile Asn Thr Tyr
            20                  25                  30

Leu Ser Trp Phe Gln Gln Lys Pro Gly Lys Ser Pro Lys Thr Leu Ile
        35                  40                  45

Tyr Arg Ala Asn Arg Leu Val Ser Gly Val Pro Ser Arg Phe Ser Gly
    50                  55                  60

Ser Gly Ser Gly Gln Asp Tyr Thr Leu Thr Ile Ser Ser Leu Gln Pro
65                  70                  75                  80

Glu Asp Met Ala Thr Tyr Tyr Cys Leu Gln Tyr Asp Glu Phe Pro Leu
                85                  90                  95

Thr Phe Gly Ala Gly Thr Lys Leu Glu Leu Lys Arg
            100                 105
```

<210> 18
<211> 20
<212> PRT
<213> Artificial Sequence

<220>
<223> Linker 1

<400> 18

Gly Gly Gly Gly Ser Gly Gly Gly Gly Ser Gly Gly Gly Gly Ser Gly
1 5 10 15

Gly Gly Gly Ser
20

<210> 19
<211> 15
<212> PRT
<213> Artificial Sequence

<220>
<223> linker 2

<400> 19

Gly Gly Gly Gly Ser Gly Gly Gly Gly Ser Gly Gly Gly Gly Ser
1 5 10 15

<210> 20
<211> 453
<212> PRT
<213> Artificial Sequence

<220>
<223> Bevacizumab-G4H

<400> 20

Glu Val Gln Leu Val Glu Ser Gly Gly Gly Leu Val Gln Pro Gly Gly
1 5 10 15

Ser Leu Arg Leu Ser Cys Ala Ala Ser Gly Tyr Thr Phe Thr Asn Tyr
20 25 30

Gly Met Asn Trp Val Arg Gln Ala Pro Gly Lys Gly Leu Glu Trp Val
35 40 45

Gly Trp Ile Asn Thr Tyr Thr Gly Glu Pro Thr Tyr Ala Ala Asp Phe
50 55 60

Lys Arg Arg Phe Thr Phe Ser Leu Asp Thr Ser Lys Ser Thr Ala Tyr

```
65                  70                  75                  80

Leu Gln Met Asn Ser Leu Arg Ala Glu Asp Thr Ala Val Tyr Tyr Cys
                85                  90                  95

Ala Lys Tyr Pro His Tyr Tyr Gly Ser Ser His Trp Tyr Phe Asp Val
            100                 105                 110

Trp Gly Gln Gly Thr Leu Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Lys Val Glu Pro Lys
    210                 215                 220

Ser Cys Asp Lys Thr His Thr Cys Pro Pro Cys Pro Ala Pro Glu Leu
225                 230                 235                 240

Leu Gly Gly Pro Ser Val Phe Leu Phe Pro Pro Lys Pro Lys Asp Thr
                245                 250                 255

Leu Met Ile Ser Arg Thr Pro Glu Val Thr Cys Val Val Val Asp Val
            260                 265                 270
```

```
Ser His Glu Asp Pro Glu Val Lys Phe Asn Trp Tyr Val Asp Gly Val
        275                 280                 285

Glu Val His Asn Ala Lys Thr Lys Pro Arg Glu Glu Gln Tyr Asn Ser
    290                 295                 300

Thr Tyr Arg Val Val Ser Val Leu Thr Val Leu His Gln Asp Trp Leu
305                 310                 315                 320

Asn Gly Lys Glu Tyr Lys Cys Lys Val Ser Asn Lys Ala Leu Pro Ala
                325                 330                 335

Pro Ile Glu Lys Thr Ile Ser Lys Ala Lys Gly Gln Pro Arg Glu Pro
            340                 345                 350

Gln Val Tyr Thr Leu Pro Pro Ser Arg Asp Glu Leu Thr Lys Asn Gln
        355                 360                 365

Val Ser Leu Thr Cys Leu Val Lys Gly Phe Tyr Pro Ser Asp Ile Ala
    370                 375                 380

Val Glu Trp Glu Ser Asn Gly Gln Pro Glu Asn Asn Tyr Lys Thr Thr
385                 390                 395                 400

Pro Pro Val Leu Asp Ser Asp Gly Ser Phe Phe Leu Tyr Ser Lys Leu
                405                 410                 415

Thr Val Asp Lys Ser Arg Trp Gln Gln Gly Asn Val Phe Ser Cys Ser
            420                 425                 430

Val Met His Glu Ala Leu His Asn His Tyr Thr Gln Lys Ser Leu Ser
        435                 440                 445

Leu Ser Pro Gly Lys
    450
```

<210> 21
<211> 1359
<212> DNA

<213>  Artificial Sequence

<220>
<223>  nucleic acid encoding Bevacizumab-G4H

<400>  21
gaggtgcagc tggtcgagtc cggggggggg ctggtgcagc caggcgggtc tctgaggctg      60

agttgcgccg cttcagggta caccttcaca aactatggaa tgaattgggt gcgccaggca     120

ccaggaaagg gactggagtg ggtcggctgg atcaacactt acaccgggga acctacctat     180

gcagccgact ttaagcggcg gttcaccttc agcctggata caagcaaatc cactgcctac     240

ctgcagatga acagcctgcg agctgaggac accgcagtct actattgtgc taaatatccc     300

cactactatg ggagcagcca ttggtatttt gacgtgtggg ggcaggggac tctggtgaca     360

gtgagcagcg caagcaccaa agggcccagc gtgtttcctc tcgcccctc ctccaaaagc     420

accagcggag gaaccgctgc tctcggatgt ctggtgaagg actacttccc tgaacccgtc     480

accgtgagct ggaatagcgg cgctctgaca agcggagtcc atacattccc tgctgtgctg     540

caaagcagcg gactctattc cctgtccagc gtcgtcacag tgcccagcag cagcctgggc     600

acccagacct acatctgtaa cgtcaaccac aagccctcca acaccaaggt ggacaagaaa     660

gtggagccca aatcctgcga caagacacac acctgtcccc cctgtcctgc tcccgaactc     720

ctcggaggcc ctagcgtctt cctctttcct cccaaaccca aggacaccct catgatcagc     780

agaacccctg aagtcacctg tgtcgtcgtg gatgtcagcc atgaggaccc cgaggtgaaa     840

ttcaactggt atgtcgatgg cgtcgaggtg cacaacgcca aaaccaagcc cagggaggaa     900

cagtacaact ccacctacag ggtggtgtcc gtgctgacag tcctccacca ggactggctg     960

aacggcaagg agtacaagtg caaggtgtcc aacaaggctc tccctgcccc cattgagaag    1020

accatcagca aggccaaagg ccaacccagg gagccccagg tctatacact gcctccctcc    1080

agggacgaac tcaccaagaa ccaggtgtcc ctgacctgcc tggtcaaggg cttttatccc    1140

agcgacatcg ccgtcgagtg ggagtccaac ggacagcccg agaataacta caagaccacc    1200

cctcctgtcc tcgactccga cggctccttc ttcctgtaca gcaaactgac cgtcgataaa    1260

tctaggtggc agcagggcaa cgtgttctct tgttccgtga tgcatgaagc actgcacaac    1320

cattataccc agaagtctct gagcctgtcc cccggcaag                           1359

<210> 22
<211> 450
<212> PRT
<213> Artificial Sequence

<220>
<223> heavy chain of VP101(hG4WT)

<400> 22

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Phe Thr Asn Tyr
            20                  25                  30

Gly Met Asn Trp Val Arg Gln Ala Pro Gly Lys Gly Leu Glu Trp Val
        35                  40                  45

Gly Trp Ile Asn Thr Tyr Thr Gly Glu Pro Thr Tyr Ala Ala Asp Phe
    50                  55                  60

Lys Arg Arg Phe Thr Phe Ser Leu Asp Thr Ser Lys Ser Thr Ala Tyr
65                  70                  75                  80

Leu Gln Met Asn Ser Leu Arg Ala Glu Asp Thr Ala Val Tyr Tyr Cys
                85                  90                  95

Ala Lys Tyr Pro His Tyr Tyr Gly Ser Ser His Trp Tyr Phe Asp Val
            100                 105                 110

Trp Gly Gln Gly Thr Leu Val Thr Val Ser Ser Ala Ser Thr Lys Gly
        115                 120                 125

Pro Ser Val Phe Pro Leu Ala Pro Cys Ser Arg Ser Thr Ser Glu Ser
    130                 135                 140

Thr Ala Ala Leu Gly Cys Leu Val Lys Asp Tyr Phe Pro Glu Pro Val
145                 150                 155                 160
```

```
Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Lys Thr Tyr Thr Cys Asn Val
        195                 200                 205

Asp His Lys Pro Ser Asn Thr Lys Val Asp Lys Arg Val Glu Ser Lys
    210                 215                 220

Tyr Gly Pro Pro Cys Pro Pro Cys Pro Ala Pro Glu Phe Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser Gln Glu
            260                 265                 270

Asp Pro Glu Val Gln Phe Asn Trp Tyr Val Asp Gly Val Glu Val His
        275                 280                 285

Asn Ala Lys Thr Lys Pro Arg Glu Glu Gln Phe Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Gly Leu Pro Ser Ser Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Gln Glu Glu Met Thr Lys Asn Gln Val Ser Leu
        355                 360                 365
```

```
Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Arg Leu Thr Val Asp
                405                 410                 415

Lys Ser Arg Trp Gln Glu Gly Asn Val Phe Ser Cys Ser Val Met His
            420                 425                 430

Glu Ala Leu His Asn His Tyr Thr Gln Lys Ser Leu Ser Leu Ser Leu
        435                 440                 445

Gly Lys
    450


<210>  23
<211>  1350
<212>  DNA
<213>  Artificial Sequence

<220>
<223>  nucleic acid encoding heavy chain of VP101(hG4WT)

<400>  23
gaggtgcagc tggtcgagtc cggggggggg ctggtgcagc caggcgggtc tctgaggctg      60

agttgcgccg cttcagggta caccttcaca aactatggaa tgaattgggt gcgccaggca     120

ccaggaaagg gactggagtg ggtcggctgg atcaacactt acaccgggga acctacctat     180

gcagccgact ttaagcggcg gttcaccttc agcctggata caagcaaatc cactgcctac     240

ctgcagatga acagcctgcg agctgaggac accgcagtct actattgtgc taaatatccc     300

cactactatg ggagcagcca ttggtatttt gacgtgtggg ggcaggggac tctggtgaca     360

gtgagcagcg caagcaccaa agggccctcg gtcttccccc tggcgccctg ctccaggagc     420

acctccgaga gcacagccgc cctgggctgc ctggtcaagg actacttccc cgaaccggtg     480
```

```
acggtgtcgt ggaactcagg cgccctgacc agcggcgtgc acaccttccc ggctgtccta      540

cagtcctcag gactctactc cctcagcagc gtggtgaccg tgccctccag cagcttgggc      600

acgaagacct acacctgcaa cgtagatcac aagcccagca acaccaaggt ggacaagaga      660

gttgagtcca aatatggtcc cccatgccca ccatgcccag cacctgagtt cctgggggga      720

ccatcagtct tcctgttccc cccaaaaccc aaggacactc tcatgatctc ccggacccct      780

gaggtcacgt gcgtggtggt ggacgtgagc caggaagacc ccgaggtcca gttcaactgg      840

tacgtggatg gcgtggaggt gcataatgcc aagacaaagc cgcgggagga gcagttcaac      900

agcacgtacc gtgtggtcag cgtcctcacc gtcctgcacc aggactggct gaacggcaag      960

gagtacaagt gcaaggtctc caacaaaggc ctcccgtcct ccatcgagaa aaccatctcc     1020

aaagccaaag ggcagccccg agagccacag gtgtacaccc tgcccccatc ccaggaggag     1080

atgaccaaga accaggtcag cctgacctgc ctggtcaaag gcttctaccc cagcgacatc     1140

gccgtggagt gggagagcaa tgggcagccg gagaacaact acaagaccac gcctcccgtg     1200

ctggactccg acggctcctt cttcctctac agcaggctaa ccgtggacaa gagcaggtgg     1260

caggagggga atgtcttctc atgctccgtg atgcatgagg ctctgcacaa ccactacaca     1320

cagaagtctc tgagcctgtc cctcggcaag                                      1350


<210>  24
<211>  453
<212>  PRT
<213>  Artificial Sequence

<220>
<223>  Immunoglobulin heavy  chain of VP101(hG1DM)

<400>  24

Glu Val Gln Leu Val Glu Ser Gly Gly Gly Leu Val Gln Pro Gly Gly
1               5                   10                  15


Ser Leu Arg Leu Ser Cys Ala Ala Ser Gly Tyr Thr Phe Thr Asn Tyr
            20                  25                  30


Gly Met Asn Trp Val Arg Gln Ala Pro Gly Lys Gly Leu Glu Trp Val
        35                  40                  45
```

```
Gly Trp Ile Asn Thr Tyr Thr Gly Glu Pro Thr Tyr Ala Ala Asp Phe
    50                  55                  60

Lys Arg Arg Phe Thr Phe Ser Leu Asp Thr Ser Lys Ser Thr Ala Tyr
65                  70                  75                  80

Leu Gln Met Asn Ser Leu Arg Ala Glu Asp Thr Ala Val Tyr Tyr Cys
                85                  90                  95

Ala Lys Tyr Pro His Tyr Tyr Gly Ser Ser His Trp Tyr Phe Asp Val
            100                 105                 110

Trp Gly Gln Gly Thr Leu Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Lys Val Glu Pro Lys
    210                 215                 220

Ser Cys Asp Lys Thr His Thr Cys Pro Pro Cys Pro Ala Pro Glu Ala
225                 230                 235                 240

Ala Gly Gly Pro Ser Val Phe Leu Phe Pro Pro Lys Pro Lys Asp Thr
```

```
                245                 250                 255

Leu Met Ile Ser Arg Thr Pro Glu Val Thr Cys Val Val Val Asp Val
            260                 265                 270

Ser His Glu Asp Pro Glu Val Lys Phe Asn Trp Tyr Val Asp Gly Val
        275                 280                 285

Glu Val His Asn Ala Lys Thr Lys Pro Arg Glu Glu Gln Tyr Asn Ser
    290                 295                 300

Thr Tyr Arg Val Val Ser Val Leu Thr Val Leu His Gln Asp Trp Leu
305                 310                 315                 320

Asn Gly Lys Glu Tyr Lys Cys Lys Val Ser Asn Lys Ala Leu Pro Ala
                325                 330                 335

Pro Ile Glu Lys Thr Ile Ser Lys Ala Lys Gly Gln Pro Arg Glu Pro
            340                 345                 350

Gln Val Tyr Thr Leu Pro Pro Ser Arg Asp Glu Leu Thr Lys Asn Gln
        355                 360                 365

Val Ser Leu Thr Cys Leu Val Lys Gly Phe Tyr Pro Ser Asp Ile Ala
    370                 375                 380

Val Glu Trp Glu Ser Asn Gly Gln Pro Glu Asn Asn Tyr Lys Thr Thr
385                 390                 395                 400

Pro Pro Val Leu Asp Ser Asp Gly Ser Phe Phe Leu Tyr Ser Lys Leu
                405                 410                 415

Thr Val Asp Lys Ser Arg Trp Gln Gln Gly Asn Val Phe Ser Cys Ser
            420                 425                 430

Val Met His Glu Ala Leu His Asn His Tyr Thr Gln Lys Ser Leu Ser
        435                 440                 445
```

Leu Ser Pro Gly Lys
450

<210> 25
<211> 1359
<212> DNA
<213> Artificial Sequence

<220>
<223> nucleic acid encoding Immunoglobulin heavy chain of VP101(hG1DM)

<400> 25
gaggtgcagc tggtcgagtc cgggggggg ctggtgcagc caggcgggtc tctgaggctg 60

agttgcgccg cttcagggta caccttcaca aactatggaa tgaattgggt gcgccaggca 120

ccaggaaagg gactggagtg ggtcggctgg atcaacactt acaccgggga acctacctat 180

gcagccgact ttaagcggcg gttcaccttc agcctggata caagcaaatc cactgcctac 240

ctgcagatga acagcctgcg agctgaggac accgcagtct actattgtgc taaatatccc 300

cactactatg ggagcagcca ttggtatttt gacgtgtggg ggcaggggac tctggtgaca 360

gtgagcagcg caagcaccaa agggcccagc gtgtttcctc tcgcccctc ctccaaaagc 420

accagcggag gaaccgctgc tctcggatgt ctggtgaagg actacttccc tgaacccgtc 480

accgtgagct ggaatagcgg cgctctgaca agcggagtcc atacattccc tgctgtgctg 540

caaagcagcg gactctattc cctgtccagc gtcgtcacag tgcccagcag cagcctgggc 600

acccagacct acatctgtaa cgtcaaccac aagccctcca acaccaaggt ggacaagaaa 660

gtggagccca aatcctgcga caagacacac acctgtcccc cctgtcctgc tcccgaagct 720

gctggaggcc ctagcgtctt cctctttcct cccaaaccca aggacaccct catgatcagc 780

agaacccctg aagtcacctg tgtcgtcgtg gatgtcagcc atgaggaccc cgaggtgaaa 840

ttcaactggt atgtcgatgg cgtcgaggtg cacaacgcca aaaccaagcc cagggaggaa 900

cagtacaact ccacctacag ggtggtgtcc gtgctgacag tcctccacca ggactggctg 960

aacggcaagg agtacaagtg caaggtgtcc aacaaggctc tccctgcccc cattgagaag 1020

accatcagca aggccaaagg ccaacccagg gagccccagg tctatacact gcctccctcc 1080

agggacgaac tcaccaagaa ccaggtgtcc ctgacctgcc tggtcaaggg cttttatccc 1140

agcgacatcg ccgtcgagtg ggagtccaac ggacagcccg agaataacta caagaccacc 1200

cctcctgtcc tcgactccga cggctccttc ttcctgtaca gcaaactgac cgtcgataaa 1260

tctaggtggc agcagggcaa cgtgttctct tgttccgtga tgcatgaagc actgcacaac 1320

cattataccc agaagtctct gagcctgtcc cccggcaag 1359

<210> 26
<211> 214
<212> PRT
<213> Artificial Sequence

<220>
<223> Immunoglobulin light chain of VP101(hG1DM)

<400> 26

```
Asp Ile Gln Met Thr Gln Ser Pro Ser Ser Leu Ser Ala Ser Val Gly
1               5                   10                  15

Asp Arg Val Thr Ile Thr Cys Ser Ala Ser Gln Asp Ile Ser Asn Tyr
            20                  25                  30

Leu Asn Trp Tyr Gln Gln Lys Pro Gly Lys Ala Pro Lys Val Leu Ile
        35                  40                  45

Tyr Phe Thr Ser Ser Leu His Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Val Pro Trp
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125
```

```
Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210


<210>  27
<211>  642
<212>  DNA
<213>  Artificial Sequence

<220>
<223>  nucleic acid encoding Immunoglobulin light chain of VP101(hG1DM)

<400>  27
gatattcaga tgactcagag cccctcctcc ctgtccgcct ctgtgggcga cagggtcacc       60

atcacatgca gtgcttcaca ggatatttcc aactacctga attggtatca gcagaagcca      120

ggaaaagcac ccaaggtgct gatctacttc actagctccc tgcactcagg agtgccaagc      180

cggttcagcg gatccggatc tggaaccgac tttactctga ccatttctag tctgcagcct      240

gaggatttcg ctacatacta ttgccagcag tattctaccg tgccatggac atttggccag      300

gggactaaag tcgagatcaa gcggaccgtg gccgctccca gtgtcttcat tttccccct       360

agcgacgaac agctgaaatc cgggacagcc tctgtggtct gtctgctgaa caacttctac      420

cctagagagg caaaagtgca gtggaaggtc gataacgccc tgcagagtgg caattcacag      480

gagagcgtga cagaacagga ctccaaagat tctacttata gtctgtcaag cacactgact      540
```

ctgagcaagg ctgactacga aaagcataaa gtgtatgcat gtgaggtcac ccaccagggg 600

ctgagcagtc cagtcaccaa gtcattcaac agaggcgagt gc 642

<210> 28
<211> 8
<212> PRT
<213> Artificial Sequence

<220>
<223> HCDR1

<400> 28

Gly Tyr Thr Phe Thr Asn Tyr Gly
1 5

<210> 29
<211> 8
<212> PRT
<213> Artificial Sequence

<220>
<223> HCDR2

<400> 29

Ile Asn Thr Tyr Thr Gly Glu Pro
1 5

<210> 30
<211> 16
<212> PRT
<213> Artificial Sequence

<220>
<223> HCDR3

<400> 30

Ala Lys Tyr Pro His Tyr Tyr Gly Ser Ser His Trp Tyr Phe Asp Val
1 5 10 15

<210> 31
<211> 6
<212> PRT
<213> Artificial Sequence

<220>
<223> LCDR1

<400> 31

Gln Asp Ile Ser Asn Tyr
1 5

<210> 32
<211> 3
<212> PRT
<213> Artificial Sequence

<220>
<223> LCDR2

<400> 32

Phe Thr Ser
1

<210> 33
<211> 9
<212> PRT
<213> Artificial Sequence

<220>
<223> LCDR3

<400> 33

Gln Gln Tyr Ser Thr Val Pro Trp Thr
1 5

<210> 34
<211> 8
<212> PRT
<213> Artificial Sequence

<220>
<223> HCDR1

<400> 34

Gly Phe Ala Phe Ser Ser Tyr Asp
1 5

<210> 35
<211> 8
<212> PRT
<213> Artificial Sequence

<220>
<223> HCDR2

<400> 35

Ile Ser Gly Gly Gly Arg Tyr Thr
1               5

<210> 36
<211> 11
<212> PRT
<213> Artificial Sequence

<220>
<223> HCDR3

<400> 36

Ala Asn Arg Tyr Gly Glu Ala Trp Phe Ala Tyr
1               5                   10

<210> 37
<211> 6
<212> PRT
<213> Artificial Sequence

<220>
<223> LCDR1

<400> 37

Gln Asp Ile Asn Thr Tyr
1               5

<210> 38
<211> 3
<212> PRT
<213> Artificial Sequence

<220>
<223> LCDR2

<400> 38

Arg Ala Asn
1

<210> 39
<211> 9
<212> PRT
<213> Artificial Sequence

<220>
<223> LCDR3

<400> 39

Leu Gln Tyr Asp Glu Phe Pro Leu Thr
1               5

<210> 40
<211> 330
<212> PRT
<213> Artificial Sequence

<220>
<223> Ig gamma-1 chain C region

<400> 40

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys

```
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285
```

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210> 41
<211> 327
<212> PRT
<213> Artificial Sequence

<220>
<223> Ig gamma-4 chain C region

<400> 41

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110

Glu Phe Leu Gly Gly Pro Ser Val Phe Leu Phe Pro Pro Lys Pro Lys

```
            115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320
```

```
Leu Ser Leu Ser Leu Gly Lys
                325


<210>  42
<211>  107
<212>  PRT
<213>  Artificial Sequence

<220>
<223>  Ig kappa chain C region

<400>  42

Arg Thr Val Ala Ala Pro Ser Val Phe Ile Phe Pro Pro Ser Asp Glu
1               5                   10                  15


Gln Leu Lys Ser Gly Thr Ala Ser Val Val Cys Leu Leu Asn Asn Phe
            20                  25                  30


Tyr Pro Arg Glu Ala Lys Val Gln Trp Lys Val Asp Asn Ala Leu Gln
        35                  40                  45


Ser Gly Asn Ser Gln Glu Ser Val Thr Glu Gln Asp Ser Lys Asp Ser
    50                  55                  60


Thr Tyr Ser Leu Ser Ser Thr Leu Thr Leu Ser Lys Ala Asp Tyr Glu
65                  70                  75                  80


Lys His Lys Val Tyr Ala Cys Glu Val Thr His Gln Gly Leu Ser Ser
                85                  90                  95


Pro Val Thr Lys Ser Phe Asn Arg Gly Glu Cys
            100                 105
```

## Description

TECHNICAL FIELD

The present invention belongs to the field of tumor treatment and immunobiology, and relates to an anti-PD-1-anti-VEGFA bispecific antibody, a pharmaceutical composition thereof and use thereof. Specifically, the present invention relates to an anti-human PD-1-anti-human VEGFA bispecific antibody, a pharmaceutical composition thereof and use thereof.

BACKGROUND

Tumor, especially a malignant tumor, is a serious health-threatening disease in the world today, and it is the second leading cause of death among various diseases. In recent years, the incidence of the disease has been increasing remarkably. Malignant tumor is characterized by poor treatment response, high late metastasis rate and poor prognosis. Although conventional treatment methods (such as radiotherapy, chemotherapy and surgical treatment) adopted clinically at present alleviate the pain to a great extent and prolong the survival time, the methods have great limitations, and it is difficult to further improve their efficacy.

There are two distinct stages of tumor growth, namely, from a slow growth stage without blood vessels to a rapid proliferation stage with blood vessels. The angiogenesis enables the tumor to acquire enough nutrition to complete the blood vessel switching stage, and if there is no angiogenesis, the primary tumor will be no more than 1-2 mm, and thus the metastasis cannot be realized.

Vascular Endothelial Growth Factor (VEGF) is a growth factor which can promote division and proliferation of endothelial cells, promote formation of new blood vessels and improve blood vessel permeability, and it binds to vascular endothelial growth factor receptors on the cell surface and plays a role by activating tyrosine kinase signal transduction pathways. In tumor tissues, tumor cells, and macrophages and mast cells invading into tumors can secrete high-level VEGF, stimulate tumor vascular endothelial cells in a paracrine form, promote proliferation and migration of endothelial cells, induce angiogenesis, promote continuous growth of tumor, improve vascular permeability, cause fibrin deposition in surrounding tissues, and promote infiltration of mononuclear cells, fibroblasts and endothelial cells, which facilitates formation of tumor stroma and entry of tumor cells into new blood vessels, and promote tumor metastasis. Therefore, inhibiting tumor angiogenesis is considered to be one of the most promising tumor treatment methods at present. The VEGF family includes: VEGFA, VEGFB, VEGFC, VEGFD and PIGF. Vascular Endothelial Growth Factor Receptors (VEGFRs) include VEGFR1 (also known as Flt1), VEGFR2 (also known as KDR or Flk1), VEGFR3 (also known as Flt4), and Neuropilin-1 (NRP-1). The first three receptors are similar in structure, belong to a tyrosine kinase superfamily, and are composed of an extramembrane region, a transmembrane segment and an intramembrane region, where the extramembrane region is composed of an immunoglobulin-like domain, and the intramembrane region is a tyrosine kinase region. VEGFR1 and VEGFR2 are located primarily on the surface of vascular endothelial cells, and VEGFR3 is located primarily on the surface of lymphatic endothelial cells.

Molecules of the VEGF family have different affinities for these receptors. VEGFA mainly acts in combination with VEGFR1, VEGFR2 and NRP-1. VEGFR1 is the earliest found receptor and has a higher affinity for VEGFA than VEGFR2 under normal physiological conditions, but it has a lower tyrosinase activity in intracellular segment than VEGFR2 (Ma Li, Chinese Journal of Birth Health and Heredity, 2016, 24 (5): 146-148).

VEGFR2 is the primary regulator of angiogenesis and vascular engineering, and has a much higher tyrosine kinase activity than VEGFR1. VEGFR2, after binding to ligand VEGFA, mediates the proliferation, differentiation and the like of vascular endothelial cells, as well as the formation process of blood vessels and the permeability of blood vessels (Roskoski R Jr. et al., Crit Rev Oncol Hematol, 2007, 62(3): 179-213). VEGFA, after binding to VEGFR2, mediates the transcriptional expression of intracellular related protein genes through the downstream PLC-γ-PKC-Raf-MEK-MAPK signaling pathway, and thus promotes the proliferation of vascular endothelial cells (Takahashi T et al., Oncogene, 1999, 18(13): 2221-2230).

VEGFR3 is one of the tyrosine kinase family members, and mainly expresses embryonic vascular endothelial cell and adult lymphatic endothelial cells, and VEGFC and VEGFD bind to VEGFR3 to stimulate proliferation and migration of lymphatic endothelial cells and promote neogenesis of lymphatic vessels; NRP-1 is a non-tyrosine kinase transmembrane protein and is incapable of independently transducing biological signals, and it is able to mediate signaling only after forming a complex with a VEGF tyrosine kinase receptor. (Ma Li, Chinese Journal of Birth Health and Heredity, 2016, 24 (5): 146-148).

VEGFA and VEGFR2 are mainly involved in regulation of angiogenesis, where before and after the binding of VEGFA to VEGFR2, a cascade reaction of numerous intermediate signals in upstream and downstream pathways is formed, and finally the physiological functions are changed by proliferation, survival, migration, permeability increase and infiltration to peripheral tissues, etc. of endothelial cells (Dong Hongchao et al., Journal of Modern Oncology, Sep. 2014, 22 (9): 2231-3).

Currently, there are several humanized monoclonal antibodies targeting human VEGF, particularly VEGFA, such as bevacizumab, which has been approved by the U.S. Food and Drug Administration for the treatment of various tumors such as non-small cell lung cancer, renal cell carcinoma, cervical cancer, and metastatic colorectal cancer in succession during 2004.

The programmed cell death receptor-1 (PD-1), also known as CD279, is a type I transmembrane glycoprotein membrane surface receptor, belongs to the CD28 immunoglobulin superfamily, and is commonly expressed in T cells, B cells, and myeloid cells. PD-1 has two natural ligands, PD-L1 and PD-L2. Both PD-L1 and PD-L2 belong to the B7 superfamily and are expressed constitutively or inducibly on the membrane surface of a variety of cells, including nonhematopoietic cells and a variety of tumor cells. PD-L1 is mainly expressed on T cells, B cells, DC and microvascular endothelial cells and a variety of tumor cells, while PD-L2 is expressed only on antigen presenting cells such as dendritic cells and macrophages. The interaction between PD-1 and its ligands can inhibit the activation of lymph, the proliferation of T cells, and the secretion of cytokines such as IL-2 and IFN-y.

A large number of researches show that a tumor microenvironment can protect tumor cells from being damaged by immune cells, expression of PD-1 in lymphocytes infiltrated in the tumor microenvironment is up-regulated, and various primary tumor tissues are PD-L1 positive in immunohistochemical analysis, such as lung cancer, liver cancer, ovarian cancer, skin cancer, colon cancer and glioma. Meanwhile, the expression of PD-L1 in the tumor is significantly correlated with poor prognosis of cancer patients. Blocking the interaction between PD-1 and its ligands can promote the tumor-specific T cell immunity and enhance the immune elimination efficiency of tumor cells. A large number of clinical trials show that antibodies targeting PD-1 or PD-L1 can promote infiltration of CD8+ T cells into tumor tissues and up-regulate anti-tumor immune effector factors such as IL-2, IFN-y, granzyme B and perforin, thereby effectively inhibiting the growth of tumors.

In addition, anti-PD-1 antibodies may also be used in the treatment of viral chronic infections. Viral chronic infections are often accompanied by a loss of function of virus-specific effector T cells and a reduction in its number. The interaction between PD-1 and PD-L1 can be blocked by injecting a PD-1 antibody, thereby effectively inhibiting the exhaustion of effector T cells in viral chronic infection.

Due to the broad anti-tumor prospect and surprising efficacy of PD-1 antibodies, it is widely accepted in the industry that antibodies targeting the PD-1 pathway will bring about breakthroughs in the treatment of a variety of tumors: for the treatment of non-small cell lung cancer, renal cell carcinoma, ovarian cancer and melanoma (Homet M. B., Parisi G., et al., Anti-PD-1 Therapy in Melanoma. Semin Oncol., Jun. 2015, 42 (3): 466-473), and lymphoma and anemia (Held SA, Heine A, et al., Advances in immunotherapy of chronic myeloid leukemia CML. Curr Cancer Drug Targets., Sep. 2013, 13 (7): 768-74), microsatellite instability-high (MSI-H) or deficient mismatch repair (dMMR) tumors (several anti-PD-1 antibody drugs have been approved by the FDA et al for the treatment of tumors with MSI-H/dMMR characteristics). The current combination of anti-angiogenic therapy and immune checkpoint inhibitors shows good efficacy in many tumors. For example, the combination of the anti-VEGF antibodies (such as bevacizumab) and PD-1/PD-L1 antibodies (such as nivolumab, pembrolizumab and atezolizumab) for use in the treatment of ovarian cancer (Joyce F. Liu et al., JAMA Oncol., 2019; 5(12): 1731-1738), non-small cell lung cancer (non-small cell lung cancer including EGFR and/or ALK sensitive mutations) (Manegold C, et al., J Thorac Oncol., Feb. 2017; 12(2): 194-207), renal cell carcinoma (Dudek AZ et al., J Clin Oncol., 2018; 36 (suppl; abstr., 4558)), hepatocellular carcinoma (Stein S et al., J Clin Oncol, 2018, 36(15_suppl): 4074; bevacizumab in combination with atezolizumab for use in the treatment of hepatocellular carcinoma endorsed by the FDA in 2020), colorectal cancer (including MSI-H/dMMR and non-MSI-H/dMMR types) (Bendell JC et al., Safety and efficacy of MPDL3280A (anti-PDL1) in combination with bevacizumab (bev) and/or FOLFOX in patients (pts) with metastatic colorectal cancer (mCRC). Presented at: American Society of Clinical Oncology; May 29-June 2, 2015; Chicago, IL. 2015; abstract 704; Hochster HS et al., Efficacy and safety of atezolizumab (atezo) and bevacizumab (bev) in a phase Ib study of microsatellite instability (MSI)-high metastatic colorectal cancer (mCRC). Presented at: American Society of Clinical Oncology Gastrointestinal Cancers Symposium; January 19-21, 2017; San Francisco, CA. 2017; abstract 673), breast cancer (Yukinori Ozaki et al., A multicenter phase II study evaluating the efficacy of nivolumab plus paclitaxel plus bevacizumab triple-combination therapy as a first-line treatment in patients with HER2-negative metastatic breast cancer: WJOG9917B NEWBEAT trial [abstract]. In: Proceedings of the 2019 San Antonio Breast Cancer Symposium; 2019 Dec 10-14; San Antonio, TX. Philadelphia (PA): AACR; Cancer Res 2020; 80(4 Suppl): Abstract nr PD1-03); the combination of VEGFR2 antibody and PD-1 antibody also showed good anti-tumor effects in adenocarcinoma of the stomach and gastroesophageal junction adenocarcinoma (Herbst RS et al., Lancet Oncol. 2019; 20(8): 1109-1123); also, the combination regimen of the PD-1 antibody (Pembrolizumab) and the angiogenesis inhibitor (Lenvatinib) showed good efficacy in the treatment of endometrial cancer, and was approved by the FDA in 2019 for use in the treatment of the endometrial cancer. For melanoma (PD-1 antibodies nivolumab and pembrolizumab have been approved by the FDA for use in the treatment of melanoma), cervical cancer (Krishnansu S. et al., N Engl J Med., 2014; 370: 734-743), glioma, prostate cancer (Antonarakis ES. et al., J Clin Oncol., Feb 10, 2020; 38(5): 395-405), urothelial cancer (Joaquim Bellmunt. et al., N Engl J Med., 2017; 376: 1015-1026; nivolumab for use in the treatment of bladder cancer endorsed by the FDA in 2017), esophageal cancer (Kato K et al., Lancet Oncol., 2019; 20(11): 1506-17), mesothelioma (Scherpereel A et al., Lancet Oncol., 2019; 20(2): 239-253) and the like, the PD-1/PDL-1 antibodies show good efficacy, and considering that the PD-1 pathway has a synergistic effect with the VEGF pathway in tumorigenesis, it can be expected that drugs that block both PD-1 and VEGF pathways will have better anti-tumor effects.

The bispecific antibody, also known as bispecific antibody, is a specific medicament that targets two different antigens simultaneously, and can be produced by immunoselection purification. In addition, the bispecific antibody can also be produced by genetic engineering, which has certain advantages due to corresponding flexibility in aspects such as the optimization of binding sites, consideration of synthetic form, and yield. Currently, the bispecific antibody has been demonstrated to exist in over 45 forms (Müller D, Kontermann RE. Bispecific antibodies for cancer immunotherapy: current perspectives. BioDrugs 2010; 24: 89-98). A number of bispecific antibodies have been developed in the form of IgG-ScFv, namely the Morrison form (Coloma M. J., Morrison S. L. Design and production of novel tetravalent bispecific antibodies. Nat Biotechnol., 1997; 15: 159-163), which has been demonstrated to be one of the ideal forms of the bispecific antibodies because of its similarity to the naturally existing IgG form and advantages in antibody engineering, expression and purification (Miller B. R., Demarest S. J., et al., Stability engineering of scFvs for the development of bispecific and multivalent antibodies. Protein Eng Des Sel 2010; 23: 549-57; Fitzgerald J, Lugovskoy A. Rational engineering of antibody therapeutics targeting multiple oncogene pathways. MAbs 2011; 3: 299-309).

ADCC (antibody-dependent cell-mediated cytotoxicity) refers to killing of a target cell by a killer cell (NK cells, macrophages, etc.) that is mediated by binding of the Fab fragment of an antibody to an epitope of a virus-infected cell or a tumor cell and binding of the Fc fragment of the antibody to an Fc receptor (FcR) on the surface of the killer cell.

CDC (complement dependent cytotoxicity) refers to that the specific binding of an antibody to a corresponding antigen on a cell membrane surface forms a complex and activates the complement system, which further forms an MAC on the surface of the target cell resulting in subsequent target cell lysis. Complements may cause lysis of various bacteria and other pathogenic organisms, and are an important defense mechanism against pathogenic organism infections. Fc receptors belong to an immunoglobulin family that are expressed on the surface of specific immune cells to recognize antibody Fc regions and mediate immune responses. After the Fab region recognizes an antigen, the Fc region of the antibody binds to the Fc receptor on the immune cell (e.g., a killer cell) to initiate the response function of the immune cell, such as phagocytosis and ADCC.

According to the type of antibody recognized by the Fc receptor and the type of expression cells, Fc receptors are mainly classified into three types, FcyR, FcαR and FcεR. FcγR can be further classified into four subtypes, FcyRI (CD64), FcyRII (CD32), FcγRIII (CD16) and FcRn (neonatal Fc receptor). Among these, FcyRI, FcyRII and FcγRIII are closely associated with ADCC effect. FcγRIII is the most predominant molecule mediating ADCC, with two highly homologous subtypes, FcγRIIIa and FcγRIIIb, in different cell types. In FcγRIIIa populations, two subtypes distinguished by sites of single-nucleotide polymorphism (SNP), FcγRIIIa_V158 with high affinity and FcγRIIIa_F158 with low affinity, are present. FcyRI has higher affinity for the Fc region of IgG and participates in ADCC process; FcyRII comprises three subtypes of FcyRIIa, FcyRIIb and FcyRIIc (also referred to as CD32a, CD32b and CD32c respectively), among which FcγRIIa has ADCC activity; for FcyRIIa, two subtypes of FcγRIIa_H131 and FcγRIIa_R131 are present in humans due to single nucleotide mutation (Hogarth PM, Pietersz GA. 2012, NATURE REVIEWS DRUG DISCOVERY, 11(4): 311-331).

The IgG family comprises four members, IgG1, IgG2, IgG3 and IgG4, which differ in amino acids in the fragment crystallizable (Fc) region of the heavy chain constant region, resulting in their varying affinities for FcyRs. IgG1 is the most abundant subtype in humans and is also the most common subtype used in monoclonal antibody medication. IgG1 is capable of binding various FcyRs and is able to induce ADCC and CDC effects. IgG2 has the lowest affinity for FcyRs, but is still able to induce monocyte-mediated ADCC by binding to FcγRIIa. IgG3 features the highest binding capacity to FcyRs, and can induce ADCC and a greater CDC effect than IgG1. The IgG4 molecules bind weakly to FcyRs other than FcyRI, and the IgG4 molecules have a lower probability of causing CDC and NK cell-mediated ADCC.

At present, there is still a need for developing a novel anti-PD-1-anti-VEGFA bispecific antibody to reduce or eliminate the damage caused by antibody-mediated ADCC and/or CDC activity against immune cells to which the anti-PD-1-anti-VEGFA bispecific antibody binds, and to improve the efficacy of the antibody drug.

## SUMMARY

By intensive research and creative efforts, the inventor correspondingly modified the Fc fragment of the anti-PD-1-anti-VEGFA antibody structure to reduce the binding capacity of the Fc region to Fc receptors, thereby reducing ADCC and CDC toxic and side effects on immune cells and increasing the drug efficacy of the anti-PD-1-anti-VEGFA antibody drug.

The present invention is detailed below.

One aspect of the present invention relates to a bispecific antibody, which comprises:

a first protein functional region targeting PD-1, and

a second protein functional region targeting VEGFA;
wherein the first protein functional region is an immunoglobulin, and the second protein functional region is a single chain antibody; or, the first protein functional region is a single chain antibody, and the second protein functional region is an immunoglobulin;
wherein,
for the immunoglobulin, the heavy chain variable region comprises HCDR1-HCDR3 of amino acid sequences set forth in SEQ ID NOs: 28-30 respectively, and the light chain variable region comprises LCDR1-LCDR3 of amino acid sequences set forth in SEQ ID NOs: 31-33 respectively; for the single chain antibody, the heavy chain variable region comprises HCDR1-HCDR3 of amino acid sequences set forth in SEQ ID NOs: 34-36 respectively, and the light chain variable region comprises LCDR1-LCDR3 of amino acid sequences set forth in SEQ ID NOs: 37-39 respectively;
or,
for the immunoglobulin, the heavy chain variable region comprises HCDR1-HCDR3 of amino acid sequences set forth in SEQ ID NOs: 34-36 respectively, and the light chain variable region comprises LCDR1-LCDR3 of amino acid sequences set forth in SEQ ID NOs: 37-39 respectively; for the single chain antibody, the heavy chain variable region comprises HCDR1-HCDR3 of amino acid sequences set forth in SEQ ID NOs: 28-30 respectively, and the light chain variable region comprises LCDR1-LCDR3 of amino acid sequences set forth in SEQ ID NOs: 31-33 respectively;
the immunoglobulin is of human IgG1 subtype;
wherein, according to the EU numbering system, the heavy chain constant region of the immunoglobulin has mutations at any 2 or 3 of positions 234, 235 and 237, and the affinity constant of the bispecific antibody to FcγRIIIa and/or C1q is reduced after the mutation as compared to that before the mutation; preferably, the affinity constant is measured by a Fortebio Octet system.

In one or more embodiments of the present invention, for the bispecific antibody, according to the EU numbering system, the heavy chain constant region of the immunoglobulin has the following mutations:

L234A and L235A; or
L234A and G237A; or
L235A and G237A;
or
L234A, L235A and G237A.

In the present invention, letters before the position number represent amino acids before mutation, and letters after the position number represent amino acids after mutation, unless otherwise specified.
The present invention further relates to a bispecific antibody, which comprises:

a first protein functional region targeting PD-1, and
a second protein functional region targeting VEGFA;
wherein the first protein functional region is an immunoglobulin, and the second protein functional region is a single chain antibody; or, the first protein functional region is a single chain antibody, and the second protein functional region is an immunoglobulin;
wherein,
for the immunoglobulin, the heavy chain variable region comprises HCDR1-HCDR3 of amino acid sequences set forth in SEQ ID NOs: 28-30 respectively, and the light chain variable region comprises LCDR1-LCDR3 of amino acid sequences set forth in SEQ ID NOs: 31-33 respectively; for the single chain antibody, the heavy chain variable region comprises HCDR1-HCDR3 of amino acid sequences set forth in SEQ ID NOs: 34-36 respectively, and the light chain variable region comprises LCDR1-LCDR3 of amino acid sequences set forth in SEQ ID NOs: 37-39 respectively;
or,
for the immunoglobulin, the heavy chain variable region comprises HCDR1-HCDR3 of amino acid sequences set forth in SEQ ID NOs: 34-36 respectively, and the light chain variable region comprises LCDR1-LCDR3 of amino acid sequences set forth in SEQ ID NOs: 37-39 respectively; for the single chain antibody, the heavy chain variable region comprises HCDR1-HCDR3 of amino acid sequences set forth in SEQ ID NOs: 28-30 respectively, and the light chain variable region comprises LCDR1-LCDR3 of amino acid sequences set forth in SEQ ID NOs: 31-33 respectively;
the immunoglobulin is of human IgG1 subtype.

The present invention further relates to a bispecific antibody, which comprises:

a first protein functional region targeting PD-1, and
a second protein functional region targeting VEGFA;

wherein the first protein functional region is an immunoglobulin, and the second protein functional region is a single chain antibody; or, the first protein functional region is a single chain antibody, and the second protein functional region is an immunoglobulin;
wherein,
for the immunoglobulin, the heavy chain variable region comprises HCDR1-HCDR3 of amino acid sequences set forth in SEQ ID NOs: 28-30 respectively, and the light chain variable region comprises LCDR1-LCDR3 of amino acid sequences set forth in SEQ ID NOs: 31-33 respectively; for the single chain antibody, the heavy chain variable region comprises HCDR1-HCDR3 of amino acid sequences set forth in SEQ ID NOs: 34-36 respectively, and the light chain variable region comprises LCDR1-LCDR3 of amino acid sequences set forth in SEQ ID NOs: 37-39 respectively; or,
for the immunoglobulin, the heavy chain variable region comprises HCDR1-HCDR3 of amino acid sequences set forth in SEQ ID NOs: 34-36 respectively, and the light chain variable region comprises LCDR1-LCDR3 of amino acid sequences set forth in SEQ ID NOs: 37-39 respectively; for the single chain antibody, the heavy chain variable region comprises HCDR1-HCDR3 of amino acid sequences set forth in SEQ ID NOs: 28-30 respectively, and the light chain variable region comprises LCDR1-LCDR3 of amino acid sequences set forth in SEQ ID NOs: 31-33 respectively;
the immunoglobulin is of human IgG1 subtype;
wherein, according to the EU numbering system, the heavy chain constant region of the immunoglobulin has the following mutations:

L234A and L235A; or
L234A and G237A; or
L235A and G237A; or
L234A, L235A and G237A.

In one or more embodiments of the present invention, for the bispecific antibody, according to the EU numbering system, the heavy chain constant region of the immunoglobulin has or further has one or more mutations selected from: N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, P331S, H268A, E318A and K320A.

In one or more embodiments of the present invention, the bispecific antibody is provided, wherein

the amino acid sequence of the heavy chain variable region of the immunoglobulin is set forth in SEQ ID NO: 1, and the amino acid sequence of the light chain variable region of the immunoglobulin is set forth in SEQ ID NO: 3; the amino acid sequence of the heavy chain variable region of the single chain antibody is selected from SEQ ID NO: 5 and SEQ ID NO: 9, and the amino acid sequence of the light chain variable region of the single chain antibody is selected from SEQ ID NO: 7, SEQ ID NO: 11 and SEQ ID NO: 17;
or,
the amino acid sequence of the heavy chain variable region of the immunoglobulin is selected from SEQ ID NO: 5 and SEQ ID NO: 9, and the amino acid sequence of the light chain variable region of the immunoglobulin is selected from SEQ ID NO: 7, SEQ ID NO: 11 and SEQ ID NO: 17; the amino acid sequence of the heavy chain variable region of the single chain antibody is set forth in SEQ ID NO: 1, and the amino acid sequence of the light chain variable region of the single chain antibody is set forth in SEQ ID NO: 3.

In one or more embodiments of the present invention, the bispecific antibody is selected from any one of the following (1)-(12):

(1)
the amino acid sequence of the heavy chain variable region of the immunoglobulin is set forth in SEQ ID NO: 1, and the amino acid sequence of the light chain variable region of the immunoglobulin is set forth in SEQ ID NO: 3; the amino acid sequence of the heavy chain variable region of the single chain antibody is set forth in SEQ ID NO: 5, and the amino acid sequence of the light chain variable region of the single chain antibody is set forth in SEQ ID NO: 7;
(2)
the amino acid sequence of the heavy chain variable region of the immunoglobulin is set forth in SEQ ID NO: 1, and the amino acid sequence of the light chain variable region of the immunoglobulin is set forth in SEQ ID NO: 3; the amino acid sequence of the heavy chain variable region of the single chain antibody is set forth in SEQ ID NO: 5, and the amino acid sequence of the light chain variable region of the single chain antibody is set forth in SEQ ID NO: 11;
(3)
the amino acid sequence of the heavy chain variable region of the immunoglobulin is set forth in SEQ ID NO: 1, and the amino acid sequence of the light chain variable region of the immunoglobulin is set forth in SEQ ID NO: 3; the amino acid sequence of the heavy chain variable region of the single chain antibody is set forth in SEQ ID NO: 5, and the amino acid sequence of the light chain variable region of the single chain antibody is set forth in SEQ ID NO: 17;

(4)

the amino acid sequence of the heavy chain variable region of the immunoglobulin is set forth in SEQ ID NO: 1, and the amino acid sequence of the light chain variable region of the immunoglobulin is set forth in SEQ ID NO: 3; the amino acid sequence of the heavy chain variable region of the single chain antibody is set forth in SEQ ID NO: 9, and the amino acid sequence of the light chain variable region of the single chain antibody is set forth in SEQ ID NO: 7;

(5)

the amino acid sequence of the heavy chain variable region of the immunoglobulin is set forth in SEQ ID NO: 1, and the amino acid sequence of the light chain variable region of the immunoglobulin is set forth in SEQ ID NO: 3; the amino acid sequence of the heavy chain variable region of the single chain antibody is set forth in SEQ ID NO: 9, and the amino acid sequence of the light chain variable region of the single chain antibody is set forth in SEQ ID NO: 11;

(6)

the amino acid sequence of the heavy chain variable region of the immunoglobulin is set forth in SEQ ID NO: 1, and the amino acid sequence of the light chain variable region of the immunoglobulin is set forth in SEQ ID NO: 3; the amino acid sequence of the heavy chain variable region of the single chain antibody is set forth in SEQ ID NO: 9, and the amino acid sequence of the light chain variable region of the single chain antibody is set forth in SEQ ID NO: 17;

(7)

the amino acid sequence of the heavy chain variable region of the immunoglobulin is set forth in SEQ ID NO: 5, and the amino acid sequence of the light chain variable region of the immunoglobulin is set forth in SEQ ID NO: 7; the amino acid sequence of the heavy chain variable region of the single chain antibody is set forth in SEQ ID NO: 1, and the amino acid sequence of the light chain variable region of the single chain antibody is set forth in SEQ ID NO: 3;

(8)

the amino acid sequence of the heavy chain variable region of the immunoglobulin is set forth in SEQ ID NO: 5, and the amino acid sequence of the light chain variable region of the immunoglobulin is set forth in SEQ ID NO: 11; the amino acid sequence of the heavy chain variable region of the single chain antibody is set forth in SEQ ID NO: 1, and the amino acid sequence of the light chain variable region of the single chain antibody is set forth in SEQ ID NO: 3;

(9)

the amino acid sequence of the heavy chain variable region of the immunoglobulin is set forth in SEQ ID NO: 5, and the amino acid sequence of the light chain variable region of the immunoglobulin is set forth in SEQ ID NO: 17; the amino acid sequence of the heavy chain variable region of the single chain antibody is set forth in SEQ ID NO: 1, and the amino acid sequence of the light chain variable region of the single chain antibody is set forth in SEQ ID NO: 3;

(10)

the amino acid sequence of the heavy chain variable region of the immunoglobulin is set forth in SEQ ID NO: 9, and the amino acid sequence of the light chain variable region of the immunoglobulin is set forth in SEQ ID NO: 7; the amino acid sequence of the heavy chain variable region of the single chain antibody is set forth in SEQ ID NO: 1, and the amino acid sequence of the light chain variable region of the single chain antibody is set forth in SEQ ID NO: 3;

(11)

the amino acid sequence of the heavy chain variable region of the immunoglobulin is set forth in SEQ ID NO: 9, and the amino acid sequence of the light chain variable region of the immunoglobulin is set forth in SEQ ID NO: 11; the amino acid sequence of the heavy chain variable region of the single chain antibody is set forth in SEQ ID NO: 1, and the amino acid sequence of the light chain variable region of the single chain antibody is set forth in SEQ ID NO: 3; and

(12)

the amino acid sequence of the heavy chain variable region of the immunoglobulin is set forth in SEQ ID NO: 9, and the amino acid sequence of the light chain variable region of the immunoglobulin is set forth in SEQ ID NO: 17; the amino acid sequence of the heavy chain variable region of the single chain antibody is set forth in SEQ ID NO: 1, and the amino acid sequence of the light chain variable region of the single chain antibody is set forth in SEQ ID NO: 3.

In one or more embodiments of the present invention, the bispecific antibody is provided, wherein
the amino acid sequence of the heavy chain of the immunoglobulin is set forth in SEQ ID NO: 24, and the amino acid sequence of the light chain of the immunoglobulin is set forth in SEQ ID NO: 26.

In one or more embodiments of the present invention, the bispecific antibody is in the form of IgG-scFv, i.e., the Morrison format.

In some embodiments of the present invention, for the bispecific antibody in one or more embodiments of the present invention,
the heavy chain constant region of the immunoglobulin is human Ig gamma-1 chain C region or human Ig gamma-4 chain C region, and the light chain constant region of the immunoglobulin is human Ig kappa chain C region.

In some embodiments of the present invention, the constant regions of the immunoglobulin are humanized. For example, the heavy chain constant region is Ig gamma-1 chain C region, ACCESSION: P01857, and the light chain constant region is Ig kappa chain C region, ACCESSION: P01834; or
the heavy chain constant region of the immunoglobulin is Ig gamma-4 chain C region, ACCESSION: P01861.1; the light chain constant region of the immunoglobulin is Ig kappa chain C region, ACCESSION: P01834.

In one embodiment of the present invention, the amino acid sequence of the heavy chain constant region Ig gamma-1 chain C region (ACCESSION: P01857) is as follows:

ASTKGPSVFPLAPSSKSTSGGTAALGCLVKDYFPEPVTVSWNSGALTSGVHTFPAVLQS
SGLYSLSSVVTVPSSSLGTQTYICNVNHKPSNTKVDKKVEPKSCDKTHTCPPCPAPELLG
GPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLP
PSRDELTKNQVSLTCLVKGFYPSDIAVEWESNGQPENNYKTTPPVLDSDGSFFLYSKLT
VDKSRWQQGNVFSCSVMHEALHNHYTQKSLSLSPGK (SEQ ID NO: 40)

In one embodiment of the present invention, the amino acid sequence of the heavy chain constant region Ig gamma-4 chain C region (ACCESSION: P01861.1) is as follows:

ASTKGPSVFPLAPCSRSTSESTAALGCLVKDYFPEPVTVSWNSGALTSGVHTFPAVLQSS
GLYSLSSVVTVPSSSLGTKTYTCNVDHKPSNTKVDKRVESKYGPPCPSCPAPEFLGGPS
VFLFPPKPKDTLMISRTPEVTCVVVDVSQEDPEVQFNWYVDGVEVHNAKTKPREEQFN
STYRVVSVLTVLHQDWLNGKEYKCKVSNKGLPSSIEKTISKAKGQPREPQVYTLPPSQE
EMTKNQVSLTCLVKGFYPSDIAVEWESNGQPENNYKTTPPVLDSDGSFFLYSRLTVDKS
RWQEGNVFSCSVMHEALHNHYTQKSLSLSLGK (SEQ ID NO: 41)

In one embodiment of the present invention, the amino acid sequence of the light chain constant region Ig kappa chain C region (ACCESSION: P01834) is as follows:

RTVAAPSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC (SEQ ID NO: 42)

In some embodiments of the present invention, the bispecific antibody is provided, wherein the single chain antibody is linked to the C-terminus of the heavy chain of the immunoglobulin. Since an immunoglobulin has two heavy chains, two single chain antibody molecules are linked to one immunoglobulin molecule. Preferably, the two single chain antibody molecules are identical. In some embodiments of the present invention, the bispecific antibody is provided, wherein two single chain antibodies are present, and one terminus of each single chain antibody is linked to the C-terminus or the N-terminus of one of the two heavy chains of the immunoglobulin.

In some embodiments of the present invention, a disulfide bond is present between the $V_H$ and the $V_L$ of the single chain antibody. Methods for introducing a disulfide bond between the $V_H$ and $V_L$ of an antibody are well known in the art, see, for example, US 5,747,654; Rajagopal et. al., Prot. Engin., 10(1997)1453-1459; Reiter et. Al., Nat. Biotechnol., 14(1996)1239-1245; Reiter et. al., Protein Engineering, 8(1995)1323-1331; Webber et. al., Molecular Immunology, 32(1995)249-258; Reiter et. al., Immunity, 2(1995)281-287; Reiter et. al., JBC, 269(1994)18327-18331; Reiter et. al., Inter. J. of Cancer, 58(1994)142-149; or Reiter et al., Cancer Res. 54(1994)2714-2718, which are incorporated herein by reference.

In one or more embodiments of the present invention, the bispecific antibody is provided, wherein the first protein functional region is linked to the second protein functional region either directly or via a linker fragment; and/or the heavy chain variable region of the single chain antibody is linked to the light chain variable region of the single chain antibody either directly or via a linker fragment.

In one or more embodiments of the present invention, for the bispecific antibody, the linker fragment is (GGGGS) n, n being a positive integer; preferably, n is 1, 2, 3, 4, 5 or 6.

In one or more embodiments of the present invention, for the bispecific antibody, the numbers of the first protein functional region and the second protein functional region are each independently 1, 2 or more.

In one or more embodiments of the present invention, the bispecific antibody is provided, wherein the single chain antibody is linked to the C-terminus of the heavy chain of the immunoglobulin.

The present invention further relates to a bispecific antibody, which comprises:

a first protein functional region targeting PD-1, and
a second protein functional region targeting VEGFA;
wherein the number of the first protein functional region is 1, and the number of the second protein functional region is 2;
the first protein functional region is an immunoglobulin, and the second protein functional region is a single chain antibody;
the amino acid sequence of the heavy chain of the immunoglobulin is set forth in SEQ ID NO: 24, and the amino acid sequence of the light chain of the immunoglobulin is set forth in SEQ ID NO: 26;
the amino acid sequence of the heavy chain variable region of the single chain antibody is set forth in SEQ ID NO: 9, and the amino acid sequence of the light chain variable region of the single chain antibody is set forth in SEQ ID NO: 17;
the single chain antibody is linked to the C-terminus of the heavy chain of the immunoglobulin;
the first protein functional region is linked to the second protein functional region via a first linker fragment; and the heavy chain variable region of the single chain antibody is linked to the light chain variable region of the single chain antibody via a second linker fragment; the first linker fragment and the second linker fragment are the same or different;
preferably, the amino acid sequences of the first linker fragment and second linker fragment are independently selected from SEQ ID NO: 18 and SEQ ID NO: 19;
preferably, the amino acid sequences of the first linker fragment and second linker fragments are set forth in SEQ ID NO: 18.

In one or more embodiments of the present invention, the bispecific antibody is provided, wherein the immunoglobulin or an antigen-binding fragment thereof binds to FcγRI with an affinity constant of less than about $10^{-6}$ M, such as less than about $10^{-7}$ M, $10^{-8}$ M or $10^{-9}$ M or less; preferably, the affinity constant is measured by a Fortebio Octet system.

In one or more embodiments of the present invention, the bispecific antibody is provided, wherein the immunoglobulin or the antigen-binding fragment thereof binds to C1q with an affinity constant of less than about $10^{-9}$ M, such as less than about $10^{-7}$ M, $10^{-8}$ M or $10^{-9}$ M or less; preferably, the affinity constant is measured by a Fortebio Octet system.

In some embodiments of the present invention, the bispecific antibody is provided, wherein the bispecific antibody binds to a VEGFA protein and/or a PD-1 protein with a $K_D$ of less than $10^{-5}$ M, such as less than $10^{-6}$ M, $10^{-7}$ M, $10^{-8}$ M, $10^{-9}$ M or $10^{-10}$ M or less; preferably, the $K_D$ is measured by a Fortebio system.

In some embodiments of the present invention, the bispecific antibody is provided, wherein, the bispecific antibody binds to the VEGFA protein with an $EC_{50}$ of less than 1 nM, less than 0.5 nM, less than 0.2 nM, less than 0.15 nM, or less than 0.14 nM; preferably, the $EC_{50}$ is detected by indirect ELISA;
and/or,
the bispecific antibody binds to the PD-1 protein with an $EC_{50}$ of less than 1 nM, less than 0.5 nM, less than 0.2 nM, less than 0.17 nM, less than 0.16 nM, or less than 0.15 nM; preferably, the $EC_{50}$ is detected by indirect ELISA.

In one or more embodiments of the present invention, the bispecific antibody is a monoclonal antibody.

In one or more embodiments of the present invention, the bispecific antibody is a humanized antibody.

Another aspect of the present invention relates to an isolated nucleic acid molecule encoding the bispecific antibody according to any embodiment of the present invention.

Yet another aspect of the present invention relates to a vector comprising the isolated nucleic acid molecule disclosed herein.

Yet another aspect of the present invention relates to a host cell comprising the isolated nucleic acid molecule or the vector described herein.

Another aspect of the present invention relates to a conjugate comprising an antibody or an antigen-binding fragment thereof and a conjugated moiety, wherein the immunoglobulin is the bispecific antibody according to any embodiment of the present invention, and the conjugated moiety is a detectable label; preferably, the conjugated moiety is a radioisotope, a fluorescent substance, a luminescent substance, a colored substance, or an enzyme.

Another aspect of the present invention relates to a kit comprising the bispecific antibody according to any embodiment of the present invention or comprising the conjugate of the present invention;
wherein preferably, the kit further comprises a second antibody capable of specifically recognizing the immunoglobulin or the antigen binding fragment thereof; optionally, the second antibody further comprises a detectable label, for example, a radioisotope, a fluorescent substance, a luminescent substance, a colored substance, or an enzyme.

Yet another aspect of the present invention relates to use of the bispecific antibody or the conjugate according to any embodiment of the present invention in preparing a kit for detecting the presence or level of PD-1 and/or VEGFA in a sample.

Another aspect of the present invention relates to a pharmaceutical composition comprising the bispecific antibody or the conjugate according to any embodiment of the present invention; optionally, the pharmaceutical composition further comprises a pharmaceutically acceptable vector and/or excipient.

The bispecific antibody of the present invention or the pharmaceutical composition of the present invention may be formulated into any dosage form known in the pharmaceutical field, such as tablet, pill, suspension, emulsion, solution, gel, capsule, powder, granule, elixir, troche, suppository, injection (including injection solution, sterile powder for injection and concentrated solution for injection), inhalant, and spray. The preferred dosage form depends on the intended mode of administration and therapeutic use. The pharmaceutical composition of the present invention should be sterile and stable under the conditions of manufacture and storage. One preferred dosage form is an injection. Such injections may be sterile injection solutions. For example, sterile injection solutions can be prepared by the following method: a necessary amount of the bispecific antibody of the present invention is added in an appropriate solvent, and optionally, other desired ingredients (including, but not limited to, pH regulators, surfactants, adjuvants, ionic strength enhancers, isotonic agents, preservatives, diluents, or any combination thereof) are added at the same time, followed by filtration and sterilization. In addition, sterile injection solutions can be prepared as sterile lyophilized powders (e.g., by vacuum drying or lyophilizing) for convenient storage and use. Such sterile lyophilized powders may be dispersed in a suitable vector (e.g., sterile pyrogen-free water) prior to use.

In addition, the bispecific antibody of the present invention may be present in a pharmaceutical composition in unit dose form for ease of administration. In some embodiments, the unit dose is at least 1 mg, at least 5 mg, at least 10 mg, at least 15 mg, at least 20 mg, at least 25 mg, at least 30 mg, at least 45 mg, at least 50 mg, at least 75 mg, or at least 100 mg. Where the pharmaceutical composition is in a liquid (e.g., injection) dosage form, it may comprise the bispecific antibody of the present invention at a concentration of at least 0.1 mg/mL, such as at least 0.25 mg/mL, at least 0.5 mg/mL, at least 1 mg/mL, at least 2.5 mg/mL, at least 5 mg/mL, at least 8 mg/mL, at least 10 mg/mL, at least 15 mg/mL, at least 25 mg/mL, at least 50 mg/mL, at least 75 mg/mL, or at least 100 mg/mL.

The bispecific antibody or the pharmaceutical composition of the present invention may be administered by any suitable method known in the art, including, but not limited to, oral, buccal, sublingual, ocular, topical, parenteral, rectal, intrathecal, intracisternal, inguinal, intravesical, topical (e.g., powder, ointment, or drop), or nasal route. However, for many therapeutic uses, the preferred route/mode of administration is parenteral (such as intravenous injection, subcutaneous injection, intraperitoneal injection, and intramuscular injection). Those skilled in the art will appreciate that the route and/or mode of administration will vary depending on the intended purpose. In a preferred embodiment, the bispecific antibody or the pharmaceutical composition of the present invention is administered by intravenous infusion or injection.

The bispecific antibody or the pharmaceutical composition provided herein can be used alone or in combination, or used in combination with additional pharmaceutically active agents (e.g., a tumor chemotherapeutic drug). Such an additional pharmaceutically active agent may be administered prior to, concurrently with, or subsequent to the administration of the bispecific antibody of the present invention or the pharmaceutical composition of the present invention. In the present invention, the administration regimen may be adjusted to achieve the optimal desired response (e.g., a therapeutic or prophylactic response). For example, it may be a single administration, may be multiple administrations over a period of time, or may be characterized by reducing or increasing the dose proportionally with the emergency degree of the treatment.

Yet further aspect of the present invention relates to use of the bispecific antibody according to any embodiment of the present invention or the conjugate according to the present invention in the preparation of a medicament for treating and/or preventing a malignant tumor; preferably, the malignant tumor is selected from colon cancer, rectal cancer, lung cancer, liver cancer, ovarian cancer, skin cancer, glioma, melanoma, lymphoma, renal tumor, prostate cancer, bladder cancer, gastrointestinal cancer, breast cancer, brain cancer, cervical cancer, esophageal cancer, microsatellite instability-high (MSI-H) and deficient mismatch repair (dMMR) cancer, urothelial cancer, mesothelioma, endometrial cancer, gastric adenocarcinoma, gastroesophageal junction adenocarcinoma and leukemia;

preferably, the lung cancer is non-small cell lung cancer or small cell lung cancer; preferably, the non-small cell lung cancer is EGFR and/or ALK sensitive mutant non-small cell lung cancer; preferably, the liver cancer is hepatocellular carcinoma;
preferably, the renal tumor is renal cell carcinoma;
preferably, the breast cancer is triple negative breast cancer;
preferably, the urothelial cancer is bladder cancer.

Yet another aspect of the present invention relates to use of the bispecific antibody according to any embodiment of the present invention or the conjugate according to the present invention in the preparation of the following medicaments:

(1)

a medicament or an agent for detecting the level of VEGFA in a sample,
a medicament or an agent for blocking binding of VEGFA to VEGFR2,
a medicament or an agent for down-regulating the activity or level of VEGFA,
a medicament or an agent for relieving the stimulation of VEGFA on vascular endothelial cell proliferation,
a medicament or an agent for inhibiting vascular endothelial cell proliferation, or
a medicament or an agent for blocking tumor angiogenesis;
and/or

(2)

a medicament or an agent for blocking the binding of PD-1 to PD-L1,
a medicament or an agent for down-regulating the activity or level of PD-1,
a medicament or an agent for relieving the immunosuppression of PD-1 in an organism,
a medicament or an agent for promoting IFN-ysecretion in T lymphocytes, or
a medicament or an agent for promoting IL-2 secretion in T lymphocytes.

In the *in vitro* experiment of the present invention, the anti-VEGFA antibody and the anti-VEGFA-anti-PD-1 bispecific antibody both can inhibit HUVEC cell proliferation, and the anti-PD-1 antibody and the anti-VEGFA-anti-PD-1 bispecific antibody both can promote the secretion of IFN-y and/or IL-2 and activate immune reaction.

Yet another aspect of the present invention relates to a method for treating and/or preventing a malignant tumor, which comprises a step of administering to a subject in need thereof an effective amount of the bispecific antibody according to any embodiment of the present invention or the conjugate according to the present invention; preferably, the malignant tumor is selected from colon cancer, rectal cancer, lung cancer, liver cancer, ovarian cancer, skin cancer, glioma, melanoma, lymphoma, renal tumor, prostate cancer, bladder cancer, gastrointestinal cancer, breast cancer, brain cancer, cervical cancer, esophageal cancer, microsatellite instability-high (MSI-H) and deficient mismatch repair (dMMR) cancer, urothelial cancer, mesothelioma, endometrial cancer, gastric adenocarcinoma, gastroesophageal junction adenocarcinoma and leukemia;

preferably, the lung cancer is non-small cell lung cancer or small cell lung cancer; preferably, the non-small cell lung cancer is EGFR and/or ALK sensitive mutant non-small cell lung cancer; preferably, the liver cancer is hepatocellular carcinoma;
preferably, the renal tumor is renal cell carcinoma;
preferably, the breast cancer is triple negative breast cancer;
preferably, the urothelial cancer is bladder cancer.

A typical non-limiting range of a therapeutically or prophylactically effective amount of the bispecific antibody of the present invention is 0.02-50 mg/kg, such as 0.1-50 mg/kg, 0.1-25 mg/kg, or 1-10 mg/kg. It should be noted that the dose may vary with the type and severity of the symptom to be treated. Furthermore, those skilled in the art will appreciate that for any particular patient, the particular administration regimen will be adjusted over time according to the needs of the patient and the professional judgment of the physician; the dose ranges given herein are for illustrative purpose only and do not limit the use or scope of the pharmaceutical composition of the present invention.

In the present invention, the subject may be a mammal, such as a human.

Yet another aspect of the present invention relates to a method *in vivo* or *in vitro* selected from:

(1)

a method for detecting the level of VEGFA in a sample,

a method for blocking the binding of VEGFA to VEGFR2,
a method for down-regulating the activity or level of VEGFA,
a method for relieving the stimulation of VEGFA on vascular endothelial cell proliferation,
a method for preparing a medicament or an agent for inhibiting vascular endothelial cell proliferation, or
a method for blocking tumor angiogenesis;
and/or

(2)

a method for blocking the binding of PD-1 to PD-L1,
a method for down-regulating the activity or level of PD-1,
a method for relieving the immunosuppression of PD-1 in an organism,
a method for promoting IFN-γ secretion in T lymphocytes, or
a method for promoting IL-2 secretion in T lymphocytes.

In one or more embodiments of the present invention, the bispecific antibody or the conjugate is used in the treatment and/or prevention of a malignant tumor; preferably, the malignant tumor is selected from colon cancer, rectal cancer, lung cancer, liver cancer, ovarian cancer, skin cancer, glioma, melanoma, lymphoma, renal tumor, prostate cancer, bladder cancer, gastrointestinal cancer, breast cancer, brain cancer, cervical cancer, esophageal cancer, microsatellite instability-high (MSI-H) and deficient mismatch repair (dMMR) cancer, urothelial cancer, mesothelioma, endometrial cancer, gastric adenocarcinoma, gastroesophageal junction adenocarcinoma and leukemia;

preferably, the lung cancer is non-small cell lung cancer or small cell lung cancer; preferably, the non-small cell lung cancer is EGFR and/or ALK sensitive mutant non-small cell lung cancer;
preferably, the liver cancer is hepatocellular carcinoma;
preferably, the renal tumor is renal cell carcinoma;
preferably, the breast cancer is triple negative breast cancer;
preferably, the urothelial cancer is bladder cancer.

In one or more embodiments of the present invention, the bispecific antibody or the conjugate is used for:

(1)

detecting the level of VEGFA in a sample,
blocking the binding of VEGFA to VEGFR2,
down-regulating the activity or level of VEGFA,
relieving the stimulation of VEGFA on vascular endothelial cell proliferation,
inhibiting vascular endothelial cell proliferation, or
blocking tumor angiogenesis;
and/or

(2)

blocking the binding of PD-1 to PD-L1,
down-regulating the activity or level of PD-1,
relieving the immunosuppression of PD-1 in an organism,
promoting IFN-y secretion in T lymphocytes, or
promoting IL-2 secretion in T lymphocytes.

Antibody drugs, especially monoclonal antibodies, have achieved good efficacy in the treatment of various diseases. Traditional experimental methods for acquiring these therapeutic antibodies are to immunize animals with the antigen and acquire antibodies targeting the antigen in the immunized animals, or to improve those antibodies with lower affinity for the antigen by affinity maturation.

The variable regions of the light chain and the heavy chain determine the binding of the antigen; the variable region of each chain contains three hypervariable regions called complementarity determining regions (CDRs). CDRs of the heavy chain (H Chain) comprise HCDR1, HCDR2, and HCDR3, and CDRs of the light chain (L Chain) comprise LCDR1, LCDR2, and LCDR3, which are named by Kabat et al., see Bethesda M.d., Sequences of Proteins of Immunological Interest, Fifth Edition, NIH Publication 1991, (1-3): 91-3242.

Preferably, CDRs may also be defined by the IMGT numbering system, see Ehrenmann, Francois, Quentin Kaas, and Marie-Paule Lefranc. "IMGT/3Dstructure-DB and IMGT/DomainGapAlign: a database and a tool for immunoglobulins or antibodies, T cell receptors, MHC, IgSF and MhcSF." Nucleic acids research 38.suppl_1 (2009): D301-D307. The amino acid sequences of the CDR regions of the monoclonal antibody sequences in (1) to (13) below are analyzed by technical means well known to those skilled in the art, for example by VBASE2 database and according to the IMGT definition, and the results are as follows:

(1) Bevacizumab

The amino acid sequence of the heavy chain variable region is set forth in SEQ ID NO: 1, and the amino acid sequence of the light chain variable region is set forth in SEQ ID NO: 3.
The amino acid sequences of the 3 CDR regions of the heavy chain variable region are as follows:

HCDR1: GYTFTNYG (SEQ ID NO: 28)
HCDR2: INTYTGEP (SEQ ID NO: 29)
HCDR3: AKYPHYYGSSHWYFDV (SEQ ID NO: 30)

the amino acid sequences of the 3 CDR regions of the light chain variable region are as follows:

LCDR1: QDISNY (SEQ ID NO: 31)
LCDR2: FTS (SEQ ID NO: 32)
LCDR3: QQYSTVPWT (SEQ ID NO: 33)

(2) 14C12, 14C12H1L1 or 14C12H1L1 (M)

The amino acid sequences of the 3 CDR regions of the heavy chain variable region are as follows:

HCDR1: GFAFSSYD (SEQ ID NO: 34)
HCDR2: ISGGGRYT (SEQ ID NO: 35)
HCDR3: ANRYGEAWFAY (SEQ ID NO: 36)

the amino acid sequences of the 3 CDR regions of the light chain variable region are as follows:

LCDR1: QDINTY (SEQ ID NO: 37)
LCDR2: RAN (SEQ ID NO: 38)
LCDR3: LQYDEFPLT (SEQ ID NO: 39)

(3) VP101 (hG1WT) or VP101 (hG1DM)

The amino acid sequences of the 9 CDR regions of its heavy chains are as follows:

HCDR1: GYTFTNYG (SEQ ID NO: 28)
HCDR2: INTYTGEP (SEQ ID NO: 29)
HCDR3: AKYPHYYGSSHWYFDV (SEQ ID NO: 30)
HCDR4: GFAFSSYD (SEQ ID NO: 34)
HCDR5: ISGGGRYT (SEQ ID NO: 35)
HCDR6: ANRYGEAWFAY (SEQ ID NO: 36)
HCDR7: QDINTY (SEQ ID NO: 37)
HCDR8: RAN (SEQ ID NO: 38)
HCDR9: LQYDEFPLT (SEQ ID NO: 39)

the amino acid sequences of the 3 CDR regions of the light chain variable region are as follows:

LCDR1: QDISNY (SEQ ID NO: 31)
LCDR2: FTS (SEQ ID NO: 32)
LCDR3: QQYSTVPWT (SEQ ID NO: 33).

For the antibody VP101 (hG1DM) of the present invention, amino acid mutations are introduced into the non-variable region of VP101 (hG1WT). According to the EU numbering system, amino acid mutations are introduced at positions 234 and 235:

VP101 (hG1DM) is obtained by introducing a leucine-to-alanine point mutation at position 234 (L234A) and a leucine-to-alanine point mutation at position 235 (L235A) in the hinge region of the heavy chain.

In the present invention, unless otherwise defined, the scientific and technical terms used herein have the meanings generally understood by those skilled in the art. In addition, the laboratory operations of cell culture, molecular genetics, nucleic acid chemistry and immunology used herein are the routine procedures widely used in the corresponding fields. Meanwhile, in order to better understand the present invention, the definitions and explanations of the relevant terms are provided below.

As used herein, when referring to the amino acid sequence of VEGFA protein (GenBank ID: NP_001165097.1), it includes the full length of the VEGFA protein, as well as a fusion protein of VEGFA, such as a fragment fused to an Fc protein fragment of mouse or human IgG (mFc or hFc). However, those skilled in the art will appreciate that in the amino acid sequence of the VEGFA protein, mutations or variations (including but not limited to, substitutions, deletions and/or additions) can be naturally generated or artificially introduced without affecting biological functions thereof. Therefore, in the present invention, the term "VEGFA protein" should include all such sequences, including their natural or artificial variants. In addition, when describing the sequence fragment of the VEGFA protein, it also includes the corresponding sequence fragments in its natural or artificial variants. In one embodiment of the present invention, the amino acid sequence of the VEGFA protein is shown as the underlined part of SEQ ID NO: 33 (without the last 6 His, a total of 302 amino acids).

As used herein, when referring to the amino acid sequence of VEGFR2 protein (also known as KDR, GenBank ID: NP_002244), it includes the full length of the VEGFR2 protein, or the extracellular fragment VEGFR2-ECD of VEGFR2, or a fragment comprising VEGFR2-ECD, and it also includes a fusion protein of VEGFR2-ECD, such as a fragment fused to an Fc protein fragment of mouse or human IgG (mFc or hFc). However, those skilled in the art will appreciate that in the amino acid sequence of the VEGFR2 protein, mutations or variations (including but not limited to, substitutions, deletions and/or additions) can be naturally generated or artificially introduced without affecting biological functions thereof. Therefore, in the present invention, the term "VEGFR2 protein" should include all such sequences, including their natural or artificial variants. In addition, when describing the sequence fragment of the VEGFR2 protein, it also includes the corresponding sequence fragments in its natural or artificial variants. In one embodiment of the present invention, the amino acid sequence of the extracellular fragment VEGFR2-ECD of VEGFR2 is set forth in SEQ ID NO: 34 (766 amino acids).

As used herein, unless otherwise specified, the VEGFR is VEGFR1 and/or VEGFR2; specific protein sequence thereof is a sequence known in the prior art, and reference may be made to the sequence disclosed in the existing literature or GenBank. For example, VEGFR1 (VEGFR1, NCBI Gene ID: 2321); VEGFR2 (VEGFR2, NCBI Gene ID: 3791).

As used herein, when referring to the amino acid sequence of PD-1 protein (programmed cell death protein 1, NCBI GenBank: NM_005018), it includes the full length of the PD-1 protein, or the extracellular fragment PD-1ECD of PD-1 or a fragment comprising PD-1ECD, and it further includes a fusion protein of PD-1ECD, such as a fragment fused to an Fc protein fragment of a mouse or human IgG (mFc or hFc). However, it will be appreciated by those skilled in the art that in the amino acid sequence of PD-1 protein, mutations or variations (including but not limited to substitutions, deletions and/or additions) can be naturally generated or artificially introduced without affecting biological functions thereof. Therefore, in the present invention, the term "PD-1 protein" should include all such sequences, including their natural or artificial variants. In addition, when describing the sequence fragment of the PD-1 protein, it also includes the corresponding sequence fragments in its natural or artificial variants.

As used herein, the term $EC_{50}$ refers to the concentration for 50% of maximal effect, i.e., the concentration that can cause 50% of the maximal effect.

As used herein, the term "antibody" refers to an immunoglobulin molecule that generally consists of two pairs of polypeptide chains (each pair with one "light" (L) chain and one "heavy" (H) chain). In a general sense, the heavy chain can be interpreted as a polypeptide chain with a larger molecular weight in an antibody, and the light chain refers to a polypeptide chain with a smaller molecular weight in an antibody. Light chains are classified as κ and λ light chains. Heavy chains are generally classified as μ, δ, γ, α, or ε, and isotypes of antibodies are defined as IgM, IgD, IgG, IgA, and IgE, respectively. In light chains and heavy chains, the variable region and constant region are linked by a "J" region of about 12 or more amino acids, and the heavy chain also comprises a "D" region of about 3 or more amino acids. Each heavy chain consists of a heavy chain variable region ($V_H$) and a heavy chain constant region ($C_H$). The heavy chain constant region consists of 3 domains (Cui, $C_{H2}$, and $C_{H3}$). Each light chain consists of a light chain variable region ($V_L$) and a light chain constant region ($C_L$). The light chain constant region consists of one domain $C_L$. The constant region of the antibody can mediate the binding of immunoglobulins to host tissues or factors, including the binding of various cells of the immune system (e.g., effector cells) to the first component (C1q) of classical complement system. The $V_H$ and $V_L$ regions can be further subdivided into highly variable regions (called complementarity determining regions (CDRs)), between which conservative regions called framework regions (FRs) are distributed. Each $V_H$ and $V_L$ consists of 3 CDRs and 4 FRs arranged from amino terminus to carboxyl terminus in the following order: FR1, CDR1, FR2, CDR2, FR3, CDR3, FR4. The variable regions ($V_H$ and $V_L$) of each heavy chain/light chain pair form an antibody binding site. The assignment of amino acids to the regions or domains may be based on Kabat Sequences of Proteins of Immunological Interest (National Institutes of Health, Bethesda, Md. (1987 and 1991)), or Chothia & Lesk J. Mol. Biol. 196(1987): 901-917; Chothia et al. Nature 342(1989): 878-883 or the definition of IMGT numbering system, see Ehrenmann, Francois, Quentin Kaas, and Marie-Paule Lefranc. "IMGT/3Dstructure-DB and IMGT/DomainGapAlign: a database and a tool for immunoglobulins or antibodies, Tcell receptors, MHC, IgSF and MhcSF." Nucleic acids research 38.suppl_1 (2009): D301-D307. In particular, the heavy chain may also comprise more than 3 CDRs, such as 6, 9, or 12. For example, in the bispecific antibody of the present invention, the heavy chain may be a ScFv with the C-terminus of the heavy chain of IgG antibody linked to another antibody, and in this case, the heavy chain comprises 9 CDRs. The term "antibody" is not limited by any specific method for producing antibody. For example, the antibody includes, in particular, a recombinant antibody, a monoclonal antibody, and a polyclonal antibody. The antibody can be antibodies of different isotypes, such as IgG (e.g., subtype IgG1, IgG2, IgG3 or IgG4), IgA1, IgA2, IgD, IgE or IgM.

As used herein, the term "antigen binding fragment", also known as the "antigen binding portion", refers to a polypeptide comprising the fragment of a full-length antibody, which maintains the ability to specifically bind to the same antigen to which the full-length antibody binds, and/or competes with the full-length antibody for the specific binding to an antigen. See generally, Fundamental Immunology, Ch. 7 (Paul, W., ed., 2nd edition, Raven Press, N.Y. (1989). An antigen-binding fragment of an antibody can be produced by recombinant DNA technique or by enzymatic or chemical cleavage of an intact antibody. In some cases, the antigen binding fragment includes Fab, Fab', $F(ab')_2$, Fd, Fv, dAb and complementarity determining region (CDR) fragment, single chain antibody fragment (e.g., scFv), chimeric antibody, diabody and polypeptide that comprises at least a portion of an antibody sufficient to impart specific antigen binding ability to a polypeptide.

As used herein, the term "Fd fragment" refers to an antibody fragment consisting of $V_H$ and $C_{H1}$ domains; the term "Fv fragment" refers to an antibody fragment consisting of the $V_L$ and $V_H$ domains of a single arm of an antibody; the term "dAb fragment" refers to an antibody fragment consisting of a $V_H$ domain (Ward et al., Nature 341 (1989): 544-546); the term "Fab fragment" refers to an antibody fragment consisting of $V_L$, $V_H$, $C_L$ and $C_{H1}$ domains; and the term "$F(ab')_2$ fragment" refers to an antibody fragment comprising two Fab fragments linked by the disulfide bridge on a hinge region.

In some cases, the antigen binding fragment of the antibody is a single chain antibody (e.g., scFv) in which the $V_L$ and $V_H$ domains are paired to form a monovalent molecule via a linker that enables them to produce a single polypeptide chain (see, e.g., Bird et al., Science 242 (1988): 423-426 and Huston et al., Proc. Natl. Acad. Sci. USA 85 (1988): 5879-5883). Such scFv molecules may have a general structure: $NH_2$-$V_L$-linker-$V_H$-COOH or $NH_2$-$V_H$-linker-$V_L$-COOH. An appropriate linker in prior art consists of a repeating GGGGS amino acid sequence or a variant thereof. For example, a linker having the amino acid sequence $(GGGGS)_4$ can be used, and variants thereof can also be used (Holliger et al., Proc. Natl. Acad. Sci. USA 90 (1993): 6444-6448). Other linkers useful in the present invention are described by Alfthan et al., Protein Eng. 8 (1995): 725-731, Choi et al., Eur. J. Immunol., 31 (2001): 94-106, Hu et al., Cancer Res. 56 (1996): 3055-3061, Kipriyanov et al., J. Mol. Biol., 293 (1999): 41-56, and Roovers et al., Cancer Immunol. (2001).

In some cases, the antigen binding fragment of the antibody is a diabody, that is, a bivalent antibody, in which the $V_H$ and $V_L$ domains are expressed on a single polypeptide chain. However, the linker used is too short to allow the pairing of the two domains on the same chain, thereby the domains are forced to pair with the complementary domains on the other chain and two antigen binding sites are generated (see, e.g., Holliger P. et al., Proc. Natl. Acad. Sci. USA 90 (1993): 6444-6448, and Poljak R. J. et al., Structure 2 (1994): 1121-1123).

Antigen binding fragments (e.g., the above mentioned antibody fragments) of antibodies can be obtained from given antibodies by using conventional techniques known to those skilled in the art (e.g., recombinant DNA technique, or enzymatic or chemical cleavage), and the antigen binding fragments of the antibodies are screened for specificity in the same way as for intact antibodies.

As used herein, unless otherwise clearly defined in the context, when referring to the term "antibody", it includes not only intact antibodies but also antigen binding fragments of antibodies. As used herein, the terms "mAb" and "monoclonal antibody" refer to an antibody or a fragment thereof that is derived from a group of highly homologous antibodies, i.e., from a group of identical antibody molecules, except for natural mutations that may occur spontaneously. The monoclonal antibody is highly specific for a single epitope on an antigen. The polyclonal antibody, relative to the monoclonal antibody, generally comprises at least two or more different antibodies which generally recognize different epitopes on an antigen. Monoclonal antibodies can generally be obtained by hybridoma technique first reported by Kohler et al. (Nature, 256: 495, 1975), and can also be obtained by recombinant DNA technique (for example, see U.S. Patent No. 4,816,567).

As used herein, the term "chimeric antibody" refers to an antibody of which a part of the light or/and heavy chains is derived from an antibody (which may be derived from a specific species or belong to a specific antibody class or subclass), and the other part of the light or/and heavy chains are derived from another antibody (which may be derived from the same or different species or belong to the same or different antibody class or subclass). But in any case, it retains the binding activity for the target antigen (U.S. Patent 4,816,567, Cabilly et al.; Morrison et al., Proc. Natl. Acad. Sci. USA, 81 (1984): 6851-6855).

As used herein, the term "humanized antibody" refers to an antibody or antibody fragment obtained when all or a part of CDR regions of a human immunoglobulin (receptor antibody) are replaced by the CDR regions of a non-human antibody (donor antibody), wherein the donor antibody may be a non-human (e.g., mouse, rat or rabbit) antibody having expected specificity, affinity or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, e.g., Jones et al., Nature, 1986, 321: 522-525; Reichmann et al., Nature, 1988, 332: 323 329; Presta, Curr. Op. Struct. Biol., 1992, 2: 593-596, and Clark, Immunol. Today 2000, 21: 397-402.

As used herein, the term "epitope" refers to a site on the antigen that an immunoglobulin or antibody specifically binds to. "Epitope" is also referred to in the field as an "antigenic determinant". The epitope or antigenic determinant generally consists of chemically active surface groups of molecules such as amino acids, carbohydrates or sugar side chains, and usually has specific three-dimensional structural characteristics and specific charge characteristics. For example, the epitope generally comprises at least 3, 4, 5, 6, 7, 8, 9, 10, 11, 12, 13, 14, or 15 consecutive or non-consecutive amino acids in a unique spatial conformation, which can be "linear" or "conformational". See, e.g., Epitope Mapping Protocols in Methods in Molecular Biology, Vol. 66, G. E. Morris, Ed. (1996). In a linear epitope, all interaction sites between a protein and an interaction molecule (e.g., an antibody) are located linearly along the primary amino acid sequence of the protein. In a conformational epitope, the interaction sites are located across amino acid residues of a protein that are separated from each other.

As used herein, the term "isolated" refers to obtaining by artificial means from natural state. If a certain "isolated" substance or component appears in nature, it may be the case that change occurs in its natural environment, or that it is isolated from the natural environment, or both. For example, a certain non-isolated polynucleotide or polypeptide naturally occurs in a certain living animal, and the same polynucleotide or polypeptide with high purity isolated in such a natural state is referred to as an isolated polynucleotide or polypeptide. The term "isolated" does not exclude the existence of artificial or synthetic substances or other impurities that do not affect the activity of the substance.

As used herein, the term "vector" refers to a nucleic acid vehicle into which a polynucleotide can be inserted. When a vector allows for the expression of the protein encoded by the inserted polynucleotide, the vector is referred to as an expression vector. The vector can be introduced into a host cell by transformation, transduction, or transfection so that the genetic substance elements carried by the vector can be expressed in the host cell. Vectors are well known to those skilled in the art, including but not limited to: plasmids; phagemids; cosmids; artificial chromosomes, such as yeast artificial chromosome (YAC), bacterial artificial chromosome (BAC), or P1-derived artificial chromosome (PAC); phages such as lambda phages or M13 phages; and animal viruses. Animal viruses that can be used as vectors include, but are not limited to retroviruses (including lentiviruses), adenoviruses, adeno-associated viruses, herpes viruses (such as herpes simplex virus), poxviruses, baculoviruses, papillomaviruses, and papovaviruses (such as SV40). A vector may comprise a variety of elements that control expression, including, but not limited to promoter sequences, transcription initiation sequences, enhancer sequences, selection elements, and reporter genes. In addition, the vector may further comprise a replication initiation site.

As used herein, the term "host cell" refers to cells to which vectors can be introduced, including, but not limited to, prokaryotic cells such as *E. coli* or *bacillus subtilis,* fungal cells such as yeast cells or *aspergillus,* insect cells such as S2 drosophila cells or Sf9, or animal cells such as fibroblasts, CHO cells, COS cells, NSO cells, HeLa cells, BHK cells, HEK 293 cells, or human cells.

As used herein, the term "specifically bind" refers to a non-random binding reaction between two molecules, such as a reaction between an antibody and an antigen it targets. In some embodiments, an antibody that specifically binds to an antigen (or an antibody that is specific for an antigen) refers to that the antibody binds to the antigen with an affinity ($K_D$) of less than about $10^{-5}$ M, such as less than about $10^{-6}$ M, $10^{-7}$ M, $10^{-8}$ M, $10^{-9}$ M or $10^{-10}$ M or less. In some embodiments of the present invention, the term "target" refers to specific binding.

As used herein, the term "$K_D$" refers to a dissociation equilibrium constant for a specific antibody-antigen interaction, which is used to describe the binding affinity between the antibody and the antigen. A smaller equilibrium dissociation constant indicates a stronger antibody-antigen binding and a higher affinity between the antibody and the antigen. Generally, an antibody binds to an antigen with a dissociation equilibrium constant ($K_D$) of less than about $10^{-5}$ M, such as less than about $10^{-6}$ M, $10^{-7}$ M, $10^{-8}$ M, $10^{-9}$ M or $10^{-10}$ M or less, for example, as determined on a BIACORE surface plasmon resonance (SPR) instrument or a Fortebio system.

As used herein, the terms "monoclonal antibody" and "mAb" have the same meaning and can be used interchangeably; the terms "polyclonal antibody" and "pAb" have the same meaning and can be used interchangeably; the terms "polypeptide" and "protein" have the same meaning and can be used interchangeably. Besides, herein, amino acids are generally represented by single-letter and three-letter abbreviations known in the art. For example, alanine can be represented by A or Ala.

As used herein, the term "pharmaceutically acceptable auxiliary material" refers to a vector and/or excipient that is pharmacologically and/or physiologically compatible with the subject and the active ingredient, which is well known in the art (see, e.g., Remington's Pharmaceutical Sciences. Edited by Gennaro AR, 19th ed. Pennsylvania: Mack Publishing Company, 1995) and includes, but is not limited to, pH regulators, surfactants, adjuvants, and ionic strength enhancers. For example, the pH regulators include, but are not limited to, phosphate buffer; the surfactants include, but are not limited to, cationic, anionic, or non-ionic surfactants, such as Tween-80; the ionic strength enhancers include, but are not limited to, sodium chloride.

As used herein, the term "adjuvant" refers to a non-specific immune enhancer, which can enhance the immune response of an organism to antigens or change the type of immune response when delivered into the organism together with the antigens or in advance. There are various adjuvants, including, but not limited to, aluminum adjuvant (e.g., aluminum hydroxide), Freund's adjuvant (e.g., complete Freund's adjuvant and incomplete Freund's adjuvant), *Corynebacterium parvum,* lipopolysaccharide, cytokine, etc. The Freund's adjuvant is the most commonly used adjuvant in animal experiments. The aluminum hydroxide adjuvant is used more frequently in clinical trials. As used herein, the term "effective amount" refers to an amount sufficient to obtain or at least partially obtain desired effects. For example, a prophylactically effective amount (e.g., for a disease associated with PD-1 binding to PD-L1 or overexpression of VEGF, such as a tumor) is an amount sufficient to prevent, stop, or delay the onset of the disease (e.g., a disease associated with PD-1 binding to PD-L1 or overexpression of VEGF, such as a tumor); a therapeutically effective amount is an amount sufficient to cure or at least partially stop the disease and its complications in a patient suffering from the disease. It is undoubtedly within the ability of those skilled in the art to determine such an effective amount. For example, the amount effective for therapeutic purpose will depend on the severity of the disease to be treated, the overall state of the patient's own immune system, the general condition of the patient such as age, body weight and gender, the route of administration, and other treatments given concurrently, etc.

The term "MSI" refers to microsatellite instability. Microsatellites are short tandem repeats throughout the human genome, including 10-50 repeats of one, two or more nucleotides. Microsatellites in certain abnormal cells, such as tumors, are altered in length by insertion or deletion of repeat units as compared to normal cells. Such alteration is referred to as MSI. Based on instability and extent, MSI can be classified as microsatellite instability-high (MSI-H), microsatellite instability-low (MSI-L) and microsatellite stable (MSS). The major cause of MSI is DNA mismatch repair (MMR) deficiency. Human mismatch repair genes (MMR genes) can express corresponding mismatch repair proteins through transcription and translation. Absence of any MMR protein may lead to mismatch repair deficiency, and basepair mismatch will accumulate in the process of DNA replication due to such deficiency, ultimately resulting in MSI. About 15% of colorectal cancers are attributed to the MSI pathway. This was first reported in colorectal cancer, and may also occur in gastric cancer, endometrial cancer, adrenocortical carcinoma and the like (Baretti M et al., Pharmacol Ther., 2018; 189: 45-62). MSI-H/dMMR characteristics were also found in mesothelioma, sarcoma, adrenocortical carcinoma, malignant melanoma and ovarian germ cell neoplasm in subsequent studies.

MSI-H and dMMR represent the results of two different assays and are biologically consistent, called MSI-H/dMMR or MSI-high/dMMR, while MSI-L and MSS are phenotypes of proficient mismatch repair (pMMR). The detection of dMMR is to perform an immunohistochemical assay of protein expression for four mismatch genes of MSH2, MLH1, MSH6 and PMS2 based on tumor specimens (including surgical specimens and aspiration specimens). Absence of any of the four proteins confirms the dMMR; positive results of all the four proteins indicate pMMR, i.e., a complete mismatch repair function. The detection of MSI is to match the length of the repeated DNA sequences (microsatellite sequences) in tumor cells and somatic cells, and to compare the lengths. When 5 standard loci are detected using PCR based on the American NCI standard, inconsistencies in two or more loci indicate instability, defined as MSI-H, one inconsistent locus indicates MSI-L, and 5 consistent loci indicate MSS. High-throughput sequencing (also referred to as next-generation sequencing, or NGS) can also be used as a method for detecting microsatellite instability. When more microsatellite loci are selected, such as more than 5 loci or additional microsatellite loci, for PCR assay, inconsistency in ≥30% loci is defined as MSI-H, consistency in all loci is defined as MSS, and inconsistency between 0 and 30% is defined as MSI-L.

Beneficial Effects

The present invention achieves one or more of the following technical effects (1) to (4):

(1) The modification of the Fc fragment of the antibody of the present invention completely eliminates the binding activity of VP101(hG1WT) and $F_C$ receptors FcyRI and FcγRIIIa_F158, thereby completely eliminating the ADCC activity.

(2) The modification of the Fc fragment of the antibody of the present invention completely eliminates the binding activity of VP101(hG1WT) and the complement C1q, thereby completely eliminating the CDC activity.

(3) The bispecific antibody of the present invention can specifically bind to VEGFA well, can effectively block the binding of VEGFA to VEGFR2, and specifically relieves the immunosuppression of VEGFA in an organism and the promotion effect of VEGFA on angiogenesis.

(4) The bispecific antibody of the present invention can specifically bind to PD-1 well, can effectively block the binding of PD-1 to PD-L1, and specifically relieves the immunosuppression of PD-1 in an organism and activate the immune response.

BRIEF DESCRIPTION OF THE DRAWINGS

FIG. 1: Affinity constant assay of VP101(hG1DM) to FcyRI. The antibody concentrations for the curve pairs from top to bottom are 50 nM, 25 nM, 12.5 nM, 6.25 nM and 3.12 nM, respectively.

FIG. 2: Affinity constant assay of bevacizumab to FcyRI. The antibody concentrations for the curve pairs from top to bottom are 50 nM, 25 nM, 12.5 nM, 6.25 nM and 3.12 nM, respectively.

FIG. 3: Affinity constant assay of nivolumab to FcyRI. The antibody concentrations for the curve pairs from top to bottom are 50 nM, 25 nM, 12.5 nM, 6.25 nM and 3.12 nM, respectively.

FIG. 4: Affinity constant assay of VP101(hGlWT) to FcyRI. The antibody concentrations for the curve pairs from top to bottom are 50 nM, 25 nM, 12.5 nM, 6.25 nM and 3.12 nM, respectively.

FIG. 5: Affinity constant assay of VP101(hG4WT) to FcyRI. The antibody concentrations for the curve pairs from top to bottom are 50 nM, 25 nM, 12.5 nM, 6.25 nM and 3.12 nM, respectively.

FIG. 6: Affinity constant assay of VP101(hG1DM) to FcγRIIa_H131. The antibody concentrations for the curve pairs from top to bottom are 200 nM, 100 nM, 50 nM, 25 nM and 12.5 nM, respectively.

FIG. 7: Affinity constant assay of bevacizumab to FcγRIIa_H131. The antibody concentrations for the curve pairs from top to bottom are 200 nM, 100 nM, 50 nM, 25 nM and 12.5 nM, respectively.

FIG. 8: Affinity constant assay of nivolumab to FcγRIIa_H131. The antibody concentrations for the curve pairs from top to bottom are 200 nM, 100 nM, 50 nM, 25 nM and 12.5 nM, respectively.

FIG. 9: Affinity constant assay of VP101(hG1WT) to FcγRIIa_H131. The antibody concentrations for the curve pairs from top to bottom are 200 nM, 100 nM, 50 nM, 25 nM and 12.5 nM, respectively.

FIG. 10: Affinity constant assay of VP101(hG4WT) to FcγRIIa_H131. The antibody concentrations for the curve pairs from top to bottom are 200 nM, 100 nM, 50 nM, 25 nM and 12.5 nM, respectively.

FIG. 11: Affinity constant assay of VP101(hG1DM) to FcγRIIa_R131. The antibody concentrations for the curve pairs from top to bottom are 200 nM, 100 nM, 50 nM, 25 nM and 12.5 nM, respectively.

FIG. 12: Affinity constant assay of bevacizumab to FcγRIIa_R131. The antibody concentrations for the curve pairs from top to bottom are 200 nM, 100 nM, 50 nM, 25 nM and 12.5 nM, respectively.

FIG. 13: Affinity constant assay of nivolumab to FcγRIIa_R131. The antibody concentrations for the curve pairs from top to bottom are 200 nM, 100 nM, 50 nM, 25 nM and 12.5 nM, respectively.

FIG. 14: Affinity constant assay of VP101(hG1WT) to FcγRIIa_R131. The antibody concentrations for the curve pairs from top to bottom are 200 nM, 100 nM, 50 nM, 25 nM and 12.5 nM, respectively.

FIG. 15: Affinity constant assay of VP101(hG4WT) to FcγRIIa_R131. The antibody concentrations for the curve pairs from top to bottom are 200 nM, 100 nM, 50 nM, 25 nM and 12.5 nM, respectively.

FIG. 16: Affinity constant assay of VP101(hG1DM) to FcγRIIIa_V158. The antibody concentrations for the curve pairs from top to bottom are 500 nM, 250 nM, 125 nM, 62.5 nM and 31.25 nM, respectively.

FIG. 17: Affinity constant assay of bevacizumab to FcγRIIIa_V158. The antibody concentrations for the curve pairs from top to bottom are 500 nM, 250 nM, 125 nM, 62.5 nM and 31.25 nM, respectively.

FIG. 18: Affinity constant assay of nivolumab to FcγRIIIa_V158. The antibody concentrations for the curve pairs from top to bottom are 500 nM, 250 nM, 125 nM, 62.5 nM and 31.25 nM, respectively.

FIG. 19: Affinity constant assay of VP101(hG1WT) to FcγRIIIa_V158. The antibody concentrations for the curve pairs from top to bottom are 500 nM, 250 nM, 125 nM, 62.5 nM and 31.25 nM, respectively.

FIG. 20: Affinity constant assay of VP101(hG4WT) to FcγRIIIa_V158. The antibody concentrations for the curve pairs from top to bottom are 500 nM, 250 nM, 125 nM, 62.5 nM and 31.25 nM, respectively.

FIG. 21: Affinity constant assay of VP101(hG1DM) to FcγRIIIa_F158. The antigen concentrations for the curve pairs from top to bottom are 500 nM, 250 nM, 125 nM, 62.5 nM and 31.25 nM, respectively.

FIG. 22: Affinity constant assay of bevacizumab to FcyRIIIa F158. The antibody concentrations for the curve pairs from top to bottom are 500 nM, 250 nM, 125 nM, 62.5 nM and 31.25 nM, respectively.

FIG. 23: Affinity constant assay of nivolumab to FcγRIIIa_F158. The antibody concentrations for the curve pairs from top to bottom are 500 nM, 250 nM, 125 nM, 62.5 nM and 31.25 nM, respectively.

FIG. 24: Affinity constant assay of VP101(hG1WT) to FcγRIIIa_F158. The antibody concentrations for the curve pairs from top to bottom are 500 nM, 250 nM, 125 nM, 62.5 nM and 31.25 nM, respectively.

FIG. 25: Affinity constant assay of VP101(hG4WT) to FcγRIIa_F158. The antibody concentrations for the curve pairs from top to bottom are 500 nM, 250 nM, 125 nM, 62.5 nM and 31.25 nM, respectively.

FIG. 26: Affinity constant assay of VP101(hG1DM) to C1q. The antibody concentrations for the curve pairs from top to bottom are 10 nM, 5 nM, 2.5 nM, 1.25 nM and 0.625 nM, respectively.

FIG. 27: Affinity constant assay of bevacizumab to C1q. The antibody concentrations for the curve pairs from top to bottom are 10 nM, 5 nM, 2.5 nM, 1.25 nM and 0.625 nM, respectively.

FIG. 28: Affinity constant assay of nivolumab to C1q. The antibody concentrations for the curve pairs from top to bottom are 10 nM, 5 nM, 2.5 nM, 1.25 nM and 0.625 nM, respectively.

FIG. 29: Affinity constant assay of VP101(hG1WT) to C1q. The antibody concentrations for the curve pairs from top to bottom are 10 nM, 5 nM, 2.5 nM, 1.25 nM and 0.625 nM, respectively.

FIG. 30: Affinity constant assay of VP101(hG4WT) to C1q. The antigen concentrations for the curve pairs from top to bottom are 10 nM, 5 nM, 2.5 nM, 1.25 nM and 0.625 nM, respectively.

FIG. 31: ADCC activity assay of VP101(hG1WT) and VP101(hG1DM) on a CHO-K1-PD1 target cell system expressing PD-1 antigen.

FIG. 32: CDC activity assay of VP101(hG1WT) and VP101(hG1DM) on a CHO-K1-PD1 target cell system expressing PD-1 antigen.

FIG. 33: Effects of the antibody VP101(hG1DM) on the secretion of cytokine IFN-y induced by PBMC and Raji-PDL1 cell mixed culture as detected by ELISA method.

FIG. 34: Effects of the antibody VP101(hG1DM) on the secretion of cytokine IL-2 induced by PBMC and Raji-PDL1 cell mixed culture as detected by ELISA method.

FIG. 35: ADCP activity assay of VP101(hG1DM) on a CHO-K1-PD1 target cell system expressing PD-1 antigen.

## DETAILED DESCRIPTION

The embodiments of the present invention will be described in detail below with reference to the examples. Those skilled in the art will understand that the following examples are only for illustrating the present invention, and should not be construed as limitations on the scope of the present invention. The cases without the specific descriptions of techniques or conditions were carried out according to the technologies or conditions described in the literature in the art (e.g., see, Guide to Molecular Cloning Experiments, authored by J. Sambrook et al., and translated by Huang Peitang et al., third edition, Science Press) or according to the product manual. Reagents or instruments used are all commercially available conventional products if the manufacturers thereof are not specified.

In the following examples of the present invention, the marketed antibody bevacizumab (trade name Avastin®) for the same target was purchased from Roche as a control antibody, or was prepared according to Preparation Example 1.

In the following examples of the present invention, the marketed antibody nivolumab for the same target (trade name Opdivo®) was purchased from BMS as a control antibody.

In the following examples of the present invention, the isotype control antibody used is human anti-hen egg lysozyme IgG (anti-HEL, or human IgG, abbreviated as hIgG) whose variable region sequences are derived from the study entitled "Affinity maturation increases the stability and plasticity of the Fv domain of anti-protein antibodies", published by Acierno et al (Acierno et al., J Mol Biol., 2007; 374(1): 130-46). The hIgG1DM and hIgG4WT used in the examples are isotype control antibodies of anti-HEL with hG1DM and hG4WTconstant region sequences. The isotype control antibodies were prepared in the laboratory of Akeso Biopharma, Inc.

### Preparation Example 1: Preparation of Anti-VEGFA Antibody Bevacizumab

Chinese Patent Publication CN1259962A is referred to for the amino acid sequences of the heavy chain variable region and the light chain variable region of the marketed anti-VEGFA monoclonal antibody Avastin (bevacizumab). Genscript was entrusted to synthesize nucleotide sequences encoding the heavy chain variable region and the light chain variable region.

Amino acid sequence of the heavy chain variable region of bevacizumab (bevacizumab-Hv): (123 aa)

EVQLVESGGGLVQPGGSLRLSCAASGYTFTNYGMNWVRQAPGKGLEWVGWINTYTG
EPTYAADFKRRFTFSLDTSKSTAYLQMNSLRAEDTAVYYCAKYPHYYGSSHWYFDVW
GQGTLVTVSS (SEQ ID NO: 1)

Nucleotide sequence encoding the heavy chain variable region of bevacizumab: (369 bp)

GAGGTGCAGCTGGTCGAGTCCGGGGGGGGGCTGGTGCAGCCAGGCGGGTCTCTGA
GGCTGAGTTGCGCCGCTTCAGGGTACACCTTCACAAACTATGGAATGAATTGGGTG
CGCCAGGCACCAGGAAAGGGACTGGAGTGGGTCGGCTGGATCAACACTTACACCG
GGGAACCTACCTATGCAGCCGACTTTAAGCGGCGGTTCACCTTCAGCCTGGATACA
AGCAAATCCACTGCCTACCTGCAGATGAACAGCCTGCGAGCTGAGGACACCGCAGT
CTACTATTGTGCTAAATATCCCCACTACTATGGGAGCAGCCATTGGTATTTTGACGT
GTGGGGGCAGGGGACTCTGGTGACAGTGAGCAGC (SEQ ID NO: 2)

Amino acid sequence of the light chain variable region of bevacizumab (bevacizumab-Lv): (107 aa)

DIQMTQSPSSLSASVGDRVTITCSASQDISNYLNWYQQKPGKAPKVLIYFTSSLHSGVPS
RFSGSGSGTDFTLTISSLQPEDFATYYCQQYSTVPWTFGQGTKVEIK (SEQ ID NO: 3)

Nucleotide sequence encoding the light chain variable region of bevacizumab: (321 bp)

GATATTCAGATGACTCAGAGCCCCTCCTCCCTGTCCGCCTCTGTGGGCGACAGGGTC
ACCATCACATGCAGTGCTTCACAGGATATTTCCAACTACCTGAATTGGTATCAGCAG
AAGCCAGGAAAAGCACCCAAGGTGCTGATCTACTTCACTAGCTCCCTGCACTCAGG
AGTGCCAAGCCGGTTCAGCGGATCCGGATCTGGAACCGACTTTACTCTGACCATTTC
TAGTCTGCAGCCTGAGGATTTCGCTACATACTATTGCCAGCAGTATTCTACCGTGCC
ATGGACATTTGGCCAGGGGACTAAAGTCGAGATCAAG (SEQ ID NO: 4)

The heavy chain constant regions were all Ig gamma-1 chain C region, ACCESSION: P01857; the light chain constant regions were all Ig kappa chain C region, ACCESSION: P01834.

The heavy chain cDNA and the light chain cDNA of bevacizumab were cloned into vector pcDNA3.1, and the recombinant expression plasmid of the antibody bevacizumab was obtained. The recombinant plasmid was transfected into 293F cells. The 293F cell culture medium was purified and then detected.

The anti-VEGFA monoclonal antibody Avastin (bevacizumab) was thus obtained.

Preparation Example 2: Sequence Design of Anti-PD-1 Antibody 14C12 and Humanized Antibody 14C12H1L1 thereof and Mutant 14C12H1L1(M)

The amino acid sequences and encoding nucleotide sequences of the heavy and light chains of anti-PD-1 antibody 14C12 and a humanized antibody 14C12H1L1 thereof are identical to those of 14C12 and 14C12H1L1 in Chinese Patent Publication No. CN106967172A, respectively.

(1) Heavy and light chain variable region sequences of 14C12

Amino acid sequence of the heavy chain variable region of 14C12: (118 aa)

EVKLVESGGGLVKPGGSLKLSCAASGFAFSSYDMSWVRQTPEKRLEWVATISGGGRYT
YYPDSVKGRFTISRDNARNTLYLQMSSLRSEDTALYYCANRYGEAWFAYWGQGTLVT
VSA (SEQ ID NO: 5)

Nucleotide sequence encoding the heavy chain variable region of 14C12: (354 bp)

GAGGTCAAACTGGTGGAGAGCGGCGGCGGGCTGGTGAAGCCCGGCGGGTCACTGA
AACTGAGCTGCGCCGCTTCCGGCTTCGCCTTTAGCTCCTACGACATGTCATGGGTGA
GGCAGACCCCTGAGAAGCGCCTGGAATGGGTCGCTACTATCAGCGGAGGCGGGCG
ATACACCTACTATCCTGACTCTGTCAAAGGGAGATTCACAATTAGTCGGGATAACG
CCAGAAATACTCTGTATCTGCAGATGTCTAGTCTGCGGTCCGAGGATACAGCTCTGT
ACTATTGTGCAAACCGGTACGGCGAAGCATGGTTTGCCTATTGGGGACAGGGCACC
CTGGTGACAGTCTCTGCC (SEQ ID NO: 6)

Amino acid sequence of the light chain variable region of 14C12: (107 aa)

DIKMTQSPSSMYASLGERVTFTCKASQDINTYLSWFQQKPGKSPKTLIYRANRLVDGVP
SRFSGSGSGQDYSLTISSLEYEDMGIYYCLQYDEFPLTFGAGTKLELK (SEQ ID NO: 7)

Nucleotide sequence encoding the light chain variable region of 14C12: (321 bp)

GACATTAAGATGACACAGTCCCCTTCCTCAATGTACGCTAGCCTGGGCGAGCGAGT
GACCTTCACATGCAAAGCATCCCAGGACATCAACACATACCTGTCTTGGTTTCAGCA
GAAGCCAGGCAAAAGCCCCAAGACCCTGATCTACCGGGCCAATAGACTGGTGGAC
GGGGTCCCCAGCAGATTCTCCGGATCTGGCAGTGGGCAGGATTACTCCCTGACCAT
CAGCTCCCTGGAGTATGAAGACATGGGCATCTACTATTGCCTGCAGTATGATGAGTT
CCCTCTGACCTTTGGAGCAGGCACAAAACTGGAACTGAAG (SEQ ID NO: 8)

(2) Heavy and light chain variable region and heavy and light chain sequences of humanized monoclonal antibody 14C12H1L1

Amino acid sequence of the heavy chain variable region of 14C12H1L1: (118 aa)

EVQLVESGGGLVQPGGSLRLSCAASGFAFSSYDMSWVRQAPGKGLDWVATISGGGRY
TYYPDSVKGRFTISRDNSKNNLYLQMNSLRAEDTALYYCANRYGEAWFAYWGQGTLV
TVSS (SEQ ID NO: 9)

Nucleotide sequence encoding the heavy chain variable region of 14C12H1L1: (354 bp)

GAAGTGCAGCTGGTCGAGTCTGGGGGAGGGCTGGTGCAGCCCGGCGGGTCACTGCG ACTGAGCTGCGCAGCTTCCGGATTCGCCTTTAGCTCCTACGACATGTCCTGGGTGCG ACAGGCACCAGGAAAGGGACTGGATTGGGTCGCTACTATCTCAGGAGGCGGGAGA TACACCTACTATCCTGACAGCGTCAAGGGCCGGTTCACAATCTCTAGAGATAACAG TAAGAACAATCTGTATCTGCAGATGAACAGCCTGAGGGCTGAGGACACCGCACTGT ACTATTGTGCCAACCGCTACGGGGAAGCATGGTTTGCCTATTGGGGGCAGGGAACC CTGGTGACAGTCTCTAGT (SEQ ID NO: 10)

Amino acid sequence of the light chain variable region of 14C12H1L1: (107 aa)

DIQMTQSPSSMSASVGDRVTFTCRASQDINTYLSWFQQKPGKSPKTLIYRANRLVSGVP SRFSGSGSGQDYTLTISSLQPEDMATYYCLQYDEFPLTFGAGTKLELK (SEQ ID NO: 11)

Nucleotide sequence encoding the light chain variable region of 14C12H1L1: (321 bp)

GACATTCAGATGACTCAGAGCCCCTCCTCCATGTCCGCCTCTGTGGGCGACAGGGTC ACCTTCACATGCCGCGCTAGTCAGGATATCAACACCTACCTGAGCTGGTTTCAGCAG AAGCCAGGGAAAAGCCCCAAGACACTGATCTACCGGGCTAATAGACTGGTGTCTGG AGTCCCAAGTCGGTTCAGTGGCTCAGGGAGCGGACAGGACTACACTCTGACCATCA GCTCCCTGCAGCCTGAGGACATGGCAACCTACTATTGCCTGCAGTATGATGAGTTCC CACTGACCTTTGGCGCCGGGACAAAACTGGAGCTGAAG (SEQ ID NO: 12)

Amino acid sequence of the heavy chain (14C12H1) of 14C12H1L1: (448 aa)

EVQLVESGGGLVQPGGSLRLSCAASGFAFSSYDMSWVRQAPGKGLDWVATISGGGRY TYYPDSVKGRFTISRDNSKNNLYLQMNSLRAEDTALYYCANRYGEAWFAYWGQGTLV TVSSASTKGPSVFPLAPSSKSTSGGTAALGCLVKDYFPEPVTVSWNSGALTSGVHTFPA VLQSSGLYSLSSVVTVPSSSLGTQTYICNVNHKPSNTKVDKKVEPKSCDKTHTCPPCPAP ELLGGPSVFLFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKAKGQPREPQV YTLPPSRDELTKNQVSLTCLVKGFYPSDIAVEWESNGQPENNYKTTPPVLDSDGSFFLY SKLTVDKSRWQQGNVFSCSVMHEALHNHYTQKSLSLSPGK (SEQ ID NO: 13)

Nucleotide sequence encoding the heavy chain (14C12H1) of 14C12H1L1: (1344 bp)

GAAGTGCAGCTGGTCGAGTCTGGGGGAGGGCTGGTGCAGCCCGGCGGGTCACTGCG ACTGAGCTGCGCAGCTTCCGGATTCGCCTTTAGCTCCTACGACATGTCCTGGGTGCG ACAGGCACCAGGAAAGGGACTGGATTGGGTCGCTACTATCTCAGGAGGCGGGAGA TACACCTACTATCCTGACAGCGTCAAGGGCCGGTTCACAATCTCTAGAGATAACAG TAAGAACAATCTGTATCTGCAGATGAACAGCCTGAGGGCTGAGGACACCGCACTGT ACTATTGTGCCAACCGCTACGGGGAAGCATGGTTTGCCTATTGGGGGCAGGGAACC CTGGTGACAGTCTCTAGTGCCAGCACCAAAGGACCTAGCGTGTTTCCTCTCGCCCCC TCCTCCAAAAGCACCAGCGGAGGAACCGCTGCTCTCGGATGTCTGGTGAAGGACTA CTTCCCTGAACCCGTCACCGTGAGCTGGAATAGCGGCGCTCTGACAAGCGGAGTCC ATACATTCCCTGCTGTGCTGCAAAGCAGCGGACTCTATTCCCTGTCCAGCGTCGTCA CAGTGCCCAGCAGCAGCCTGGGCACCCAGACCTACATCTGTAACGTCAACCACAAG CCCTCCAACACCAAGGTGGACAAGAAAGTGGAGCCCAAATCCTGCGACAAGACAC ACACCTGTCCCCCCTGTCCTGCTCCCGAACTCCTCGGAGGCCCTAGCGTCTTCCTCTT TCCTCCCAAACCCAAGGACACCCTCATGATCAGCAGAACCCCTGAAGTCACCTGTG TCGTCGTGGATGTCAGCCATGAGGACCCCGAGGTGAAATTCAACTGGTATGTCGAT GGCGTCGAGGTGCACAACGCCAAAACCAAGCCCAGGGAGGAACAGTACAACTCCA CCTACAGGGTGGTGTCCGTGCTGACAGTCCTCCACCAGGACTGGCTGAACGGCAAG GAGTACAAGTGCAAGGTGTCCAACAAGGCTCTCCCTGCCCCCATTGAGAAGACCAT CAGCAAGGCCAAAGGCCAACCCAGGGAGCCCCAGGTCTATACACTGCCTCCCTCCA GGGACGAACTCACCAAGAACCAGGTGTCCCTGACCTGCCTGGTCAAGGGCTTTTAT CCCAGCGACATCGCCGTCGAGTGGGAGTCCAACGGACAGCCCGAGAATAACTACA AGACCACCCCTCCTGTCCTCGACTCCGACGGCTCCTTCTTCCTGTACAGCAAGCTGA CCGTGGACAAAAGCAGGTGGCAGCAGGGAAACGTGTTCTCCTGCAGCGTGATGCAC GAAGCCCTCCACAACCACTACACCCAGAAAAGCCTGTCCCTGAGCCCCGGCAAA (SEQ ID NO: 14)

Amino acid sequence of the light chain (14C12L1) of 14C12H1L1: (214 aa)

DIQMTQSPSSMSASVGDRVTFTCRASQDINTYLSWFQQKPGKSPKTLIYRANRLVSGVP SRFSGSGSGQDYTLTISSLQPEDMATYYCLQYDEFPLTFGAGTKLELKRTVAAPSVFIFP PSDEQLKSGTASVVCLLNNFYPREAKVQWKVDNALQSGNSQESVTEQDSKDSTYSLSS TLTLSKADYEKHKVYACEVTHQGLSSPVTKSFNRGEC (SEQ ID NO: 15)

Nucleotide sequence encoding the light chain (14C12L1) of 14C12H1L1: (642 bp)

GACATTCAGATGACTCAGAGCCCCTCCTCCATGTCCGCCTCTGTGGGCGACAGGGTC
ACCTTCACATGCCGCGCTAGTCAGGATATCAACACCTACCTGAGCTGGTTTCAGCAG
AAGCCAGGGAAAAGCCCCAAGACACTGATCTACCGGGCTAATAGACTGGTGTCTGG
AGTCCCAAGTCGGTTCAGTGGCTCAGGGAGCGGACAGGACTACACTCTGACCATCA
GCTCCCTGCAGCCTGAGGACATGGCAACCTACTATTGCCTGCAGTATGATGAGTTCC
CACTGACCTTTGGCGCCGGGACAAAACTGGAGCTGAAGCGAACTGTGGCCGCTCCC
TCCGTCTTCATTTTTCCCCCTTCTGACGAACAGCTGAAATCAGGCACAGCCAGCGTG
GTCTGTCTGCTGAACAATTTCTACCCTAGAGAGGCAAAAGTGCAGTGGAAGGTCGA
TAACGCCCTGCAGTCCGGCAACAGCCAGGAGAGTGTGACTGAACAGGACTCAAAA
GATAGCACCTATTCCCTGTCTAGTACACTGACTCTGTCCAAGGCTGATTACGAGAAG
CACAAAGTGTATGCATGCGAAGTGACACATCAGGGACTGTCAAGCCCCGTGACTAA
GTCTTTTAACCGGGGCGAATGT (SEQ ID NO: 16)

(3) Heavy and light chain variable region sequences of 14C12H1L1(M)

Individual amino acids in the framework region (light chain) were mutated on the basis of 14C12H1L1 to obtain 14C12H1L1(M).

Heavy chain variable region 14C12H1(M) of 14C12H1L1(M):
is identical with the heavy chain variable region 14C12H1 of 14C12H1L1, namely, the amino acid sequence is set forth in SEQ ID NO: 9.

Light chain variable region 14C12L1(M) of 14C12H1L1(M): (108 aa, mutation positions underlined in the amino acid sequence based on 14C12H1L1)

DIQMTQSPSSMSASVGDRVTFTCRASQDINTYLSWFQQKPGKSPKTLIYRANRLVSGVP
SRFSGSGSGQDYTLTISSLQPEDMATYYCLQYDEFPLTFGAGTKLELKR (SEQ ID NO: 17)

Preparation Example 3: Sequence Design of Bispecific Antibodies

1. Sequence design

The structure of the bispecific antibody of the present invention is in the Morrison form (IgG-scFv), i.e., C-termini of two heavy chains of an IgG antibody are each linked to a scFv fragment of another antibody, and the main composition design of the heavy and light chains is as shown in Table 1 below.

On the basis of bevacizumab, the VP101 antibody, in which amino acid sequences of the heavy chain variable region and the light chain variable region of 14C12H1L1(M) are taken as a ScFv fragment part, is referred to as VP101(M). Compared to 14C12H1L1, 14C12H1L1(M) effectively optimized the structure of the bispecific antibody and improved the effectiveness.

Table 1: Compositional Design of Heavy and Light Chains of VP101(M) and VP101(G4M)

| Bispecific antibody No. | Immunoglobulin moiety | | Linker fragment | scFv part |
|---|---|---|---|---|
| | Heavy chain | Light chain | | |
| VP101(M) | Bevacizum ab-H | Bevacizumab-L | Linker1 | 14C12H1(M)v- Linker1-14C12L1(M)v |
| VP101(G4M) | Bevacizum ab-G4H | Bevacizumab-L | Linker1 | 14C12H1(M)v- Linker1 14C12L1(M)v |

In Table 1 above:

(1) Those with "V" labeled at lower right corner refer to the variable region of corresponding heavy chain or the variable region of corresponding light chain. For those without "V" label, the corresponding heavy or light chain is the full length comprising the constant region. The corresponding sequences described in the above preparation examples are referred to for the amino acid sequences of these variable regions or the full length and the nucleotide sequences encoding them.
(2) The amino acid sequence of Linker 1 is GGGGSGGGGSGGGGSG
GGGS (SEQ ID NO: 18)

Optionally, the amino acid sequence GGGGSGGGGSGGGGS (SEQ ID NO: 19) can be used as Linker 2 in place of the aforementioned Linker 1.
(3) Bevacizumab-H used Ig gamma-1 chain C region (ACCESSION: P01857) as the heavy chain constant region.
(4) Bevacizumab-G4H used Ig gamma-4 chain C region (ACCESSION: P01861.1) as the heavy chain constant region.

2. Expression and purification of antibody VP101(M)

The heavy chain cDNA sequence and the light chain cDNA sequence of VP101 were each cloned into vector pUC57simple (provided by Genscript) to obtain plasmids pUC57simple-VP101H and pUC57simple-VP101L, respectively.
Plasmids pUC57simple-VP101H and pUC57simple-VP101L were enzyme-digested (HindIII&EcoRI), and heavy and light chains isolated by electrophoresis were subcloned into vector pcDNA3.1, and recombinant plasmids were extracted to co-transfect 293F cells. After 7 days of cell culture, the culture medium was centrifuged at high speed, and the supernatant was concentrated and loaded onto a HiTrap MabSelect SuRe column. The protein was further eluted in one step with Elution Buffer, and the target sample antibody VP101 was isolated and buffer exchanged into PBS.

3. Detection of antibody VP101(M)

The purified sample was added to both a reduced protein electrophoresis loading buffer and a non-reduced protein electrophoresis loading buffer, and then boiled for SDS-PAGE electrophoresis detection.
For differentiation from the mutated antibody of Preparation Example 4, VP101(M) is also referred to as VP101(hG1WT) in the present invention. VP101(M) described above is the "wild-type", comprising an Ig gamma-1 chain C region (ACCESSION: P01857) as the heavy chain constant region and an Ig kappa chain C region (ACCESSION: P01834) as the light chain constant region.

Amino acid sequence of the heavy chain of the immunoglobulin moiety in VP101(hG1WT): (453 aa)

EVQLVESGGGLVQPGGSLRLSCAASGYTFTNYGMNWVRQAPGKGLEWVGWINTYTG

EPTYAADFKRRFTFSLDTSKSTAYLQMNSLRAEDTAVYYCAKYPHYYGSSHWYFDVW

GQGTLVTVSSASTKGPSVFPLAPSSKSTSGGTAALGCLVKDYFPEPVTVSWNSGALTSG

VHTFPAVLQSSGLYSLSSVVTVPSSSLGTQTYICNVNHKPSNTKVDKKVEPKSCDKTHT

CPPCPAPELLGGPSVFLFPPKPKDTLMISRTPEVTCVVVDVSHEDPEVKFNWYVDGVEV HNAKTKPREEQYNSTYRVVSVLTVLHQDWLNGKEYKCKVSNKALPAPIEKTISKAKGQ PREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPGK (SEQ ID NO: 20)

Nucleotide sequence encoding the heavy chain of the immunoglobulin moiety in VP101(hG1WT): (1359 bp)

GAGGTGCAGCTGGTCGAGTCCGGGGGGGGGCTGGTGCAGCCAGGCGGGTCTCTGA GGCTGAGTTGCGCCGCTTCAGGGTACACCTTCACAAACTATGGAATGAATTGGGTG CGCCAGGCACCAGGAAAGGGACTGGAGTGGGTCGGCTGGATCAACACTTACACCG GGGAACCTACCTATGCAGCCGACTTTAAGCGGCGGTTCACCTTCAGCCTGGATACA AGCAAATCCACTGCCTACCTGCAGATGAACAGCCTGCGAGCTGAGGACACCGCAGT CTACTATTGTGCTAAATATCCCCACTACTATGGGAGCAGCCATTGGTATTTTGACGT GTGGGGGCAGGGGACTCTGGTGACAGTGAGCAGCGCAAGCACCAAAGGGCCCAGC GTGTTTCCTCTCGCCCCCTCCTCCAAAAGCACCAGCGGAGGAACCGCTGCTCTCGGA TGTCTGGTGAAGGACTACTTCCCTGAACCCGTCACCGTGAGCTGGAATAGCGGCGC TCTGACAAGCGGAGTCCATACATTCCCTGCTGTGCTGCAAAGCAGCGGACTCTATTC CCTGTCCAGCGTCGTCACAGTGCCCAGCAGCAGCCTGGGCACCCAGACCTACATCT GTAACGTCAACCACAAGCCCTCCAACACCAAGGTGGACAAGAAAGTGGAGCCCAA ATCCTGCGACAAGACACACACCTGTCCCCCCTGTCCTGCTCCCGAACTCCTCGGAGG CCCTAGCGTCTTCCTCTTTCCTCCCAAACCCAAGGACACCCTCATGATCAGCAGAAC CCCTGAAGTCACCTGTGTCGTCGTGGATGTCAGCCATGAGGACCCCGAGGTGAAAT TCAACTGGTATGTCGATGGCGTCGAGGTGCACAACGCCAAAACCAAGCCCAGGGAG GAACAGTACAACTCCACCTACAGGGTGGTGTCCGTGCTGACAGTCCTCCACCAGGA CTGGCTGAACGGCAAGGAGTACAAGTGCAAGGTGTCCAACAAGGCTCTCCCTGCCC CCATTGAGAAGACCATCAGCAAGGCCAAAGGCCAACCCAGGGAGCCCCAGGTCTA TACACTGCCTCCCTCCAGGGACGAACTCACCAAGAACCAGGTGTCCCTGACCTGCC TGGTCAAGGGCTTTTATCCCAGCGACATCGCCGTCGAGTGGGAGTCCAACGGACAG CCCGAGAATAACTACAAGACCACCCCTCCTGTCCTCGACTCCGACGGCTCCTTCTTC CTGTACAGCAAACTGACCGTCGATAAATCTAGGTGGCAGCAGGGCAACGTGTTCTC TTGTTCCGTGATGCATGAAGCACTGCACAACCATTATACCCAGAAGTCTCTGAGCCT GTCCCCCGGCAAG (SEQ ID NO: 21)

For differentiation from the mutated antibody of Preparation Example 4, VP101(G4M) is also referred to as VP101(hG4WT) in the present invention. VP101(G4M) described above is the "wild-type", comprising an Ig gamma-4 chain C region (ACCESSION: P01861.1) as the heavy chain constant region and an Ig kappa chain C region (ACCESSION: P01834) as the light chain constant region.

Amino acid sequence of the heavy chain of the immunoglobulin moiety in VP101 (hG4WT): (450 aa)

EVQLVESGGGLVQPGGSLRLSCAASGYTFTNYGMNWVRQAPGKGLEWVGWINTYTG
EPTYAADFKRRFTFSLDTSKSTAYLQMNSLRAEDTAVYYCAKYPHYYGSSHWYFDVW
GQGTLVTVSSASTKGPSVFPLAPCSRSTSESTAALGCLVKDYFPEPVTVSWNSGALTSG
VHTFPAVLQSSGLYSLSSVVTVPSSSLGTKTYTCNVDHKPSNTKVDKRVESKYGPPCPP
CPAPEFLGGPSVFLFPPKPKDTLMISRTPEVTCVVVDVSQEDPEVQFNWYVDGVEVHN
AKTKPREEQFNSTYRVVSVLTVLHQDWLNGKEYKCKVSNKGLPSSIEKTISKAKGQPR
EPQVYTLPPSQEEMTKNQVSLTCLVKGFYPSDIAVEWESNGQPENNYKTTPPVLDSDGS
FFLYSRLTVDKSRWQEGNVFSCSVMHEALHNHYTQKSLSLSLGK (SEQ ID NO: 22)

Nucleotide sequence encoding the heavy chain of the immunoglobulin moiety in VP101(hG4WT): (1350 bp)

GAGGTGCAGCTGGTCGAGTCCGGGGGGGGGCTGGTGCAGCCAGGCGGGTCTCTGA
GGCTGAGTTGCGCCGCTTCAGGGTACACCTTCACAAACTATGGAATGAATTGGGTG
CGCCAGGCACCAGGAAAGGGACTGGAGTGGGTCGGCTGGATCAACACTTACACCG
GGGAACCTACCTATGCAGCCGACTTTAAGCGGCGGTTCACCTTCAGCCTGGATACA
AGCAAATCCACTGCCTACCTGCAGATGAACAGCCTGCGAGCTGAGGACACCGCAGT
CTACTATTGTGCTAAATATCCCCACTACTATGGGAGCAGCCATTGGTATTTTGACGT
GTGGGGGCAGGGGACTCTGGTGACAGTGAGCAGCGCAAGCACCAAAGGGCCCTCG
GTCTTCCCCCTGGCGCCCTGCTCCAGGAGCACCTCCGAGAGCACAGCCGCCCTGGG
CTGCCTGGTCAAGGACTACTTCCCCGAACCGGTGACGGTGTCGTGGAACTCAGGCG

CCCTGACCAGCGGCGTGCACACCTTCCCGGCTGTCCTACAGTCCTCAGGACTCTACT
CCCTCAGCAGCGTGGTGACCGTGCCCTCCAGCAGCTTGGGCACGAAGACCTACACC
TGCAACGTAGATCACAAGCCCAGCAACACCAAGGTGGACAAGAGAGTTGAGTCCA
AATATGGTCCCCCATGCCCACCATGCCCAGCACCTGAGTTCCTGGGGGGACCATCA
GTCTTCCTGTTCCCCCCAAAACCCAAGGACACTCTCATGATCTCCCGGACCCCTGAG
GTCACGTGCGTGGTGGTGGACGTGAGCCAGGAAGACCCCGAGGTCCAGTTCAACTG
GTACGTGGATGGCGTGGAGGTGCATAATGCCAAGACAAAGCCGCGGGAGGAGCAG
TTCAACAGCACGTACCGTGTGGTCAGCGTCCTCACCGTCCTGCACCAGGACTGGCTG
AACGGCAAGGAGTACAAGTGCAAGGTCTCCAACAAAGGCCTCCCGTCCTCCATCGA
GAAAACCATCTCCAAAGCCAAAGGGCAGCCCCGAGAGCCACAGGTGTACACCCTG
CCCCCATCCCAGGAGGAGATGACCAAGAACCAGGTCAGCCTGACCTGCCTGGTCAA
AGGCTTCTACCCCAGCGACATCGCCGTGGAGTGGGAGAGCAATGGGCAGCCGGAG
AACAACTACAAGACCACGCCTCCCGTGCTGGACTCCGACGGCTCCTTCTTCCTCTAC
AGCAGGCTAACCGTGGACAAGAGCAGGTGGCAGGAGGGGAATGTCTTCTCATGCTC
CGTGATGCATGAGGCTCTGCACAACCACTACACACAGAAGTCTCTGAGCCTGTCCC
TCGGCAAG (SEQ ID NO: 23)

Preparation Example 4: Non-Variable Region Amino Acid Mutation Design Based on Humanized Bispecific Antibody VP101(hG1WT)

On the basis of VP101(hG1WT) obtained in Preparation Example 3, VP101(hG1DM) was obtained by introducing a leucine-to-alanine point mutation at position 234 (L234A) and a leucine-to-alanine point mutation at position 235 (L235A) in the heavy chain.

Amino acid sequence of the heavy chain of the immunoglobulin moiety in VP101(hG1DM): (453 aa, mutation positions underlined)

EVQLVESGGGLVQPGGSLRLSCAASGYTFTNYGMNWVRQAPGKGLEWVGWINTYTG
EPTYAADFKRRFTFSLDTSKSTAYLQMNSLRAEDTAVYYCAKYPHYYGSSHWYFDVW
GQGTLVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCDKTHT
CPPCPAPEAAGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTISKAKGQ
PREPQVYTLPPSRDELTKNQVSLTCLVKGFYPSDIAVEWESNGQPENNYKTTPPVLDSD
GSFFLYSKLTVDKSRWQQGNVFSCSVMHEALHNHYTQKSLSLSPGK (SEQ ID NO: 24)

Nucleotide sequence encoding the heavy chain of the immunoglobulin moiety in VP101(hG1DM): (1359 bp, mutation

positions underlined)

GAGGTGCAGCTGGTCGAGTCCGGGGGGGGGCTGGTGCAGCCAGGCGGGTCTCTGA
GGCTGAGTTGCGCCGCTTCAGGGTACACCTTCACAAACTATGGAATGAATTGGGTG
CGCCAGGCACCAGGAAAGGGACTGGAGTGGGTCGGCTGGATCAACACTTACACCG
GGGAACCTACCTATGCAGCCGACTTTAAGCGGCGGTTCACCTTCAGCCTGGATACA
AGCAAATCCACTGCCTACCTGCAGATGAACAGCCTGCGAGCTGAGGACACCGCAGT
CTACTATTGTGCTAAATATCCCCACTACTATGGGAGCAGCCATTGGTATTTTGACGT
GTGGGGGCAGGGGACTCTGGTGACAGTGAGCAGCGCAAGCACCAAAGGGCCCAGC
GTGTTTCCTCTCGCCCCCTCCTCCAAAAGCACCAGCGGAGGAACCGCTGCTCTCGGA
TGTCTGGTGAAGGACTACTTCCCTGAACCCGTCACCGTGAGCTGGAATAGCGGCGC
TCTGACAAGCGGAGTCCATACATTCCCTGCTGTGCTGCAAAGCAGCGGACTCTATTC
CCTGTCCAGCGTCGTCACAGTGCCCAGCAGCAGCCTGGGCACCCAGACCTACATCT
GTAACGTCAACCACAAGCCCTCCAACACCAAGGTGGACAAGAAAGTGGAGCCCAA
ATCCTGCGACAAGACACACACCTGTCCCCCCTGTCCTGCTCCCGAAGCTGCTGGAG
GCCCTAGCGTCTTCCTCTTTCCTCCCAAACCCAAGGACACCCTCATGATCAGCAGAA
CCCCTGAAGTCACCTGTGTCGTCGTGGATGTCAGCCATGAGGACCCCGAGGTGAAA
TTCAACTGGTATGTCGATGGCGTCGAGGTGCACAACGCCAAAACCAAGCCCAGGGA
GGAACAGTACAACTCCACCTACAGGGTGGTGTCCGTGCTGACAGTCCTCCACCAGG
ACTGGCTGAACGGCAAGGAGTACAAGTGCAAGGTGTCCAACAAGGCTCTCCCTGCC
CCCATTGAGAAGACCATCAGCAAGGCCAAAGGCCAACCCAGGGAGCCCCAGGTCT
ATACACTGCCTCCCTCCAGGGACGAACTCACCAAGAACCAGGTGTCCCTGACCTGC
CTGGTCAAGGGCTTTTATCCCAGCGACATCGCCGTCGAGTGGGAGTCCAACGGACA
GCCCGAGAATAACTACAAGACCACCCCTCCTGTCCTCGACTCCGACGGCTCCTTCTT
CCTGTACAGCAAACTGACCGTCGATAAATCTAGGTGGCAGCAGGGCAACGTGTTCT
CTTGTTCCGTGATGCATGAAGCACTGCACAACCATTATACCCAGAAGTCTCTGAGCC
TGTCCCCCGGCAAG (SEQ ID NO: 25)

The amino acid sequences and encoding nucleotide sequences of the light chain of the immunoglobulin moiety of VP101(hG1DM), VP101(hG1WT) and VP101(hG4WT) are identical.

Amino acid sequence of the light chain of the immunoglobulin moiety in VP101(hG1DM): (214 aa)

DIQMTQSPSSLSASVGDRVTITCSASQDISNYLNWYQQKPGKAPKVLIYFTSSLHSGVPS
RFSGSGSGTDFTLTISSLQPEDFATYYCQQYSTVPWTFGQGTKVEIKRTVAAPSVFIFPPS
DEQLKSGTASVVCLLNNFYPREAKVQWKVDNALQSGNSQESVTEQDSKDSTYSLSSTL
TLSKADYEKHKVYACEVTHQGLSSPVTKSFNRGEC (SEQ ID NO: 26)

Nucleotide sequence encoding the light chain of the immunoglobulin moiety in VP101(hG1DM): (642 bp)

GATATTCAGATGACTCAGAGCCCCTCCTCCCTGTCCGCCTCTGTGGGCGACAGGGTC
ACCATCACATGCAGTGCTTCACAGGATATTTCCAACTACCTGAATTGGTATCAGCAG
AAGCCAGGAAAAGCACCCAAGGTGCTGATCTACTTCACTAGCTCCCTGCACTCAGG
AGTGCCAAGCCGGTTCAGCGGATCCGGATCTGGAACCGACTTTACTCTGACCATTTC
TAGTCTGCAGCCTGAGGATTTCGCTACATACTATTGCCAGCAGTATTCTACCGTGCC
ATGGACATTTGGCCAGGGGACTAAAGTCGAGATCAAGCGGACCGTGGCCGCTCCCA
GTGTCTTCATTTTTCCCCCTAGCGACGAACAGCTGAAATCCGGGACAGCCTCTGTGG
TCTGTCTGCTGAACAACTTCTACCCTAGAGAGGCAAAAGTGCAGTGGAAGGTCGAT
AACGCCCTGCAGAGTGGCAATTCACAGGAGAGCGTGACAGAACAGGACTCCAAAG
ATTCTACTTATAGTCTGTCAAGCACACTGACTCTGAGCAAGGCTGACTACGAAAAG
CATAAAGTGTATGCATGTGAGGTCACCCACCAGGGGCTGAGCAGTCCAGTCACCAA
GTCATTCAACAGAGGCGAGTGC (SEQ ID NO: 27)

Example 1: Affinity Constant Assay of FcγRI to VP101(hG1WT) and VP101(hG1DM)

The Fc receptor FcyRI, also known as CD64, can bind to the Fc fragment of IgG antibodies and is involved in antibody-dependent cell-mediated cytotoxicity (ADCC). The binding capacity of a therapeutic monoclonal antibody to Fc receptors will influence the safety and efficacy of the antibody.

The affinity constants of VP101(hG1WT) and VP101(hG1DM) to FcyRI were determined using a Fortebio Octet system in this experiment to evaluate the ADCC activity of each antibody.

The method for determining the affinity constants of the antibodies to FcyRI using the Fortebio Octet system is briefly described as follows: a sample dilution buffer was a solution of PBS, 0.02% Tween-20 and 0.1% BSA, pH 7.4. 1 μg/mL FcyRIa was immobilized on an HISIK sensor for 50 s. The sensor was equilibrated in a buffer for 60 s, and the binding of the immobilized CD64 on the sensor to the antibodies at concentrations of 3.12-50 nM (serial two-fold dilution) was determined for 120 s. The antibodies were dissociated in the buffer for 120 s. The sensor was refreshed 4 times in 10 mM glycine pH 1.5, each for 5 s. The detection temperature was 30 °C and the frequency was 0.3 Hz. The data were analyzed by 1:1 model fitting to obtain affinity constants.

The affinity constants of FcyRI to VP101(hG1WT), VP101(hG4WT) and VP101(hG1DM) as well as control antibodies nivolumab and bevacizumab are shown in Table 1 and FIGs. 1-5.

Table 1: Kinetics for Binding of VP101(hG1WT) and VP101(hG1DM) and Isotypes Thereof to FcγRI

| Antibody | $K_D$ (M) | kon (1/Ms) | SE (kon) | kdis (1/s) | SE (kdis) | Rmax (nm) |
|---|---|---|---|---|---|---|
| VP101(hG1DM) | N/A | N/A | N/A | N/A | N/A | N/A |
| Bevacizumab | 3.68E-09 | 6.61E+05 | 2.07E+04 | 2.44E-03 | 9.02E-05 | 0.46-0.49 |
| Nivolumab | 6.20E-09 | 6.98E+05 | 2.22E+04 | 4.32E-03 | 9.88E-05 | 0.48-0.53 |
| VP101(hGIWT) | 3.95E-09 | 5.67E+05 | 1.82E+04 | 2.24E-03 | 9.02E-05 | 0.68-0.80 |

(continued)

| Antibody | $K_D$ (M) | kon (1/Ms) | SE (kon) | kdis (1/s) | SE (kdis) | Rmax (nm) |
|---|---|---|---|---|---|---|
| VP101(hG4WT) | 8.52E-09 | 6.28E+05 | 2.12E+04 | 5.35E-03 | 1.07E-04 | 0.61-0.65 |
| N/A indicates that the antibody had no binding or an extremely weak binding signal to the antigen, and thus the results were not analyzed and no corresponding data was obtained. | | | | | | |

The results show that VP101(hG1WT) can bind to FcyRI with an affinity constant of 3.95E-09M; VP101(hG4WT) can bind to FcyRI with an affinity constant of 8.52E-09M; bevacizumab can bind to FcyRI with an affinity constant of 3.68E-09M; nivolumab can bind to FcyRI with an affinity constant of 6.20E-09M; VP101(hG1DM) had no binding or an extremely weak binding signal to FcyRI, and thus the results were not analyzed and no corresponding data were obtained. The results show that the affinity of other antibodies to FcyRI is similar except for no binding of VP101(hG1DM) to FcyRI. The binding activity of VP101(hG1DM) was effectively eliminated.

Example 2: Affinity Constant Assay of FcγRIIa_H131 to VP101(hG1WT) and VP101(hG1DM)

The Fc receptor FcγRIIa_H131 (also known as CD32a_H131), can bind to the Fc fragment of IgG antibodies and mediate ADCC effects.

The affinity constants of VP101(hG1WT) and VP101(hG1DM) to FcγRIIa_H131 were determined using a Fortebio Octet system in this experiment to evaluate the ADCC activity of each antibody.

The method for determining the affinity constants of VP101(hG1WT) and VP101(hG1DM) to FcγRIIa_H131 using a Fortebio Octet system is briefly described as follows: the immobilization dilution buffer was a solution of PBS, 0.02% Tween-20 and 0.1% BSA, pH 7.4, and the analyte dilution buffer was a solution of PBS, 0.02% Tween-20, 0.02% casein and 0.1% BSA, pH 7.4. 5 μg/mL FcγRIIa_H131 was immobilized on an NTA sensor for an immobilization time of 60 s. The sensor was equilibrated in a buffer of PBS, 0.02% Tween-20, 0.02% casein and 0.1% BSA (pH 7.4) for 600 s of blocking, and the binding of the immobilized FcγRIIa_H131 on the sensor to the antibodies at concentrations of 12.5-200 nM (serial two-fold dilution) was determined for 60 s. The antibodies were dissociated in the buffer for 60 s. The sensor was refreshed in 10 mM glycine pH 1.7 and 10 nM nickel sulfate. The detection temperature was 30 °C and the frequency was 0.6 Hz. The data were analyzed by 1:1 model fitting to obtain affinity constants.

The affinity constants of FcγRIIa_H131 to VP101(hG1WT), VP101(hG4WT) and VP101(hG1DM) as well as control antibodies nivolumab and bevacizumab are shown in Table 2 and FIGs. 6-10.

Table 2: Kinetics for Binding of VP101(hG1WT) and VP101(hG1DM) and Isotypes Thereof to FcyRIIa H131

| Antibody | $K_D$ (M) | kon (1/Ms) | SE (kon) | kdis (1/s) | SE (kdis) | Rmax (nm) |
|---|---|---|---|---|---|---|
| VP101(hG1DM) | 3.57E-08 | 1.15E+05 | 1.01E+04 | 4.11E-03 | 3.27E-04 | 0.41-0.53 |
| Bevacizumab | 6.44E-08 | 2.10E+05 | 1.78E+04 | 1.36E-02 | 5.14E-04 | 0.49-0.68 |
| Nivolumab | N/A | N/A | N/A | N/A | N/A | N/A |
| VP101(hGIWT) | 2.28E-08 | 2.41E+05 | 1.48E+04 | 5.50E-03 | 3.49E-04 | 1.40-1.52 |
| VP101(hG4WT) | 3.68E-08 | 2.13E+05 | 2.43E+04 | 7.83E-03 | 6.65E-04 | 0.41-0.57 |
| N/A indicates that the antibody had no binding or an extremely weak binding signal to the antigen, and thus the results were not analyzed and no corresponding data was obtained. | | | | | | |

The results show that VP101(hG1WT) can bind to FcγRIIa_H131 with an affinity constant of 2.28E-08M; VP101(hG4WT) can bind to FcγRIIa_H131 with an affinity constant of 3.68E-08M; bevacizumab can bind to FcγRIIa_H131 with an affinity constant of 6.44E-08M; VP101(hG1DM) can bind to FcγRIIa_H131 with an affinity constant of 3.57E-08M; while nivolumab had no binding or an extremely weak binding signal to FcγRIIa_H131, and thus the results were not analyzed and no corresponding data were obtained.

The results show that, except for no binding of nivolumab to FcγRIIa_H131, other antibodies bound to FcyRIIa H131 with the following strong-to-weak affinities: VP101(hG1WT), VP101(hG1DM), VP101(hG4WT) and bevacizumab.

Example 3: Affinity Constant Assay of FcγRIIa_R131 to VP101(hG1WT) and VP101(hG1DM)

The Fc receptor FcγRIIa_R131 (also known as CD32a_R131), can bind to the Fc fragment of IgG antibodies and mediate ADCC effects.

The affinity constants of VP101(hG1WT) and VP101(hG1DM) to FcγRIIa_R131 were determined using a Fortebio Octet system in this experiment to evaluate the ADCC activity of each antibody.

The method for determining the affinity constants of VP101(hG1WT) and VP101(hG1DM) using a Fortebio Octet system is briefly described as follows: the immobilization dilution buffer was a solution of PBS, 0.02% Tween-20 and 0.1% BSA, pH 7.4, and the analyte dilution buffer was a solution of PBS, 0.02% Tween-20, 0.02% casein and 0.1% BSA, pH 7.4. 5 μg/mL FcyRIIa R131 was immobilized on an NTA sensor for an immobilization time of 60 s. The sensor was equilibrated in a buffer of PBS, 0.02% Tween-20, 0.02% casein and 0.1% BSA (pH 7.4) for 600 s of blocking, and the binding of the immobilized FcyRIIa R131 on the sensor to the antibodies at concentrations of 12.5-200 nM (serial two-fold dilution) was determined for 60 s. The antibodies were dissociated in the buffer for 60 s. The sensor was refreshed in 10 mM glycine pH 1.7 and 10 nM nickel sulfate. The detection temperature was 30 °C and the frequency was 0.6 Hz. The data were analyzed by 1:1 model fitting to obtain affinity constants.

The affinity constants of FcγRIIa_R131 to VP101(hG1WT), VP101(hG4WT) and VP101(hG1DM) as well as control antibodies nivolumab and bevacizumab are shown in Table 3 and FIGs. 11-15.

Table 3: Kinetics for Binding of VP101 (hG1WT) and VP101(hG1DM) and Isotypes Thereof to FcγRIIa_R131

| Antibody | KD (M) | kon (1/Ms) | SE (kon) | kdis (1/s) | SE (kdis) | Rmax (nm) |
|---|---|---|---|---|---|---|
| VP101(hG1DM) | 3.35E-08 | 1.20E+05 | 9.72E+03 | 4.03E-03 | 3.08E-04 | 0.57-0.69 |
| Bevacizumab | 5.16E-08 | 2.59E+05 | 1.72E+04 | 1.33E-02 | 4.52E-04 | 0.42-0.69 |
| Nivolumab | 6.93E-08 | 4.78E+05 | 1.09E+05 | 3.31E-02 | 2.54E-03 | 0.08-0.16 |
| VP101(hGIWT) | 2.42E-08 | 2.14E+05 | 1.30E+04 | 5.17E-03 | 3.28E-04 | 1.75-1.92 |
| VP101(hG4WT) | 3.57E-08 | 1.99E+05 | 1.23E+04 | 7.09E-03 | 3.40E-04 | 0.81-1.05 |
| N/A indicates that the antibody had no binding or an extremely weak binding signal to the antigen, and thus the results were not analyzed and no corresponding data was obtained. | | | | | | |

The results show that VP101(hG1WT) can bind to FcγRIIa_R131 with an affinity constant of 2.42E-08M; VP101(hG4WT) can bind to FcγRIIa_R131 with an affinity constant of 3.57E-08M; bevacizumab can bind to FcγRIIa_R131 with an affinity constant of 5.16E-08M; nivolumab can bind to FcγRIIa_R131 with an affinity constant of 6.93E-08M; VP101(hG1DM) can bind to FcγRIIa_R131 with an affinity constant of 3.35E-08M.

The results show that the antibodies bound to FcγRIIa_R131 with the following strong-to-weak affinities: VP101(hG1WT), VP101(hG1DM), VP101(hG4WT), bevacizumab and nivolumab.

Example 4: Affinity Constant Assay of FcγRIIb to VP101(hG1WT) and VP101(hG1DM)

The Fc receptor FcyRIIb (also known as CD32b), can bind to the Fc fragment of IgG antibodies and regulate functions of immune cells.

The affinity constants of VP101(hG1WT) and VP101(hG1DM) to FcyRIIb were determined using a Fortebio Octet system in this experiment to evaluate the binding ability of VP101(hG1WT) and VP101(hG1DM) to the Fc receptor.

The method for determining the affinity constants of VP101(hG1WT) and VP101(hG1DM) to FcyRIIb using a Fortebio Octet system is briefly described as follows: the immobilization dilution buffer was a solution of PBS, 0.02% Tween-20 and 0.1% BSA, pH 7.4, and the analyte dilution buffer was a solution of PBS, 0.02% Tween-20, 0.02% casein and 0.1% BSA, pH 7.4. 5 μg/mL hFCGR2B-his was immobilized on an NTA sensor for an immobilization time of 60 s. The sensor was equilibrated in a buffer of PBS, 0.02% Tween-20, 0.02% casein and 0.1% BSA (pH 7.4) for 600 s of blocking, and the binding of the immobilized hFCGR2B-his on the sensor to the antibodies at concentrations of 12.5-200 nM (serial two-fold dilution) was determined for 60 s. The antibodies were dissociated in the buffer for 60 s. The sensor was refreshed in 10 mM glycine pH 1.7 and 10 nM nickel sulfate. The detection temperature was 30 °C and the frequency was 0.6 Hz. The data were analyzed by 1:1 model fitting to obtain affinity constants.

Example 5: Affinity Constant Assay of FcγRIIIa_V158 to VP101(hG1WT) and VP101(hG1DM)

The Fc receptor FcγRIIIa_V158 (also known as CD16a_V158), can bind to the Fc fragment of IgG antibodies and mediate ADCC effects.

The affinity constants of VP101(hG1WT) and VP101(hG1DM) to FcγRIIIa_V158 were determined using a Fortebio Octet system in this experiment to evaluate the ADCC activity of each antibody.

The method for determining the affinity constant of the antibodies to FcγRIIIa_V158 by the Fortebio Octet system is briefly described as follows: the sample dilution buffer was a solution of PBS, 0.02% Tween-20 and 0.1% BSA, pH 7.4. 5 μg/mL FcγRIIIa_V158 was immobilized on an HIS1K sensor for 120 s. The sensor was equilibrated in a buffer for 60 s, and the binding of the immobilized hFcGR3A(V158)-his on the sensor to the antibodies at concentrations of 31.25-500 nM (serial two-fold dilution) was determined for 60 s. The antibodies were dissociated in the buffer for 60 s. The sensor was refreshed 4 times in 10 mM glycine pH 1.5, each for 5 s. The detection temperature was 30 °C and the frequency was 0.3 Hz. The data were analyzed by 1:1 model fitting to obtain affinity constants.

The affinity constants of FcγRIIIa_V158 to VP101(hG1WT), VP101(hG4WT) and VP101(hG1DM) as well as control antibodies nivolumab and bevacizumab are shown in Table 4 and FIGs. 16-20.

Table 4: Kinetics for Binding of VP101 (hG1WT) and VP101(hG1DM) and Isotypes Thereof to FcγRIIIa_V158

| Antibody | $K_D$ (M) | kon (1/Ms) | SE (kon) | kdis (1/s) | SE (kdis) | Rmax (nm) |
|---|---|---|---|---|---|---|
| VP101(hG1DM) | 1.34E-07 | 6.05E+05 | 2.36E+05 | 8.11E-02 | 7.42E-03 | 0.07-0.21 |
| Bevacizumab | 2.76E-08 | 5.06E+05 | 1.14E+05 | 1.39E-02 | 1.41E-03 | 0.13-0.51 |
| Nivolumab | N/A | N/A | N/A | N/A | N/A | N/A |
| VP101(hGIWT) | 4.35E-08 | 2.39E+05 | 3.14E+04 | 1.04E-02 | 8.73E-04 | 0.80-1.22 |
| VP101(hG4WT) | N/A | N/A | N/A | N/A | N/A | N/A |
| N/A indicates that the antibody had no binding or an extremely weak binding signal to the antigen, and thus the results were not analyzed and no corresponding data was obtained. | | | | | | |

The results show that VP101(hG1WT) can bind to FcγRIIIa_V158 with an affinity constant of 4.35E-08M; VP101(hG1DM) can bind to FcyRIIIa V158 with an affinity constant of 1.34E-07M; bevacizumab can bind to FcyRIIIa V158 with an affinity constant of 2.76E-08M; while nivolumab and VP101(hG4WT) had no binding or an extremely low binding signal to FcyRIIIa V158, and thus the results were not analyzed and no corresponding data were obtained. The results show that, except for no binding of nivolumab and VP101(hG4WT) to FcyRIIIa V158, other antibodies bound to FcγRIIIa_V158 with the following strong-to-weak affinities: bevacizumab, VP101(hG1WT) and VP101(hG1DM).

Example 6: Affinity Constant Assay of FcγRIIIa_F158 to VP101(hG1WT) and VP101(hG1DM)

The Fc receptor FcγRIIIa_F158 (also known as CD16a_F158), can bind to the Fc fragment of IgG antibodies and mediate ADCC effects.

The affinity constants of VP101(hG1WT) and VP101(hG1DM) to FcγRIIIa_F158 were determined using a Fortebio Octet system in this experiment to evaluate the ADCC activity of each antibody.

The method for determining the affinity constants of VP101(hG1WT) and VP101(hG1DM) to FcγRIIIa_F158 using a Fortebio Octet system is briefly described as follows: the sample dilution buffer was a solution of PBS, 0.02% Tween-20 and 0.1% BSA, pH 7.4. 5 μg/mL FcγRIIIa_F158 was immobilized on an HIS1K sensor for 120 s. The sensor was equilibrated in a buffer for 60 s, and the binding of the immobilized hFcGR3A(F158)-his on the sensor to the antibodies at concentrations of 31.25-500 nM (serial two-fold dilution) was determined for 60 s. The antibody was dissociated in the buffer for 60 s. The sensor was refreshed 4 times in 10 mM glycine pH 1.5, each for 5 s. The detection temperature was 30 °C and the frequency was 0.3 Hz. The data were analyzed by 1:1 model fitting to obtain affinity constants.

The affinity constants of FcγRIIIa_F158 to VP101(hG1WT), VP101(hG4WT) and VP101(hG1DM) as well as control antibodies nivolumab and bevacizumab are shown in Table 5 and FIGs. 21-25.

Table 5: Kinetics for Binding of VP101(hG1WT) and VP101(hG1DM) and Isotypes Thereof to FcγRIIIa_F158

| Antibody | $K_D$ (M) | kon (1/Ms) | SE (kon) | kdis (1/s) | SE (kdis) | Rmax (nm) |
|---|---|---|---|---|---|---|
| VP101(hG1DM) | N/A | N/A | N/A | N/A | N/A | N/A |
| Bevacizumab | 9.32E-08 | 2.64E+05 | 7.16E+04 | 2.46E-02 | 2.09E-03 | 0.08-0.20 |
| Nivolumab | N/A | N/A | N/A | N/A | N/A | N/A |
| VP101(hGIWT) | 7.41E-08 | 2.47E+05 | 5.20E+04 | 1.83E-02 | 1.55E-03 | 0.15-0.48 |

(continued)

| Antibody | $K_D$ (M) | kon (1/Ms) | SE (kon) | kdis (1/s) | SE (kdis) | Rmax (nm) |
|---|---|---|---|---|---|---|
| VP101(hG4WT) | N/A | N/A | N/A | N/A | N/A | N/A |
| N/A indicates that the antibody had no binding or an extremely weak binding signal to the antigen, and thus the results were not analyzed and no corresponding data was obtained. | | | | | | |

The results show that VP101(hG1WT) can bind to FcγRIIIa_F158 with an affinity constant of 7.41E-08M; bevacizumab can bind to FcγRIIIa_F158 with an affinity constant of 9.32E-08M; while nivolumab, VP101(hG4WT) and VP101(hG1DM) had no binding or an extremely low binding signal to FcyRIIIa F158, and thus the results were not analyzed and no corresponding data were obtained.

The results show that, except for no binding of nivolumab, VP101(hG4WT) and VP101(hG1DM) to FcγRIIIa_F158, other antibodies bound to FcγRIIIa_F158 with the following strong-to-weak affinities: VP101(hG1WT) and bevacizumab.

<u>Example 7: Affinity Constant Assay of C1q to VP101(hG1WT) and VP101(hG1DM)</u>

Serum complement C1q can bind to the Fc fragment of IgG antibodies and mediate CDC effects. The binding capacity of a therapeutic monoclonal antibody to C1q will influence the safety and efficacy of the antibody.

The affinity constants of VP101(hG1WT) and VP101(hG1DM) to C1q were determined using a Fortebio Octet system in this experiment to evaluate the CDC activity of each antibody.

The method for determining the affinity constant of the antibodies to C1q using a Fortebio Octet system is briefly described as follows: the sample dilution buffer was a solution of PBS, 0.02% Tween-20 and 0.1% BSA, pH 7.4. 50 μg/mL of antibodies were immobilized on an FAB2G sensor at an immobilization height of about 2.0 nm. The sensor was equilibrated in a buffer for 60 s, and the binding of the immobilized antibodies on the sensor to C1q at concentrations of 0.625-10 nM (serial two-fold dilution) was determined for 60 s. The antigen and the antibodies were dissociated in the buffer for 60 s. The sensor was refreshed 4 times in 10 mM glycine pH 1.7, each for 5 s. The shaking speed of the sample plate was 1000 rpm, the temperature was 30 °C and the frequency was 0.6 Hz. The data were analyzed by 1:1 model fitting to obtain affinity constants. The data acquisition software was Fortebio Data Acquisition 7.0, and the data analysis software was Fortebio Data Analysis 7.0.

The affinity constants of C1q to VP101(hG1WT), VP101(hG4WT) and VP101(hG1DM) as well as control antibodies nivolumab and bevacizumab are shown in Table 6 and FIGs. 26-30.

Table 6: Kinetics for Binding of VP101(hG1WT) and VP101(hG1DM) and Isotypes Thereof to C1q

| Antibody | $K_D$ (M) | kon (1/Ms) | SE (kon) | kdis (1/s) | SE (kdis) | Rmax (nm) |
|---|---|---|---|---|---|---|
| VP101(hG1DM) | N/A | N/A | N/A | N/A | N/A | N/A |
| Bevacizumab | 1.14E-09 | 6.52E+06 | 5.64E+05 | 7.40E-03 | 5.58E-04 | 0.51-0.63 |
| Nivolumab | N/A | N/A | N/A | N/A | N/A | N/A |
| VP101(hGIWT) | 9.76E-10 | 5.73E+06 | 5.49E+05 | 5.59E-03 | 6.12E-04 | 0.32-0.51 |
| VP101(hG4WT) | N/A | N/A | N/A | N/A | N/A | N/A |
| N/A indicates that the antibody had no binding or an extremely weak binding signal to the antigen, and thus the results were not analyzed and no corresponding data was obtained. | | | | | | |

The results show that VP101(hG1WT) can bind to C1q with an affinity constant of 9.76E-10M; bevacizumab can bind to C1q with an affinity constant of 1.14E-09M; while nivolumab, VP101(hG4WT) and VP101(hG1DM) had no binding or an extremely low binding signal to C1q, and thus the results were not analyzed and no corresponding data were obtained.

The results show that, except for no binding of nivolumab, VP101(hG4WT) and VP101(hG1DM) to FcγRIIIa_F158, other antibodies bound to C1q, and VP101(hG1WT) had a similar affinity to bevacizumab.

Example 8: ADCC Activity Assay of VP101(hG1WT) and VP101(hG1DM) on CHO-K1-PD1 Cells Expressing PD-1 Antigen

In order to detect the ADCC effect of the antibodies VP101(hG1WT) and VP101(hG1DM) at the cellular level, the inventors constructed CHO-K1-PD1 cells expressing PD-1 antigen, and established a system of co-culture of normal human PBMC and target cells for detecting the ADCC activity of the antibodies at the cytological level.

The method for detecting the ADCC activity of VP101(hG1WT) and VP101(hG1DM) on CHO-K1-PD1 cells expressing PD-1 antigen is specified as follows:

In this experiment, a human PD-1 overexpression vector pCDH-CMV-PD1FL-Puro (pCDH-CMV-Puro purchased from Youbio) was firstly constructed, the expression vector was packaged by virus and then infected CHO-K1 cells, and a CHO-K1-PD1 stable cell line of the drug-resistant stable expression membrane PD-1 protein was obtained after dosing and screening with Puromycin (2 μg/mL). Normal human PBMCs were isolated according to the Ficoll peripheral blood mononuclear cell isolation instruction. The isolated PBMCs were resuspended in a 1640 complete medium and stained with trypan blue, cells were counted and the viability of cells was determined. The cells were incubated overnight in a saturated humidity incubator at 37 °C and 5% $CO_2$. The next day, CHO-K1-PD1 cells and PBMC were collected and centrifuged to remove the supernatant, and then the cell pellets were resuspended in RPMI-1640 (containing 1% BSA) (hereinafter referred to as assay medium), centrifuged and washed 2 times; the cells were counted and the viability of cells was determined, the concentration of the cells was adjusted to a proper range by using the assay medium, and the CHO-K1-PD1 cell suspension (30,000/well) was added into a 96-well plate according to the experimental design; 50 μL of antibody was added, mixed well, and pre-incubated for 1 h at room temperature; after pre-incubation, the cells were added with PBMC (900,000/50 μL/well), mixed well, and incubated for 4 h in a incubator at 37 °C and 5% $CO_2$. After 4 h, the 96-well plate was removed and centrifuged for 5 min at 250× g; 100 μL of the cell supernatant was carefully transferred to a new 96-well flat-bottom microplate (do not pipette the cell precipitate). 100 μL of freshly prepared reaction solution was added to each well according to the Cytotoxicity Detection Kit instruction. The cells were incubated for 30 min at room temperature in the dark. OD values at 490 nm and 650 nm were measured, wherein the OD values of each group = $OD_{490nm}$ - $OD_{650nm}$. ADCC activity was calculated for each group according to the formula ADCC(%) = (treatment group - negative control group)/(maximum LDH release in target cell - spontaneous LDH release in target cell) × 100%.

The detection results of the ADCC activity of VP101(hG1WT) and VP101(hG1DM) on CHO-K1-PD1 cells expressing the PD-1 antigen are expressed as ADCC%, and the results are shown in FIG. 31.

The results show that the positive control 14C12H1L1(G1WT) has significant ADCC activity in the mixed culture system of PBMC and CHO-K1-PD1, indicating that the ADCC system is normal. Compared to the isotype control antibody hIgG1DM, VP101(hG1WT) showed significant ADCC activity and exhibited dose-dependence, whereas VP101(G1DM) did not see ADCC activity. The results show that VP101(hG1DM) produced by mutation based on VP101(hG1WT) has no ADCC activity at the cytological level, and the ADCC effect is eliminated.

Example 9: CDC Activity Assay of VP101(hG1WT) and VP101(HG1DM) on CHO-K1-PD1 Cells Expressing PD-1 Antigen

In order to detect the CDC effect of the antibodies VP101(hG1WT) and VP101(hG1DM) at the cytological level, the inventors constructed CHO-K1-PD1 cells expressing PD-1 antigen (see Example 8 for the construction method), and established a system of co-culture of target cells and normal human complement serum for detecting the CDC activity of the antibodies at the cytological level.

The method for detecting the CDC activity of VP101(hG1WT) and VP101(hG1DM) on CHO-K1-PD1 cells expressing PD-1 antigen is specified as follows:

On the day of detection, CHO-K1-PD1 cells were collected by trypsinization and centrifuged at 170× g for 5 min; the cell pellets were resuspended in RPMI-1640 (containing 1% BSA) (hereinafter referred to as assay medium), repeatedly centrifuged and washed 2 times; the cells were counted and the viability of cells was determined, the concentration of the cells was adjusted to a proper range by using the assay medium, and the CHO-K1-PD1 cell suspension (30,000/well) was added into a 96-well plate according to the experimental design; 50 μL of antibody was added, mixed well, and pre-incubated for 10 min at room temperature; after pre-incubation, the cells were added with normal human complement serum (final concentration: 2%) at 50 μL/well, mixed well, and incubated for 4 h in an incubator at 37 °C and 5% $CO_2$. After 4 h, the cells were centrifuged for 5 min at 250× g; 100 μL of the cell supernatant was carefully transferred to a new 96-well flat-bottom plate (do not pipette the cell precipitate). 100 μL of freshly prepared reaction solution was added to each well according to the Cytotoxicity Detection Kit instruction. The cells were incubated for 30 min at room temperature in the dark. OD values at 490 nm and 650 nm were measured, wherein the OD values of each group = $OD_{490nm}$ - $OD_{650nm}$. CDC activity was calculated for each group according to the formula CDC(%) = (treatment group - negative control group)/(-maximum LDH release in target cell - spontaneous LDH release in target cell) × 100%.

The detection results of the CDC activity of VP101(hG1WT) and VP101(hG1DM) on CHO-K1-PD1 cells expressing the PD-1 antigen are expressed as CDC%, and the results are shown in FIG. 32.

The results show that CDC% of the positive control antibody 14C12H1L1(G1WT) had significant difference from that of the isotype control antibody hIgG1DM group in the mixed culture system of normal human complement serum and CHO-K1-PD1, indicating that the CDC detection system is normal. At equivalent dose levels, there was no significant difference in CDC% for both VP101(hG1WT) and VP101(hG1DM) compared to the isotype control.

Example 10: Pharmacodynamic Activities of VP101(hG1DM) in Mixed Culture System (MLR) of Peripheral Blood Mononuclear Cells and Raji-PDL1 Cells

In this experiment, a human PD-L1 overexpression vector plenti6.3-PD-L1-BSD (plenti6.3-BSD purchased from invitrogen) was firstly constructed, the expression vector was packaged by virus and then infected Raji cells, and a Raji-PDL1 stable cell line for stably expressing membrane PD-L1 protein was obtained after dosing and screening with BSD (10 μg/mL). Normal human PBMCs were isolated according to the Ficoll peripheral blood mononuclear cell isolation instruction, and the isolated PBMCs were resuspended in a 1640 complete medium, counted and frozen. The PBMCs were recovered, added with SEB (Staphylococcus aureus enterotoxin B) and stimulated to culture for two days. Two days later, Raji-PDL1 cells in the logarithmic phase were collected, added with mitomycin C (Sigma, working concentration was 25 μg/mL) and incubated in an incubator for 60 min, and the mitomycin C-treated Raji-PDL1 cells were centrifuged and washed; PBMCs stimulated with SEB for two days were collected and washed; these cells were mixed and cultured according to a proportion of 1:1 of the cell number under the condition of the presence or absence of antibodies. 3 days later, cell supernatant was collected by centrifugation, and IL-2 and IFN-y concentrations in the supernatant were detected by ELISA.

The results of secretion of IFN-γ are shown in FIG. 33. The results show that VP101(hG1DM) can effectively promote the secretion of IFN-y, and its activity is significantly superior to that of nivolumab.

The results of secretion of IL-2 are shown in FIG. 34. The results show that VP101(hG1DM) can effectively promote the secretion of IL-2 in a dose-dependent relationship, and its activity is significantly superior to that of nivolumab.

Example 11: No Antibody-Dependent Phagocytic Activity of VP101(hG1DM)on PD-1-Positive Cells

Antibody dependent cellular phagoxytosis (ADCP) means that the Fc fragment of an antibody bound to a cell surface antigen binds to the Fc receptor of a phagocytically active cell (such as a macrophage), and thus mediates phagocytosis of the antibody-bound cells. For immune checkpoint inhibitor antibodies, such as the PD-1 antibody, the presence of ADCP activity will cause damage to immune cells expressing PD-1 that exert an anti-tumor killing effect, thereby affecting their anti-tumor activity.

In this experiment, murine macrophages were taken as effector cells, PD-1 overexpression CHO-K1-PD1 cell lines (see Example 8 for the construction method) were taken as target cells. The ADCP effect mediated by the cells was detected. In this experiment, flow cytometry was adopted to detect the ADCP activity of VP101(hG1DM) on cells expressing PD-1, and the results show that VP101(hG1DM) had no ADCP activity, whereas the marketed antibody nivolumab for the same target had significant ADCP activity. The method is specifically as follows:

The femoral bone marrow of C57 mice (purchased from Guangdong Medical Laboratory Animal Center) was first aseptically collected and lysed by erythrocyte lysis buffer on ice for 5 min. The lysis was terminated with DMEM complete medium (containing 10% FBS), and the lysate was centrifuged at 1000 rpm and washed twice. The cell pellet was resuspended in 10 mL of DMEM complete medium and M-CSF were added at a working concentration of 100 ng/mL. The cells were cultured for 7 days at 37 °C and 5% $CO_2$ in a cell culture chamber for induction. Half of the medium was exchanged and M-CSF was added on days 3 and 5. The induction of cells was completed on day 7. The cells were digested with 0.25% trypsin. Macrophages were collected, and centrifuged at 750× g for 5 min. The supernatant was removed and the cells were suspended in DMEM complete medium (containing 10% FBS) and counted. The cells were adjusted to a proper density and filled into a 96-well conical bottom plate for later use.

CHO-K1-PD1 cells were collected by conventional methods, centrifuged at 170× g for 5 min, resuspended and counted, and the viability was determined. The cells were washed once with PBS. Carboxyfluorescein diacetate succinimidyl ester (CFSE) was diluted to 2.5 μM with PBS, and the cells were resuspended with an appropriate amount of diluted CFSE (staining density: 10,000,000 cells/mL) and incubated in an incubator for 20 min. 6 mL of DMEM complete medium (containing 10% FBS) was added to stop staining. The cells were centrifuged at 170× g for 5 min, and the supernatant was removed; 1 mL of DMEM complete medium was added. The cells were incubated in an incubator for 10 min. The antibodies were diluted with DMEM complete medium to 20 μg/mL, 2 μg/mL and 0.2 μg/mL (working concentrations were 10 μg/mL, 1 μg/mL and 0.1 μg/mL), and isotype control antibodies hIgG1DM and hIgG4 were designed. Fresh induced mature macrophages were collected and centrifuged at 750× g for 5 min, and the supernatant was removed; the cells were counted and transferred to a 96-well conical bottom plate, and centrifuged at 1000× g for 5 min, and the supernatant was removed; the cell density of CHO-K1-PD 1-CFSE was adjusted; the diluted antibodies and the target cells were mixed according to the experimental design according to a proportion of 50 μL: 50 μL into the 96-well conical bottom plate containing the macrophages. The cells were resuspended and mixed well, and incubated in an incubator at 37 °C for 2 h. 150 μL of normal-temperature 1% PBSA was added into each well and centrifuged at 1000× g for 5 min, and the supernatant was removed; the cells were washed once with 200 μL of PBSA; APC anti-mouse/human CD11b antibody (500-fold diluted with PBSA) was added to the corresponding samples at 100 μL/sample, mixed well and incubated on ice for 40 min. 150 μL of 1% PBSA was added into each well and centrifuged at 1000× g for 5 min, and the supernatant was removed; each well was washed once with 200 μL of PBSA. 200 μL of 1% PBSA was added into each well for resuspension followed by analysis using a Beckman flow cytometer.

Macrophages in the system were $APC^+$ positive, and macrophages involved in phagocytosis were APC and CFSE double positive. The phagocytosis rate was determined as the ratio of the number of double positive cells to the number of APC positive cells, and the antibody-dependent ADCP activity was evaluated. The ADCP activity of each group, represented by P%, was calculated according to the following formulas:

$$P\% = \frac{\text{Number of macrophages involved in phagocytosis}}{\text{Total number of macrophages}} \times 100\%$$

The results are shown in FIG. 35.

The results show that nivolumab had a significant ADCP effect in the macrophage + CHO-K1-PD1 system; the phagocytosis rate of VP101(hG1DM) was comparable to that of the isotype control antibody, indicating that VP101(hG1DM) had no ADCP effect. The results indicate that VP101(hG1DM) is likely to have a better anti-tumor effect.

Although specific embodiments of the present invention have been described in detail, those skilled in the art will understand. Various modifications and substitutions can be made to those details according to all the teachings that have been disclosed, and these changes are all within the protection scope of the present invention. The full scope of the present invention is given by the appended claims and any equivalent thereof.

EMBODIMENTS

1. A bispecific antibody, comprising:

a first protein functional region targeting PD-1, and
a second protein functional region targeting VEGFA;
wherein the first protein functional region is an immunoglobulin, and the second protein functional region is a single chain antibody; or, the first protein functional region is a single chain antibody, and the second protein functional region is an immunoglobulin;
wherein,
for the immunoglobulin, the heavy chain variable region comprises HCDR1-HCDR3 of amino acid sequences set forth in SEQ ID NOs: 28-30 respectively, and the light chain variable region comprises LCDR1-LCDR3 of amino acid sequences set forth in SEQ ID NOs: 31-33 respectively; for the single chain antibody, the heavy chain variable region comprises HCDR1-HCDR3 of amino acid sequences set forth in SEQ ID NOs: 34-36 respectively, and the light chain variable region comprises LCDR1-LCDR3 of amino acid sequences set forth in SEQ ID NOs: 37-39 respectively;
or,
for the immunoglobulin, the heavy chain variable region comprises HCDR1-HCDR3 of amino acid sequences set forth in SEQ ID NOs: 34-36 respectively, and the light chain variable region comprises LCDR1-LCDR3 of amino acid sequences set forth in SEQ ID NOs: 37-39 respectively; for the single chain antibody, the heavy chain variable region comprises HCDR1-HCDR3 of amino acid sequences set forth in SEQ ID NOs: 28-30 respectively, and the light chain variable region comprises LCDR1-LCDR3 of amino acid sequences set forth in SEQ ID NOs: 31-33 respectively;
the immunoglobulin is of human IgG1 subtype;
wherein, according to the EU numbering system, the heavy chain constant region of the immunoglobulin has mutations at any 2 or 3 of positions 234, 235 and 237, and the affinity constant of the bispecific antibody to FcγRIIIa and/or C1q is reduced after the mutation as compared to that before the mutation; preferably, the affinity constant is measured by a Fortebio Octet system.

2. The bispecific antibody according to embodiment 1, wherein, according to the EU numbering system, the heavy chain constant region of the immunoglobulin has the following mutations:

L234A and L235A; or
L234A and G237A; or
L235A and G237A;
or
L234A, L235A and G237A.

3. A bispecific antibody, comprising:

a first protein functional region targeting PD-1, and
a second protein functional region targeting VEGFA;
wherein the first protein functional region is an immunoglobulin, and the second protein functional region is a single chain antibody; or, the first protein functional region is a single chain antibody, and the second protein functional region is an immunoglobulin;
wherein,
for the immunoglobulin, the heavy chain variable region comprises HCDR1-HCDR3 of amino acid sequences set forth in SEQ ID NOs: 28-30 respectively, and the light chain variable region comprises LCDR1-LCDR3 of amino acid sequences set forth in SEQ ID NOs: 31-33 respectively; for the single chain antibody, the heavy chain variable region comprises HCDR1-HCDR3 of amino acid sequences set forth in SEQ ID NOs: 34-36 respectively, and the light chain variable region comprises LCDR1-LCDR3 of amino acid sequences set forth in SEQ ID NOs: 37-39 respectively;
or,
for the immunoglobulin, the heavy chain variable region comprises HCDR1-HCDR3 of amino acid sequences set forth in SEQ ID NOs: 34-36 respectively, and the light chain variable region comprises LCDR1-LCDR3 of amino acid sequences set forth in SEQ ID NOs: 37-39 respectively; for the single chain antibody, the heavy chain variable region comprises HCDR1-HCDR3 of amino acid sequences set forth in SEQ ID NOs: 28-30 respectively, and the light chain variable region comprises LCDR1-LCDR3 of amino acid sequences set forth in SEQ ID NOs: 31-33 respectively;
the immunoglobulin is of human IgG1 subtype;
wherein, according to the EU numbering system, the heavy chain constant region of the immunoglobulin has the following mutations:

L234A and L235A; or
L234A and G237A; or
L235A and G237A; or
L234A, L235A and G237A.

4. The bispecific antibody according to any one of embodiments 1-3, wherein, according to the EU numbering system, the heavy chain constant region of the immunoglobulin has one or more mutations selected from:
N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, P331S, H268A, E318A and K320A.

5. The bispecific antibody according to any one of embodiments 1-4, wherein,

the amino acid sequence of the heavy chain variable region of the immunoglobulin is set forth in SEQ ID NO: 1, and the amino acid sequence of the light chain variable region of the immunoglobulin is set forth in SEQ ID NO: 3; the amino acid sequence of the heavy chain variable region of the single chain antibody is selected from SEQ ID NO: 5 and SEQ ID NO: 9, and the amino acid sequence of the light chain variable region of the single chain antibody is selected from SEQ ID NO: 7, SEQ ID NO: 11 and SEQ ID NO: 17;
or,
the amino acid sequence of the heavy chain variable region of the immunoglobulin is selected from SEQ ID NO: 5 and SEQ ID NO: 9, and the amino acid sequence of the light chain variable region of the immunoglobulin is selected from SEQ ID NO: 7, SEQ ID NO: 11 and SEQ ID NO: 17; the amino acid sequence of the heavy chain variable region of the single chain antibody is set forth in SEQ ID NO: 1, and the amino acid sequence of the light chain variable region of the single chain antibody is set forth in SEQ ID NO: 3.

6. The bispecific antibody according to any one of embodiments 1-5, wherein the bispecific antibody is selected from any one of the following (1)-(12):

(1)
the amino acid sequence of the heavy chain variable region of the immunoglobulin is set forth in SEQ ID NO: 1, and the amino acid sequence of the light chain variable region of the immunoglobulin is set forth in SEQ ID NO: 3; the amino acid sequence of the heavy chain variable region of the single chain antibody is set forth in SEQ ID NO: 5, and the amino acid sequence of the light chain variable region of the single chain antibody is set forth in SEQ ID NO: 7;
(2)
the amino acid sequence of the heavy chain variable region of the immunoglobulin is set forth in SEQ ID NO: 1, and the amino acid sequence of the light chain variable region of the immunoglobulin is set forth in SEQ ID NO: 3; the amino acid sequence of the heavy chain variable region of the single chain antibody is set forth in SEQ ID NO: 5, and the amino acid sequence of the light chain variable region of the single chain antibody is set forth in SEQ ID NO: 11;
(3)
the amino acid sequence of the heavy chain variable region of the immunoglobulin is set forth in SEQ ID NO: 1, and the amino acid sequence of the light chain variable region of the immunoglobulin is set forth in SEQ ID NO: 3; the amino acid sequence of the heavy chain variable region of the single chain antibody is set forth in SEQ ID NO: 5, and the amino acid sequence of the light chain variable region of the single chain antibody is set forth in SEQ ID NO: 17;
(4)
the amino acid sequence of the heavy chain variable region of the immunoglobulin is set forth in SEQ ID NO: 1, and the amino acid sequence of the light chain variable region of the immunoglobulin is set forth in SEQ ID NO: 3; the amino acid sequence of the heavy chain variable region of the single chain antibody is set forth in SEQ ID NO: 9, and the amino acid sequence of the light chain variable region of the single chain antibody is set forth in SEQ ID NO: 7;
(5)
the amino acid sequence of the heavy chain variable region of the immunoglobulin is set forth in SEQ ID NO: 1, and the amino acid sequence of the light chain variable region of the immunoglobulin is set forth in SEQ ID NO: 3; the amino acid sequence of the heavy chain variable region of the single chain antibody is set forth in SEQ ID NO: 9, and the amino acid sequence of the light chain variable region of the single chain antibody is set forth in SEQ ID NO: 11;
(6)
the amino acid sequence of the heavy chain variable region of the immunoglobulin is set forth in SEQ ID NO: 1, and the amino acid sequence of the light chain variable region of the immunoglobulin is set forth in SEQ ID NO: 3; the amino acid sequence of the heavy chain variable region of the single chain antibody is set forth in SEQ ID NO: 9, and the amino acid sequence of the light chain variable region of the single chain antibody is set forth in SEQ ID NO: 17;
(7)
the amino acid sequence of the heavy chain variable region of the immunoglobulin is set forth in SEQ ID NO: 5, and the amino acid sequence of the light chain variable region of the immunoglobulin is set forth in SEQ ID NO: 7; the amino acid sequence of the heavy chain variable region of the single chain antibody is set forth in SEQ ID NO: 1, and the amino acid sequence of the light chain variable region of the single chain antibody is set forth in SEQ ID NO: 3;
(8)
the amino acid sequence of the heavy chain variable region of the immunoglobulin is set forth in SEQ ID NO: 5, and the amino acid sequence of the light chain variable region of the immunoglobulin is set forth in SEQ ID NO: 11; the amino acid sequence of the heavy chain variable region of the single chain antibody is set forth in SEQ ID NO: 1, and the amino acid sequence of the light chain variable region of the single chain antibody is set forth in SEQ ID NO: 3;
(9)
the amino acid sequence of the heavy chain variable region of the immunoglobulin is set forth in SEQ ID NO: 5, and the amino acid sequence of the light chain variable region of the immunoglobulin is set forth in SEQ ID NO: 17; the amino acid sequence of the heavy chain variable region of the single chain antibody is set forth in SEQ ID NO: 1, and the amino acid sequence of the light chain variable region of the single chain antibody is set forth in SEQ ID NO: 3;
(10)

the amino acid sequence of the heavy chain variable region of the immunoglobulin is set forth in SEQ ID NO: 9, and the amino acid sequence of the light chain variable region of the immunoglobulin is set forth in SEQ ID NO: 7; the amino acid sequence of the heavy chain variable region of the single chain antibody is set forth in SEQ ID NO: 1, and the amino acid sequence of the light chain variable region of the single chain antibody is set forth in SEQ ID NO: 3;

(11)

the amino acid sequence of the heavy chain variable region of the immunoglobulin is set forth in SEQ ID NO: 9, and the amino acid sequence of the light chain variable region of the immunoglobulin is set forth in SEQ ID NO: 11; the amino acid sequence of the heavy chain variable region of the single chain antibody is set forth in SEQ ID NO: 1, and the amino acid sequence of the light chain variable region of the single chain antibody is set forth in SEQ ID NO: 3; and

(12)

the amino acid sequence of the heavy chain variable region of the immunoglobulin is set forth in SEQ ID NO: 9, and the amino acid sequence of the light chain variable region of the immunoglobulin is set forth in SEQ ID NO: 17; the amino acid sequence of the heavy chain variable region of the single chain antibody is set forth in SEQ ID NO: 1, and the amino acid sequence of the light chain variable region of the single chain antibody is set forth in SEQ ID NO: 3.

7. The bispecific antibody according to any one of embodiments 1-6, wherein, the amino acid sequence of the heavy chain of the immunoglobulin is set forth in SEQ ID NO: 24, and the amino acid sequence of the light chain of the immunoglobulin is set forth in SEQ ID NO: 26.

8. The bispecific antibody according to any one of embodiments 1-7, wherein the immunoglobulin or an antigen-binding fragment thereof binds to FcγRI with an affinity constant of less than about $10^{-6}$ M, such as less than about $10^{-7}$ M, $10^{-8}$ M or $10^{-9}$ M or less; preferably, the affinity constant is measured by a Fortebio Octet system.

9. The bispecific antibody according to any one of embodiments 1-8, wherein the immunoglobulin or the antigen-binding fragment thereof binds to C1q with an affinity constant of less than about $10^{-9}$ M, such as less than about $10^{-7}$M, $10^{-8}$ M or $10^{-9}$ M or less; preferably, the affinity constant is measured by a Fortebio Octet system.

10. The bispecific antibody according to any one of embodiments 1-9, wherein the first protein functional region is linked to the second protein functional region either directly or via a linker fragment; and/or the heavy chain variable region of the single chain antibody is linked to the light chain variable region of the single chain antibody either directly or via a linker fragment.

11. The bispecific antibody according to embodiment 10, wherein the linker fragment is (GGGGS)n, n being a positive integer; preferably, n is 1, 2, 3, 4, 5 or 6.

12. The bispecific antibody according to any one of embodiments 1-11, wherein the numbers of the first protein functional region and second protein functional region are each independently 1, 2 or more.

13. The bispecific antibody according to any one of embodiments 1-12, wherein the single chain antibody is linked to a C-terminus of the heavy chain of the immunoglobulin.

14. A bispecific antibody, comprising:

a first protein functional region targeting PD-1, and
a second protein functional region targeting VEGFA;
wherein the number of the first protein functional region is 1, and the number of the second protein functional region is 2;
the first protein functional region is an immunoglobulin, and the second protein functional region is a single chain antibody;
the amino acid sequence of the heavy chain of the immunoglobulin is set forth in SEQ ID NO: 24, and the amino acid sequence of the light chain of the immunoglobulin is set forth in SEQ ID NO: 26;
the amino acid sequence of the heavy chain variable region of the single chain antibody is set forth in SEQ ID NO: 9, and the amino acid sequence of the light chain variable region of the single chain antibody is set forth in SEQ ID NO: 17;
the single chain antibody is linked to the C-terminus of the heavy chain of the immunoglobulin;

the first protein functional region is linked to the second protein functional region via a first linker fragment; and the heavy chain variable region of the single chain antibody is linked to the light chain variable region of the single chain antibody via a second linker fragment; the first linker fragment and the second linker fragment are the same or different;

preferably, the amino acid sequences of the first linker fragment and second linker fragment are independently selected from SEQ ID NO: 18 and SEQ ID NO: 19;

preferably, the amino acid sequences of the first linker fragment and second linker fragments are set forth in SEQ ID NO: 18.

15. An isolated nucleic acid molecule, encoding the bispecific antibody according to any one of embodiments 1-14.

16. A vector, comprising the isolated nucleic acid molecule according to embodiment 15.

17. A host cell, comprising the isolated nucleic acid molecule according to embodiment 15 or the vector according to embodiment 16.

18. A conjugate, comprising an antibody or an antigen-binding fragment thereof and a conjugated moiety, wherein the immunoglobulin is the bispecific antibody according to any one of embodiments 1-14, and the conjugated moiety is a detectable label; preferably, the conjugated moiety is a radioisotope, a fluorescent substance, a luminescent substance, a colored substance, or an enzyme.

19. A kit, comprising the bispecific antibody according to any one of embodiments 1-14 or the conjugate according to embodiment 18;

wherein preferably, the kit further comprises a second antibody capable of specifically recognizing the immunoglobulin or the antigen binding fragment thereof; optionally, the second antibody further comprises a detectable label, for example, a radioisotope, a fluorescent substance, a luminescent substance, a colored substance, or an enzyme.

20. Use of the bispecific antibody according to any one of embodiments 1-14 or the conjugate according to embodiment 18 in the preparation of a kit for detecting the presence or level of PD-1 and/or VEGFA in a sample.

21. A pharmaceutical composition, comprising the bispecific antibody according to any one of embodiments 1-14 or the conjugate according to embodiment 18, wherein, optionally, the pharmaceutical composition further comprises a pharmaceutically acceptable vector and/or excipient.

22. Use of the bispecific antibody according to any one of embodiments 1-14 or the conjugate according to embodiment 18 in the preparation of a medicament for treating and/or preventing a malignant tumor; preferably, the malignant tumor is selected from colon cancer, rectal cancer, lung cancer, liver cancer, ovarian cancer, skin cancer, glioma, melanoma, lymphoma, renal tumor, prostate cancer, bladder cancer, gastrointestinal cancer, breast cancer, brain cancer, cervical cancer, esophageal cancer, microsatellite instability-high (MSI-H) and deficient mismatch repair (dMMR) cancer, urothelial cancer, mesothelioma, endometrial cancer, gastric adenocarcinoma, gastroesophageal junction adenocarcinoma and leukemia;

preferably, the lung cancer is non-small cell lung cancer or small cell lung cancer; preferably, the non-small cell lung cancer is EGFR and/or ALK sensitive mutant non-small cell lung cancer;

preferably, the liver cancer is hepatocellular carcinoma;

preferably, the renal tumor is renal cell carcinoma;

preferably, the breast cancer is triple negative breast cancer;

preferably, the urothelial cancer is bladder cancer.

23. A method for treating and/or preventing a malignant tumor, comprising a step of administering to a subject in need thereof an effective amount of the bispecific antibody according to any one of embodiments 1-14 or the conjugate according to embodiment 18; preferably, the malignant tumor is selected from colon cancer, rectal cancer, lung cancer, liver cancer, ovarian cancer, skin cancer, glioma, melanoma, lymphoma, renal tumor, prostate cancer, bladder cancer, gastrointestinal cancer, breast cancer, brain cancer, cervical cancer, esophageal cancer, microsatellite instability-high (MSI-H) and deficient mismatch repair (dMMR) cancer, urothelial cancer, mesothelioma, endometrial cancer, gastric adenocarcinoma, gastroesophageal junction adenocarcinoma and leukemia;

preferably, the lung cancer is non-small cell lung cancer or small cell lung cancer; preferably, the non-small cell lung cancer is EGFR and/or ALK sensitive mutant non-small cell lung cancer;
preferably, the liver cancer is hepatocellular carcinoma;
preferably, the renal tumor is renal cell carcinoma;
preferably, the breast cancer is triple negative breast cancer;
preferably, the urothelial cancer is bladder cancer.

24. The bispecific antibody according to any one of embodiments 1-14 for use in the treatment and/or prevention of a malignant tumor; preferably, the malignant tumor is selected from colon cancer, rectal cancer, lung cancer, liver cancer, ovarian cancer, skin cancer, glioma, melanoma, lymphoma, renal tumor, prostate cancer, bladder cancer, gastrointestinal cancer, breast cancer, brain cancer, cervical cancer, esophageal cancer, microsatellite instability-high (MSI-H) and deficient mismatch repair (dMMR) cancer, urothelial cancer, mesothelioma, endometrial cancer, gastric adenocarcinoma, gastroesophageal junction adenocarcinoma and leukemia;

preferably, the lung cancer is non-small cell lung cancer or small cell lung cancer; preferably, the non-small cell lung cancer is EGFR and/or ALK sensitive mutant non-small cell lung cancer;
preferably, the liver cancer is hepatocellular carcinoma;
preferably, the renal tumor is renal cell carcinoma;
preferably, the breast cancer is triple negative breast cancer;
preferably, the urothelial cancer is bladder cancer.

25. The conjugate according to embodiment 18 for use in the treatment and/or prevention of a malignant tumor; preferably, the malignant tumor is selected from colon cancer, rectal cancer, lung cancer, liver cancer, ovarian cancer, skin cancer, glioma, melanoma, lymphoma, renal tumor, prostate cancer, bladder cancer, gastrointestinal cancer, breast cancer, brain cancer, cervical cancer, esophageal cancer, microsatellite instability-high (MSI-H) and deficient mismatch repair (dMMR) cancer, urothelial cancer, mesothelioma, endometrial cancer, gastric adenocarcinoma, gastroesophageal junction adenocarcinoma and leukemia;

preferably, the lung cancer is non-small cell lung cancer or small cell lung cancer; preferably, the non-small cell lung cancer is EGFR and/or ALK sensitive mutant non-small cell lung cancer;
preferably, the liver cancer is hepatocellular carcinoma;
preferably, the renal tumor is renal cell carcinoma;
preferably, the breast cancer is triple negative breast cancer;
preferably, the urothelial cancer is bladder cancer.

## Claims

**1.** A bispecific antibody, comprising:

a first protein functional region targeting PD-1, and
a second protein functional region targeting VEGFA;
wherein the first protein functional region is a single chain antibody and the second protein functional region is an immunoglobulin;
wherein,
for the immunoglobulin, the heavy chain variable region comprises HCDR1-HCDR3 of amino acid sequences set forth in SEQ ID NOs: 28-30 respectively, and the light chain variable region comprises LCDR1-LCDR3 of amino acid sequences set forth in SEQ ID NOs: 31-33 respectively; for the single chain antibody, the heavy chain variable region comprises HCDR1-HCDR3 of amino acid sequences set forth in SEQ ID NOs: 34-36 respectively, and the light chain variable region comprises LCDR1-LCDR3 of amino acid sequences set forth in SEQ ID NOs: 37-39 respectively;
the immunoglobulin is of human IgG 1 subtype;
wherein the heavy chain of the immunoglobulin comprises the amino acid sequence set forth in SEQ ID NO: 40 with the following mutations, according to the EU numbering system:
L234A and L235A.

**2.** The bispecific antibody of claim 1, wherein the number of the first protein functional region is 2, and the number of the second protein functional region is 1.

**3.** The bispecific antibody of claim 1 or 2, wherein the single chain antibody is a scFv and wherein the bispecific antibody comprises the scFv linked to the C-terminus of a heavy chain of the immunoglobulin.

**4.** The bispecific antibody of claim 3, wherein the bispecific antibody is in the IgG-scFv form.

**5.** The bispecific antibody of any one of claims 1-4, wherein the light chain of the immunoglobulin comprises a human Ig kappa constant region, optionally wherein the amino acid sequence of the human Ig kappa constant region is set forth in SEQ ID NO: 42.

**6.** The bispecific antibody of any one of claims 1-5, wherein the amino acid sequence of the heavy chain of the immunoglobulin is set forth in SEQ ID NO: 24, and the amino acid sequence of the light chain of the immunoglobulin is set forth in SEQ ID NO: 26.

**7.** The bispecific antibody of any one of claims 1-6, wherein the amino acid sequence of the heavy chain variable region of the single chain antibody is set forth in SEQ ID NO: 9, and the amino acid sequence of the light chain variable region of the single chain antibody is set forth in SEQ ID NO: 17.

**8.** The bispecific antibody of any one of claims 1-7, wherein the single chain antibody is a scFv comprising the N-terminus of the heavy chain variable region of the scFv linked to the C-terminus of the heavy chain of the immunoglobulin, and the C-terminus of the heavy chain variable region of the scFv linked to the N-terminus of the light chain variable region of the scFv.

**9.** The bispecific antibody of any one of claims 1-8, wherein the single chain antibody is attached to the heavy chain of the immunoglobulin via a linker (i) and wherein the heavy chain variable region of the single chain antibody is attached to the light chain variable region of the single chain antibody via a linker (ii), wherein the linker of (i) and the linker of (ii) are each a (GGGGS)n amino acid sequence, n being a positive integer.

**10.** The bispecific antibody of claim 4, comprising the immunoglobulin targeting VEGF-A and two scFvs targeting PD-1, wherein:

(a) the amino acid sequence of the heavy chain of the immunoglobulin is set forth in SEQ ID NO: 24, and the amino acid sequence of the light chain of the immunoglobulin is set forth in SEQ ID NO: 26;
(b) for each of the two scFvs: the amino acid sequence of the heavy chain variable region is set forth in SEQ ID NO: 9, the amino acid sequence of the light chain variable region is set forth in SEQ ID NO: 17, the N-terminus of the heavy chain variable region of the scFv is attached via a linker (i) to the C-terminus of one of the two heavy chains of the immunoglobulin, and the C-terminus of the heavy chain variable region of the scFv is attached via a linker (ii) to the N-terminus of the light chain variable region of the scFv; and
(c) the amino acid sequence of linker (i) and the amino acid sequence of the linker (ii) are the same or different.

**11.** The bispecific antibody of claim 10, wherein the amino acid sequence of the linker of (i) is set forth in SEQ ID NO:18 and the amino acid sequence of the linker of (ii) is set forth in SEQ ID NO: 18.

**12.** An isolated nucleic acid molecule, encoding the bispecific antibody according to any one of claims 1-11.

**13.** A vector, comprising the isolated nucleic acid molecule according to claim 12.

**14.** A host cell, comprising the isolated nucleic acid molecule according to claim 12 or the vector according to claim 13.

**15.** The bispecific antibody according to any one of claims 1-11, for use as a medicament,

preferably wherein the bispecific antibody is for use in the treatment and/or prevention of a malignant tumor in a subject,
more preferably wherein the malignant tumor is selected from colon cancer, rectal cancer, lung cancer, liver cancer, ovarian cancer, skin cancer, glioma, melanoma, lymphoma, renal tumor, prostate cancer, bladder cancer, gastrointestinal cancer, breast cancer, brain cancer, cervical cancer, esophageal cancer, microsatellite instability-high (MSI-H) and deficient mismatch repair (dMMR) cancer, urothelial cancer, mesothelioma, endometrial cancer, gastric adenocarcinoma, gastroesophageal junction adenocarcinoma and leukemia,
even more preferably wherein the lung cancer is non-small cell lung cancer or small cell lung cancer, optionally wherein the non-small cell lung cancer is EGFR and/or ALK sensitive mutant non-small cell lung cancer.

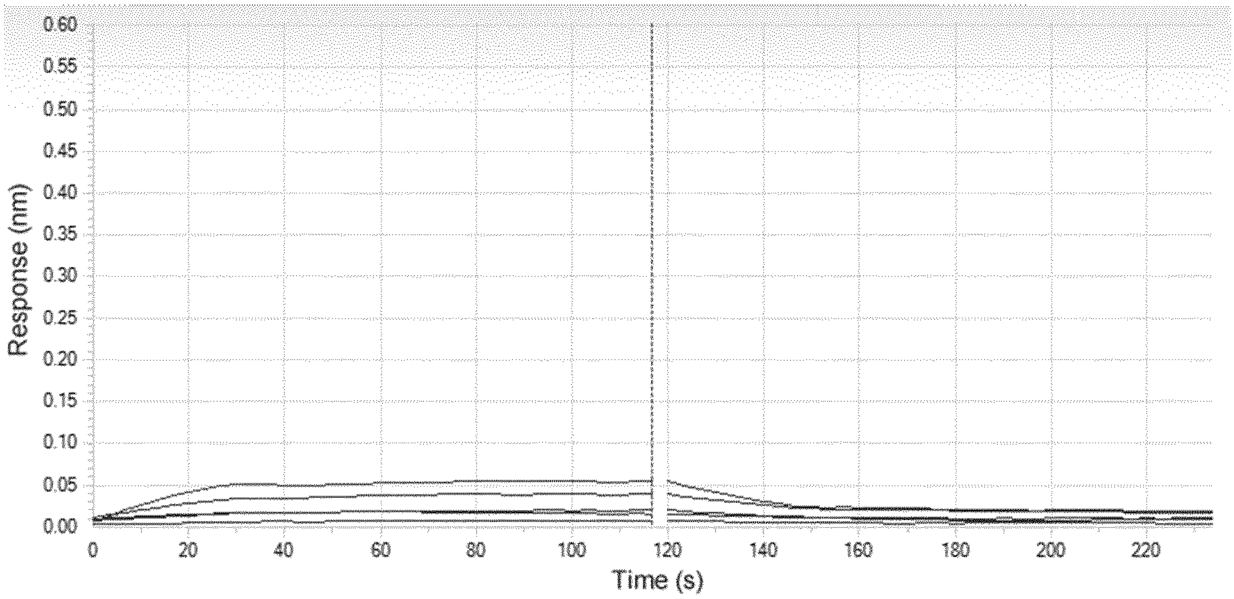
0.60
0.55
0.50
0.45
0.40
0.35
0.30
0.25
0.20
0.15
0.10
0.05
0.00
Response (nm)
0
20
40
60
80
100
120
140
160
180
200
220
Time (s)

FIG. 1

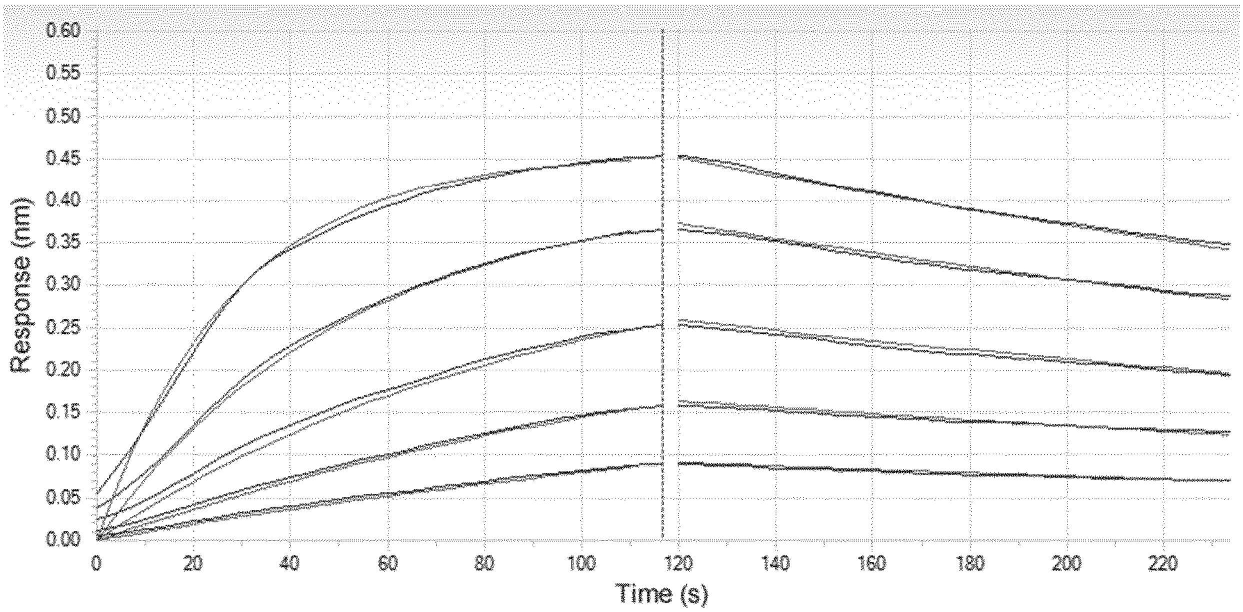
0.60
0.55
0.50
0.45
0.40
0.35
0.30
0.25
0.20
0.15
0.10
0.05
0.00
Response (nm)
0
20
40
60
80
100
120
140
160
180
200
220
Time (s)

FIG. 2

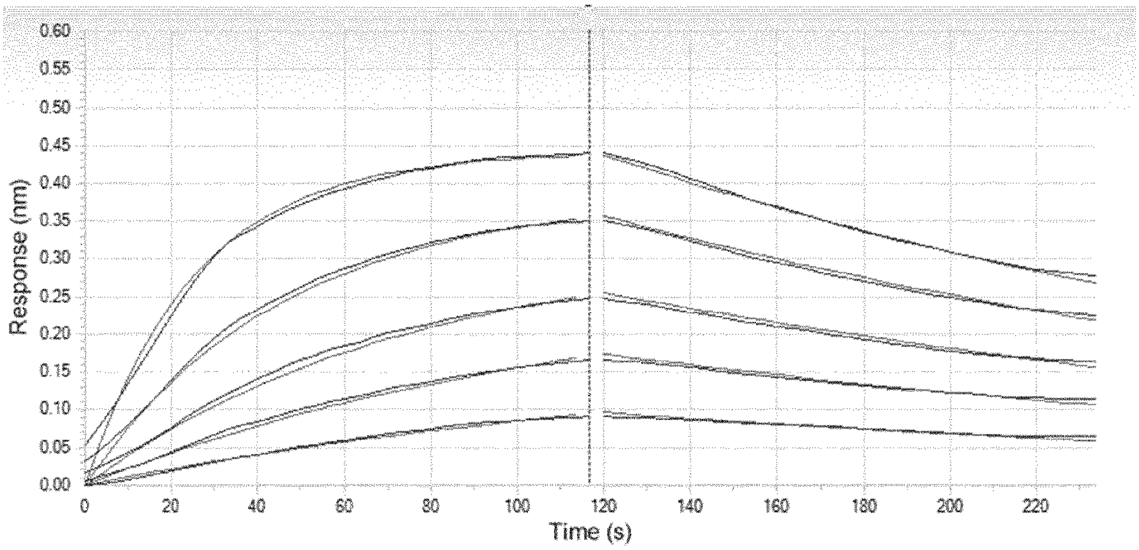
0.60
0.55
0.50
0.45
0.40
0.35
0.30
0.25
0.20
0.15
0.10
0.05
0.00
Response (nm)
0
20
40
60
80
100
120
140
160
180
200
220
Time (s)
FIG. 3

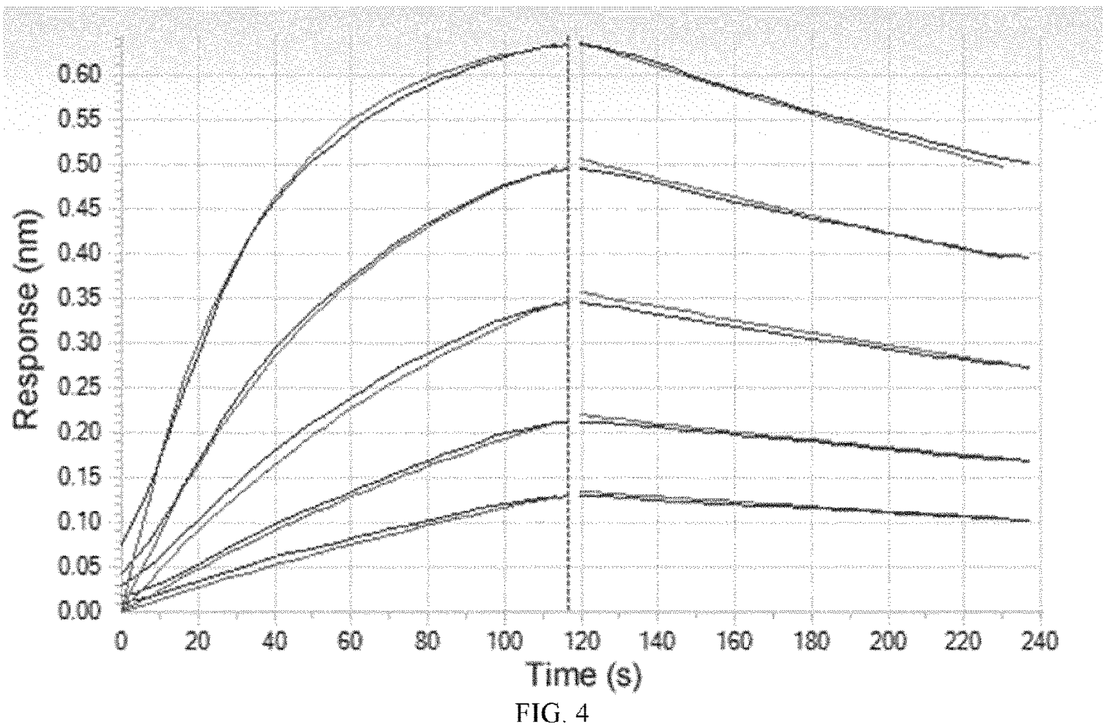
0.60
0.55
0.50
0.45
0.40
0.35
0.30
0.25
0.20
0.15
0.10
0.05
0.00
Response (nm)
0
20
40
60
80
100
120
140
160
180
200
220
240
Time (s)
FIG. 4

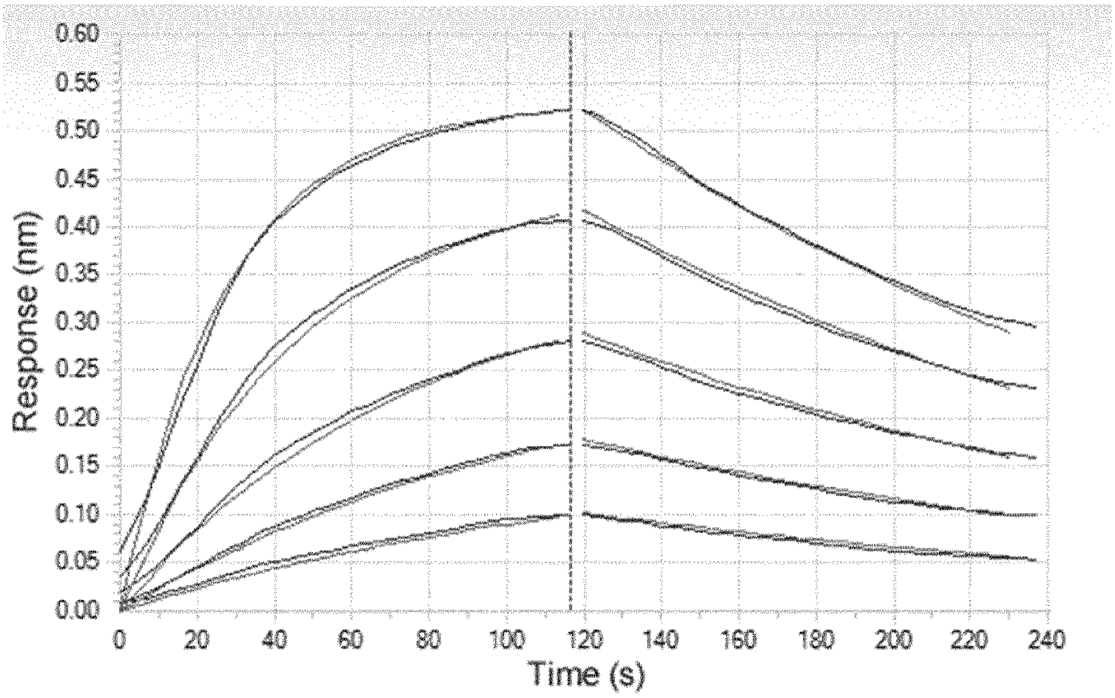
0.60
0.55
0.50
0.45
0.40
0.35
0.30
0.25
0.20
0.15
0.10
0.05
0.00
Response (nm)
0
20
40
60
80
100
120
140
160
180
200
220
240
Time (s)
FIG. 5

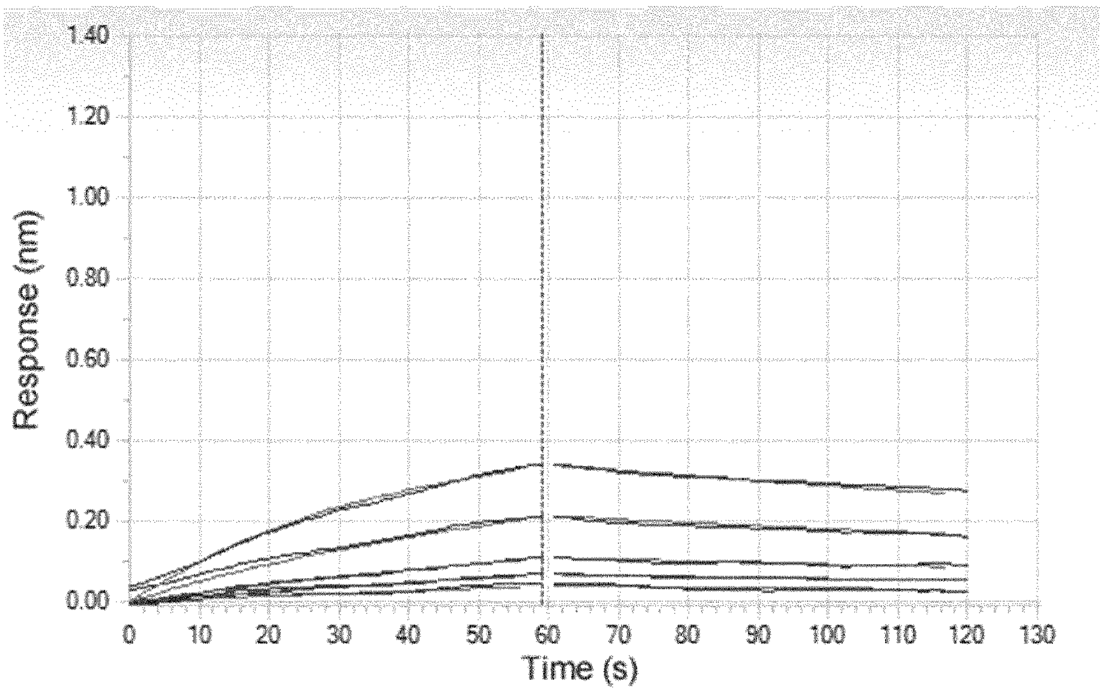
1.40
1.20
1.00
0.80
0.60
0.40
0.20
0.00
Response (nm)
0
10
20
30
40
50
60
70
80
90
100
110
120
130
Time (s)
FIG. 6

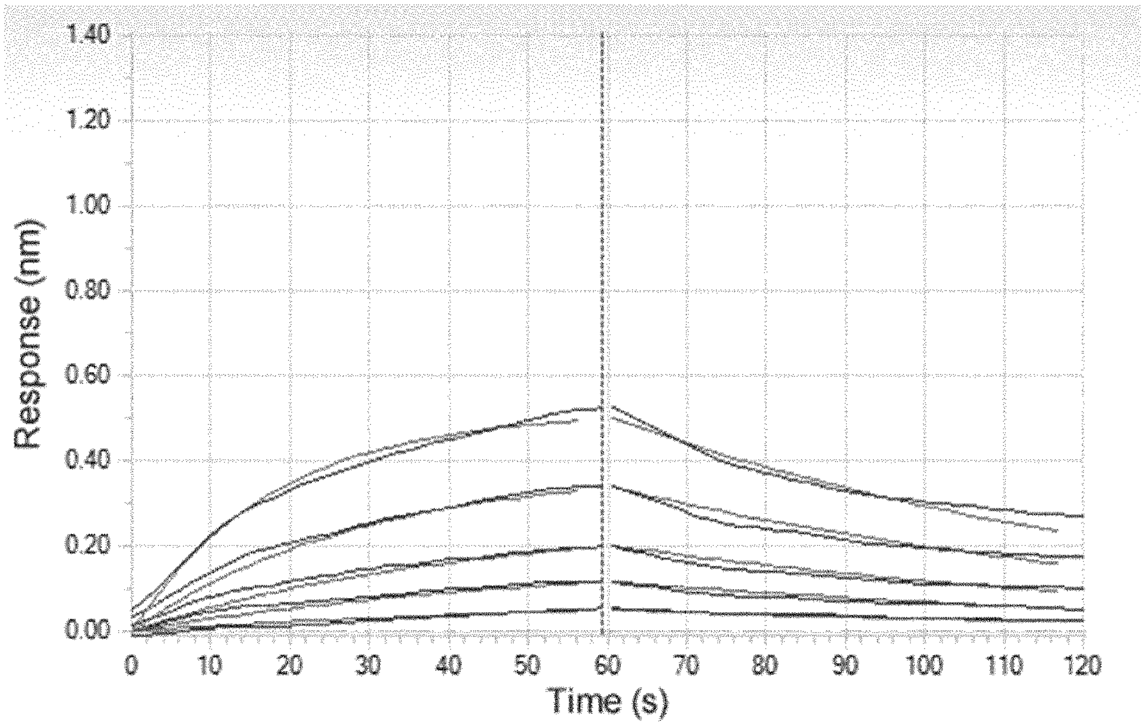
1.40
1.20
1.00
0.80
0.60
0.40
0.20
0.00
Response (nm)
0
10
20
30
40
50
60
70
80
90
100
110
120
Time (s)

FIG. 7

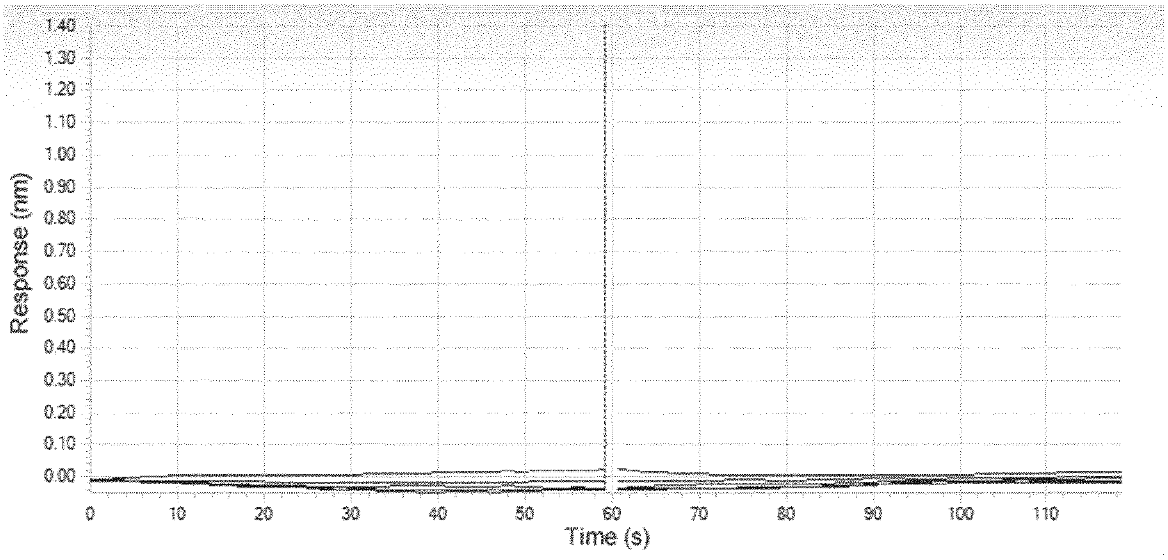
1.40
1.30
1.20
1.10
1.00
0.90
0.80
0.70
0.60
0.50
0.40
0.30
0.20
0.10
0.00
Response (nm)
0
10
20
30
40
50
60
70
80
90
100
110
Time (s)

FIG. 8

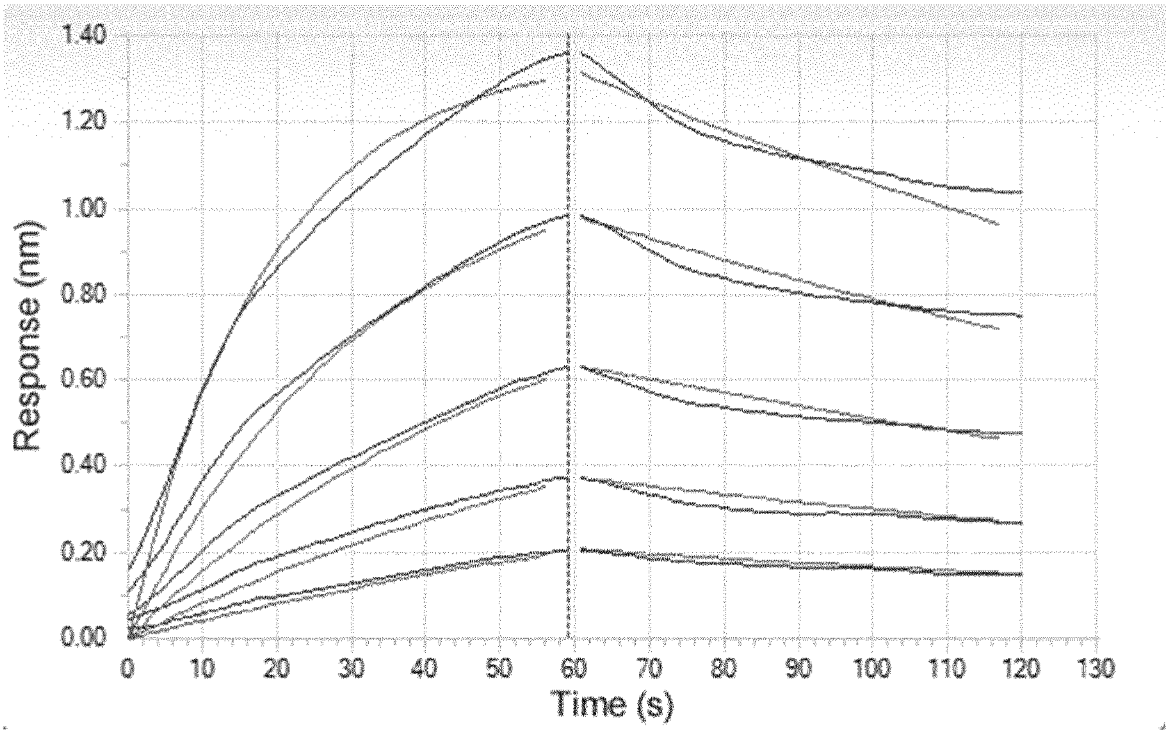
1.40
1.20
1.00
0.80
0.60
0.40
0.20
0.00
Response (nm)
0
10
20
30
40
50
60
70
80
90
100
110
120
130
Time (s)

FIG. 9

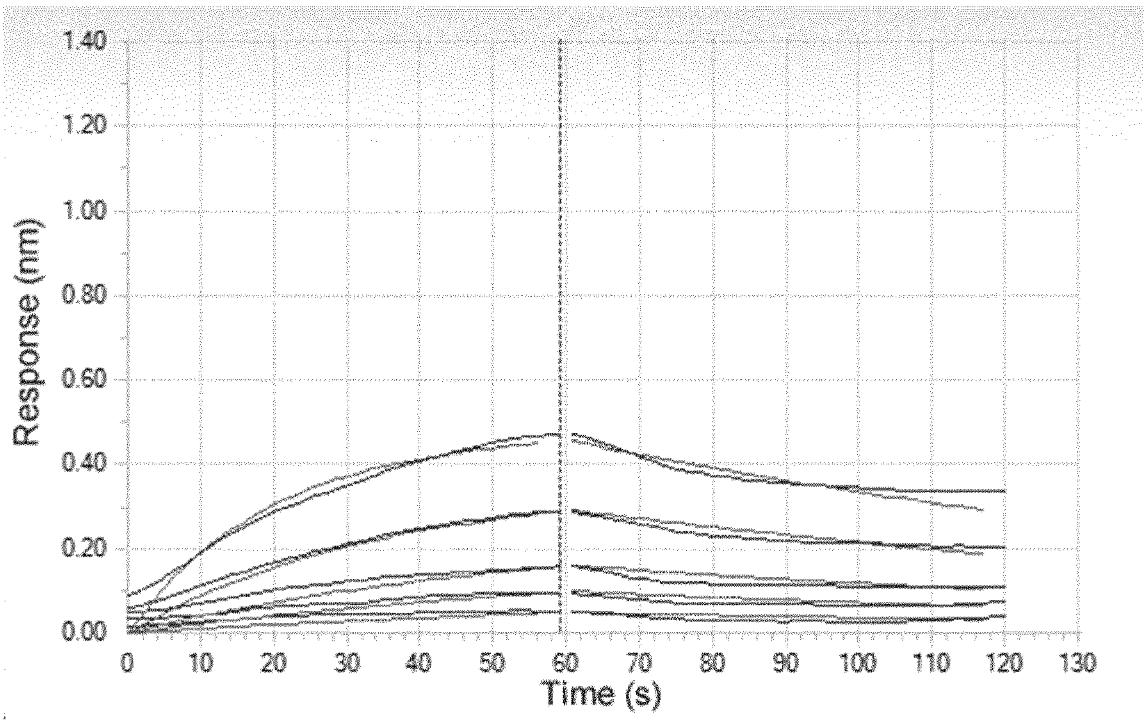
1.40
1.20
1.00
0.80
0.60
0.40
0.20
0.00
Response (nm)
0
10
20
30
40
50
60
70
80
90
100
110
120
130
Time (s)

FIG. 10

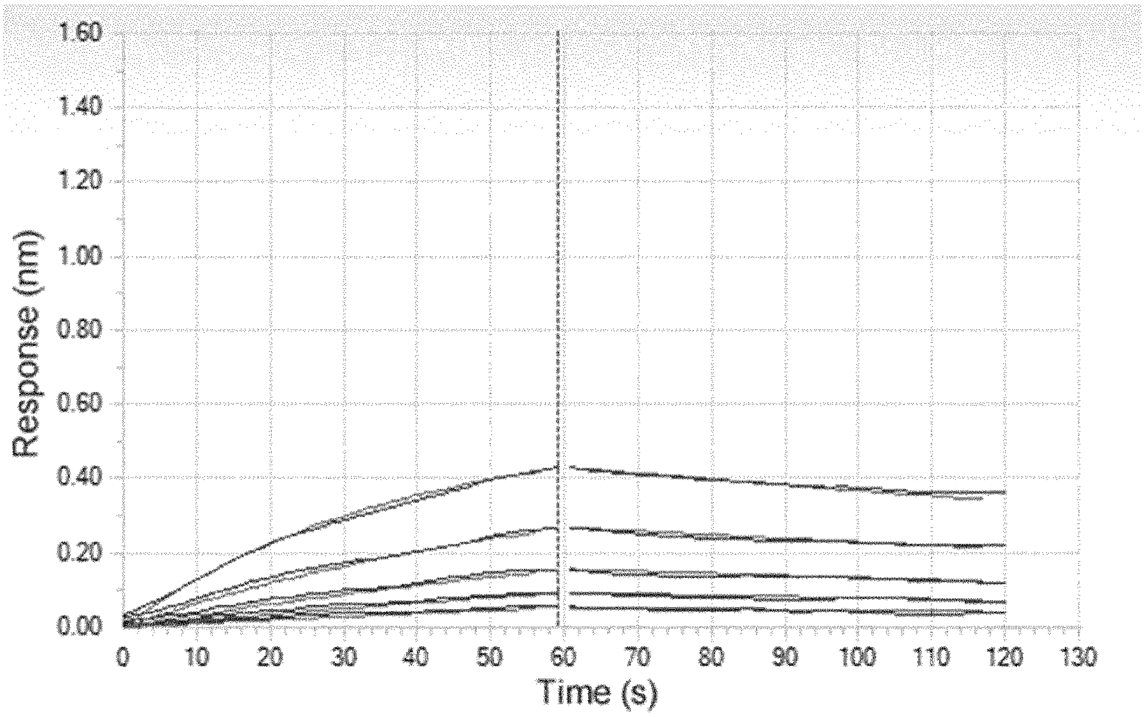
1.60
1.40
1.20
1.00
0.80
0.60
0.40
0.20
0.00
Response (nm)
0
10
20
30
40
50
60
70
80
90
100
110
120
130
Time (s)

FIG. 11

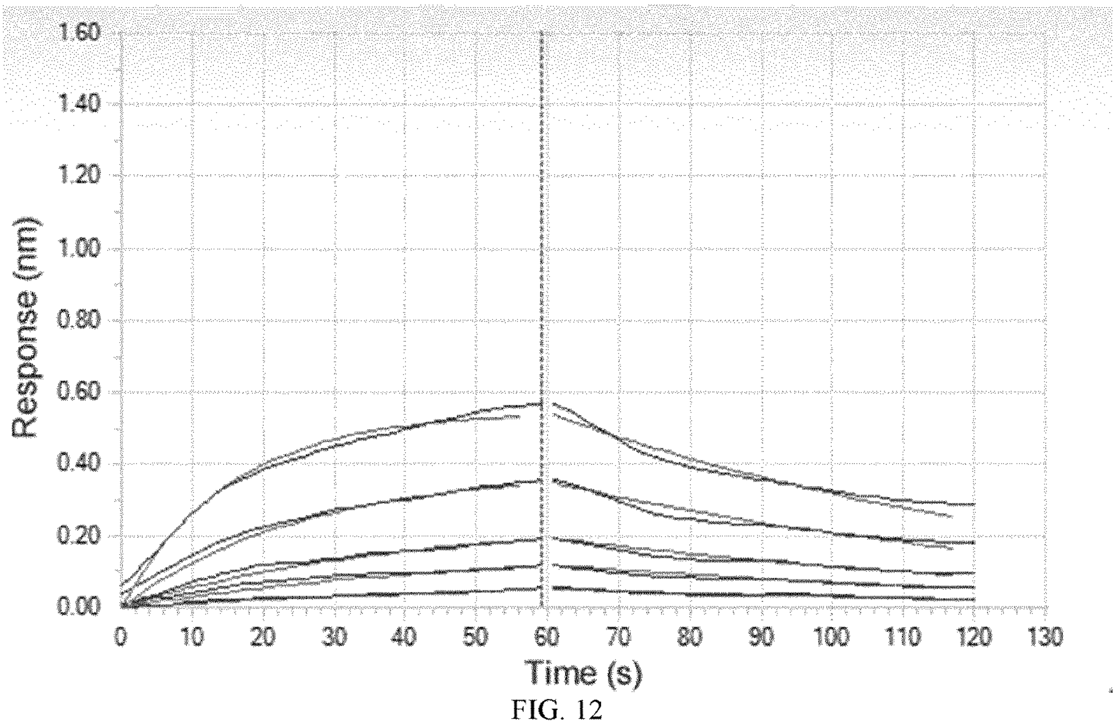
1.60
1.40
1.20
1.00
0.80
0.60
0.40
0.20
0.00
Response (nm)
0
10
20
30
40
50
60
70
80
90
100
110
120
130
Time (s)

FIG. 12

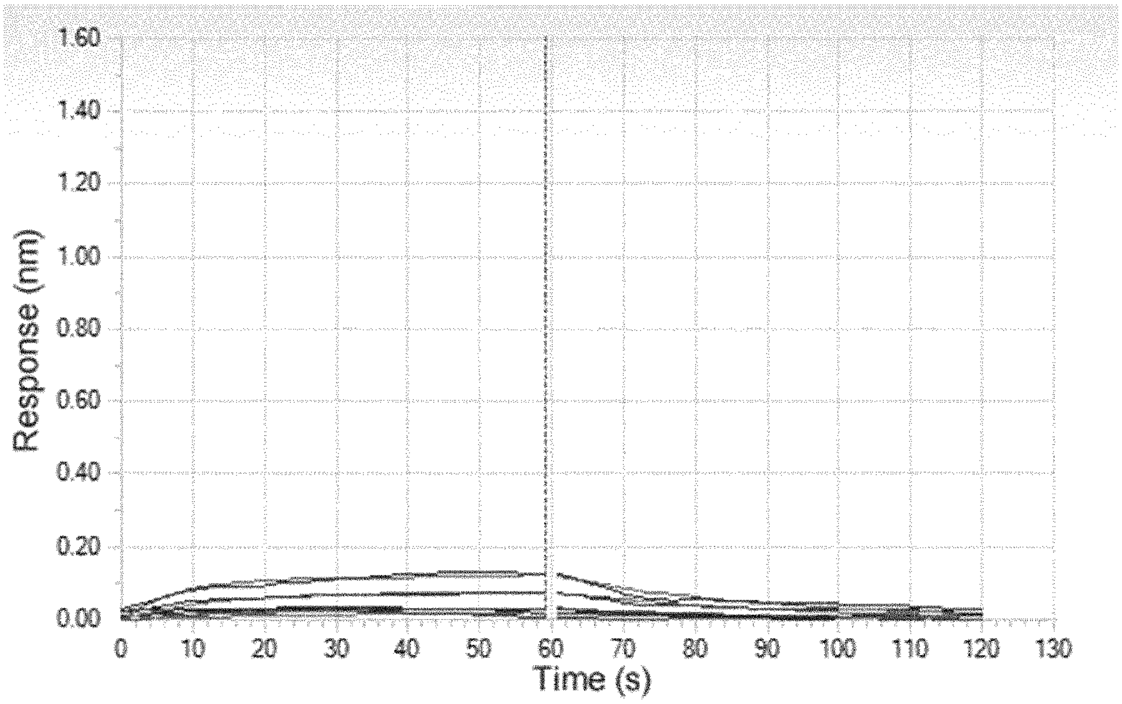
1.60
1.40
1.20
1.00
0.80
0.60
0.40
0.20
0.00
Response (nm)
0
10
20
30
40
50
60
70
80
90
100
110
120
130
Time (s)

FIG. 13

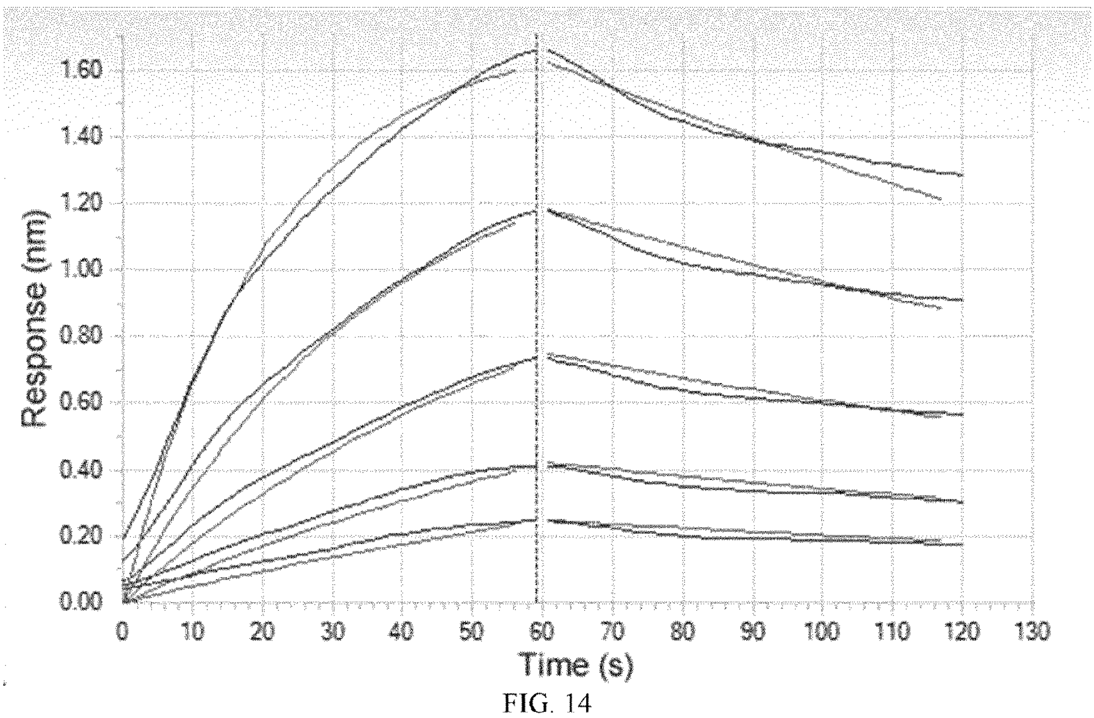
1.60
1.40
1.20
1.00
0.80
0.60
0.40
0.20
0.00
Response (nm)
0
10
20
30
40
50
60
70
80
90
100
110
120
130
Time (s)

FIG. 14

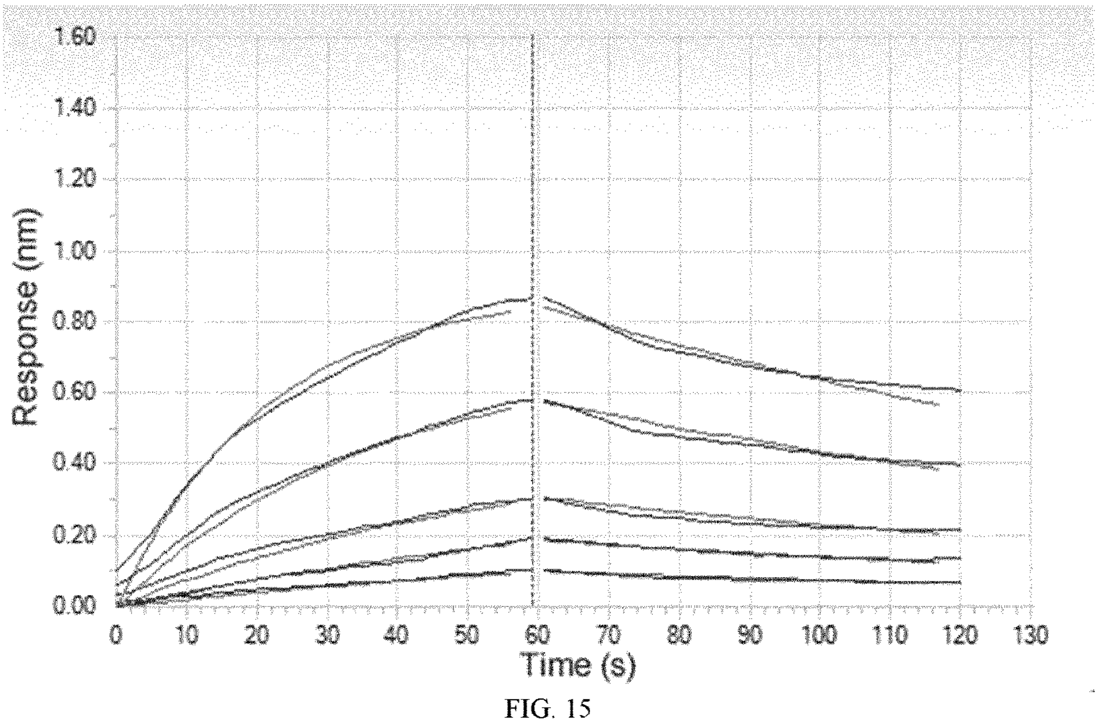
1.60
1.40
1.20
1.00
0.80
0.60
0.40
0.20
0.00
Response (nm)
0
10
20
30
40
50
60
70
80
90
100
110
120
130
Time (s)

FIG. 15

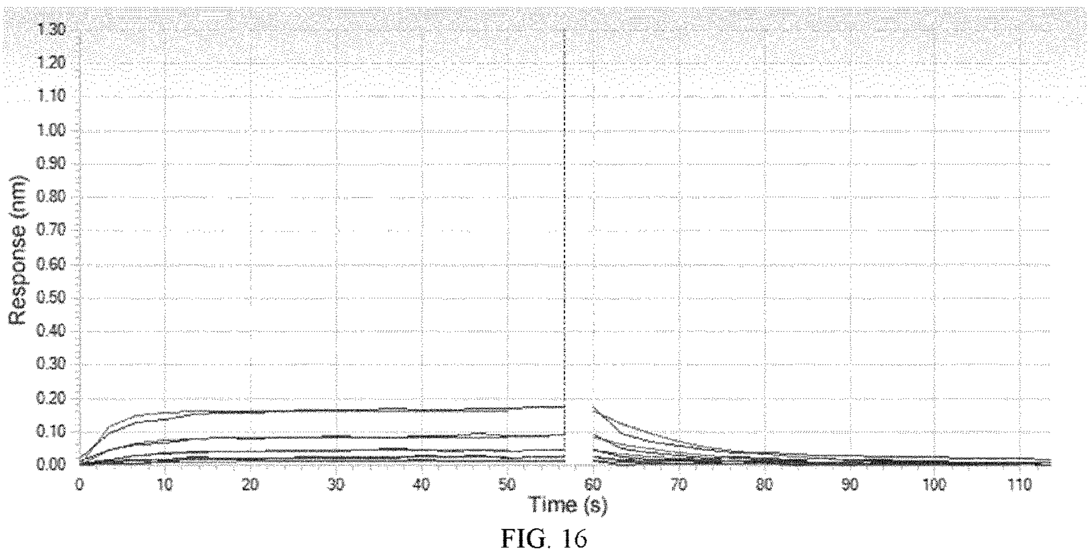
1.30
1.20
1.10
1.00
0.90
0.80
0.70
0.60
0.50
0.40
0.30
0.20
0.10
0.00
Response (nm)
0
10
20
30
40
50
60
70
80
90
100
110
Time (s)

FIG. 16

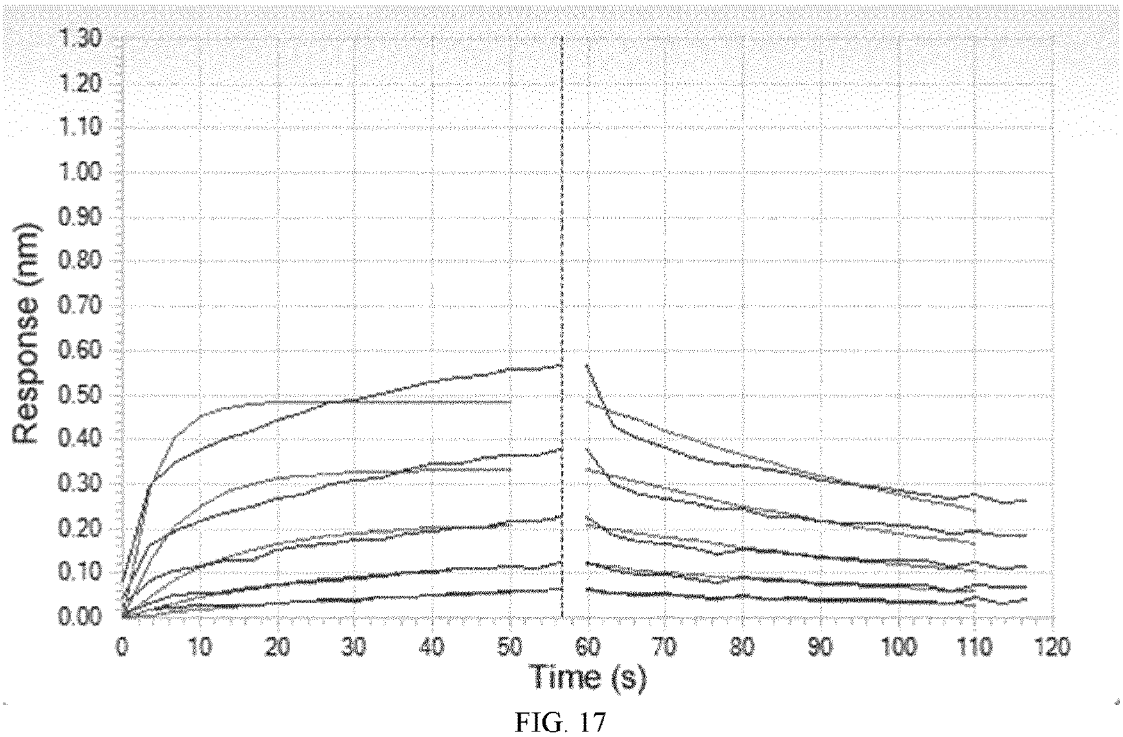
1.30
1.20
1.10
1.00
0.90
0.80
0.70
0.60
0.50
0.40
0.30
0.20
0.10
0.00
Response (nm)
0 10 20 30 40 50 60 70 80 90 100 110 120
Time (s)
FIG. 17

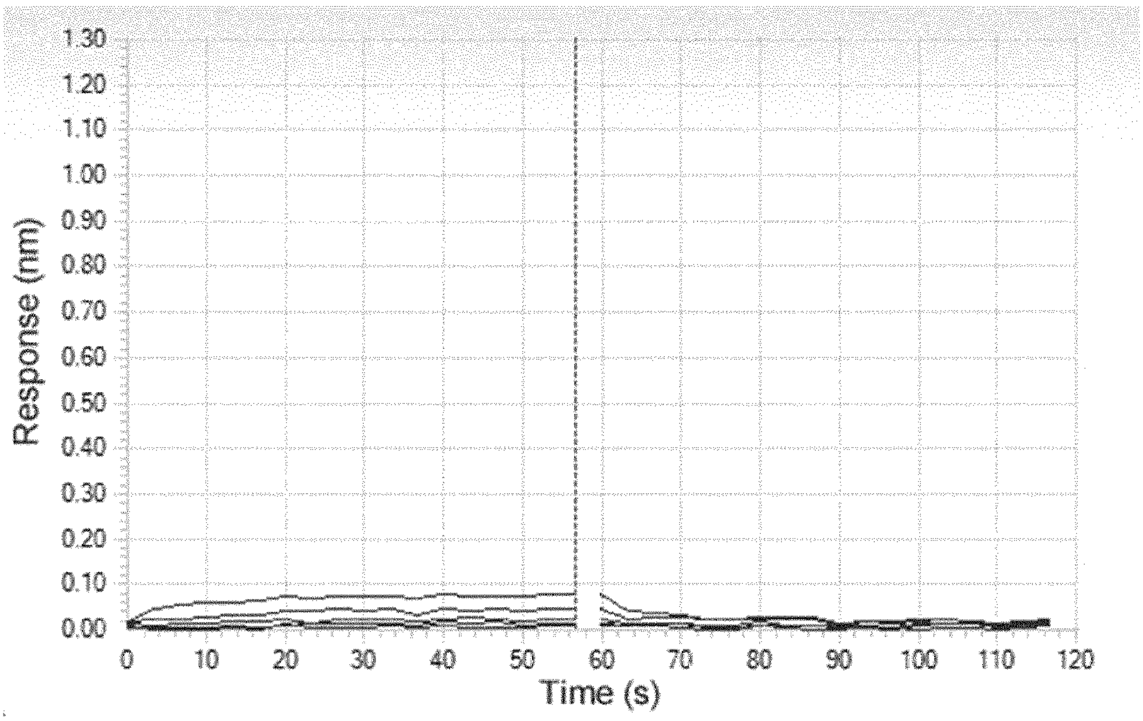
1.30
1.20
1.10
1.00
0.90
0.80
0.70
0.60
0.50
0.40
0.30
0.20
0.10
0.00
Response (nm)
0 10 20 30 40 50 60 70 80 90 100 110 120
Time (s)
FIG. 18

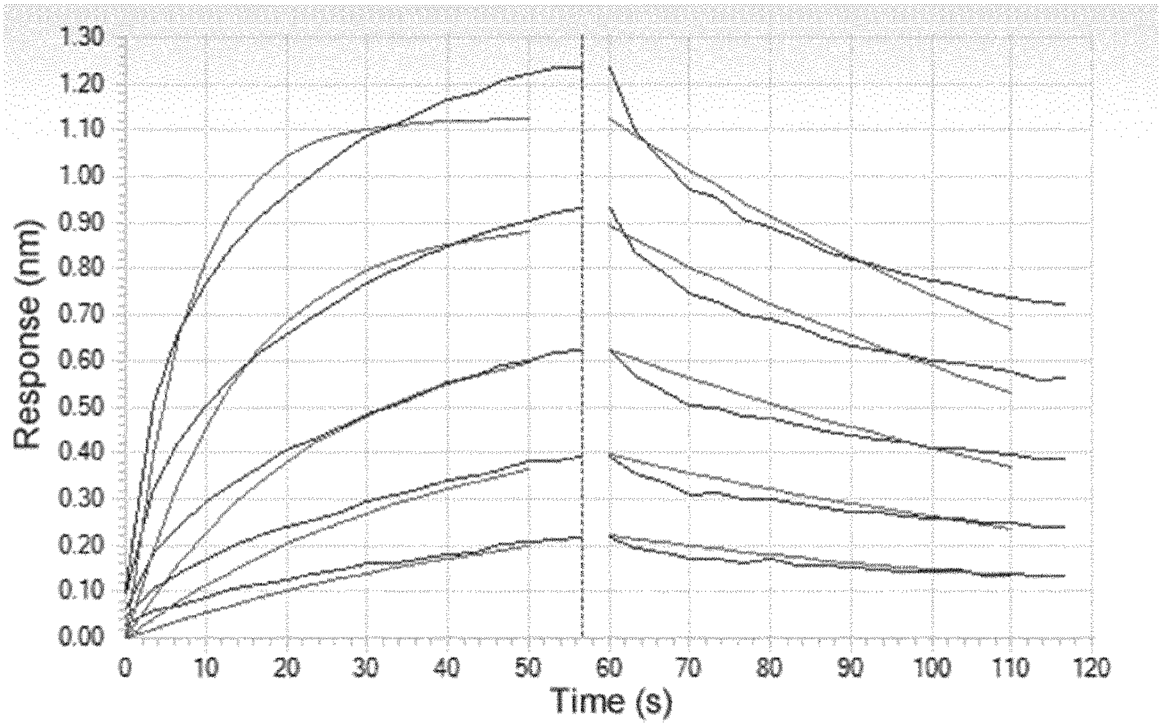
1.30
1.20
1.10
1.00
0.90
0.80
0.70
0.60
0.50
0.40
0.30
0.20
0.10
0.00
Response (nm)
0
10
20
30
40
50
60
70
80
90
100
110
120
Time (s)
FIG. 19

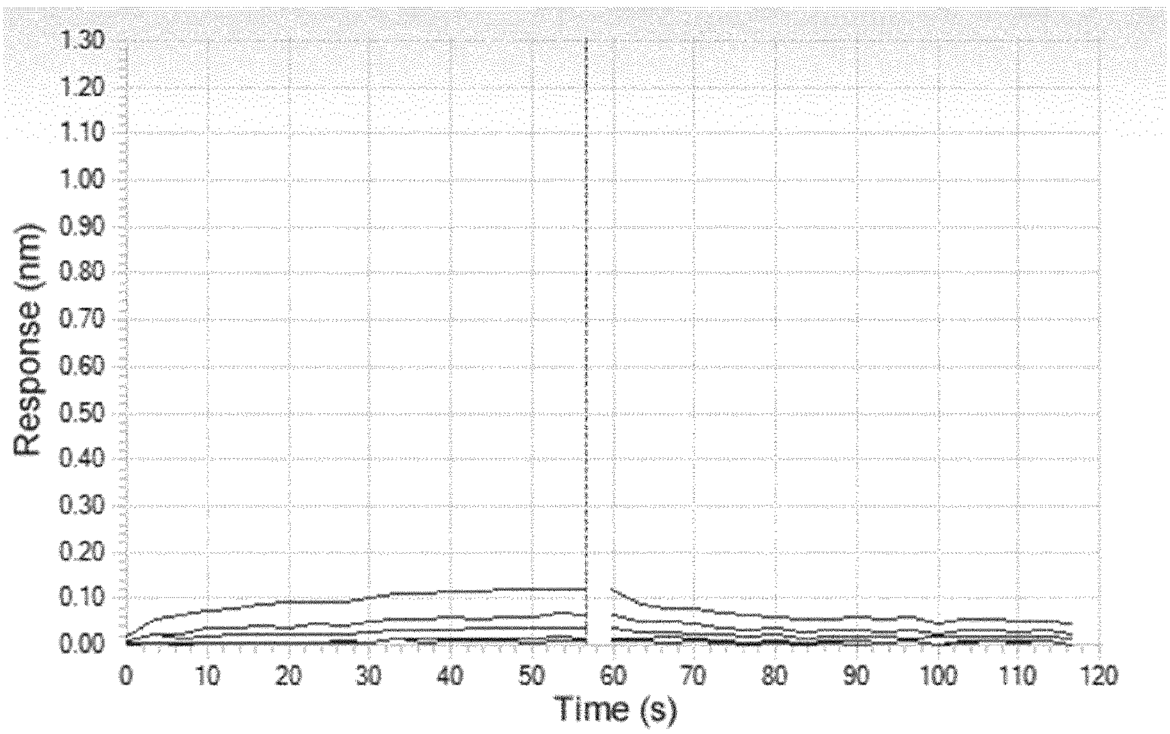
1.30
1.20
1.10
1.00
0.90
0.80
0.70
0.60
0.50
0.40
0.30
0.20
0.10
0.00
Response (nm)
0
10
20
30
40
50
60
70
80
90
100
110
120
Time (s)
FIG. 20

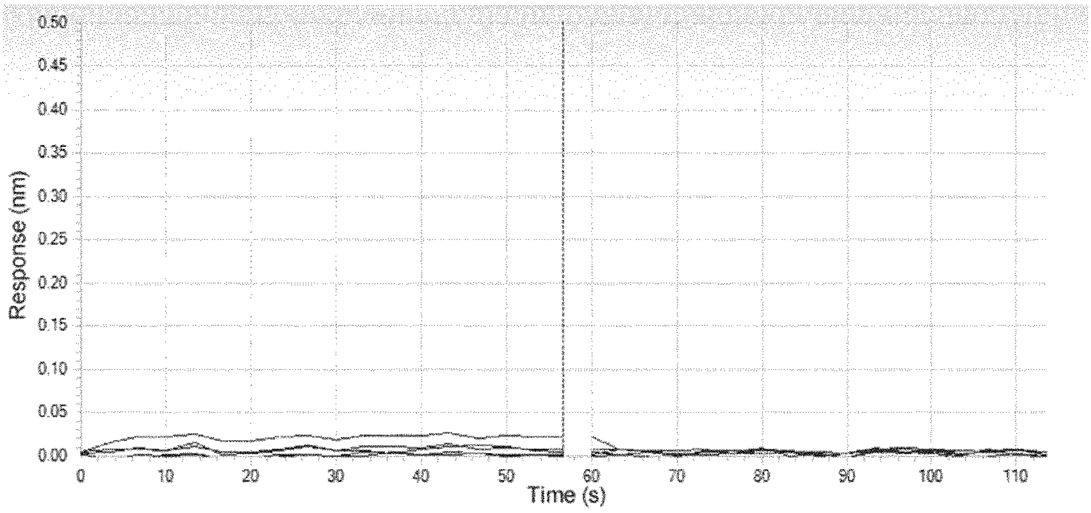
0.50
0.45
0.40
0.35
0.30
0.25
0.20
0.15
0.10
0.05
0.00
Response (nm)
0
10
20
30
40
50
60
70
80
90
100
110
Time (s)
FIG. 21

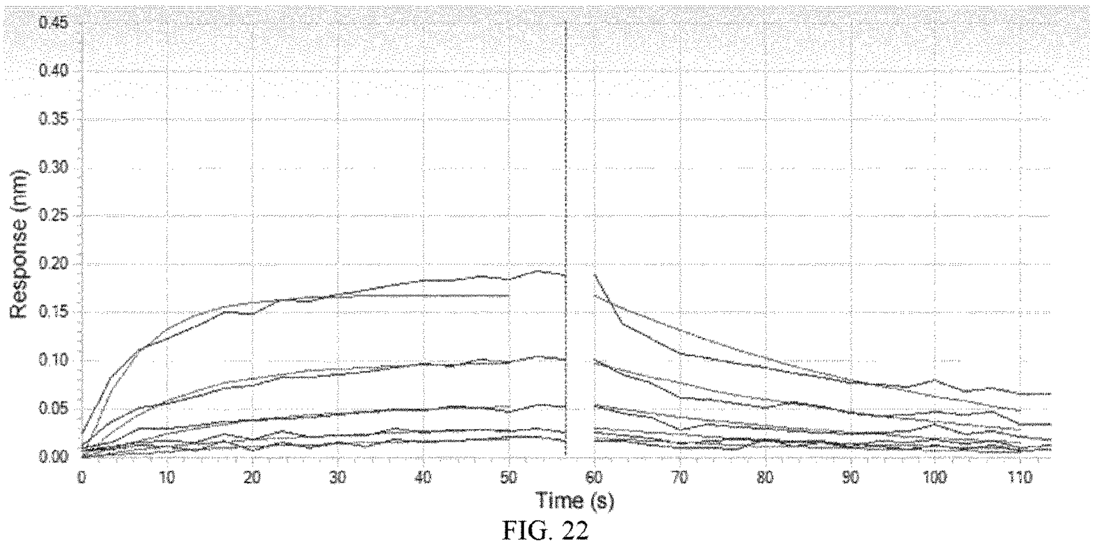
0.45
0.40
0.35
0.30
0.25
0.20
0.15
0.10
0.05
0.00
Response (nm)
0
10
20
30
40
50
60
70
80
90
100
110
Time (s)
FIG. 22

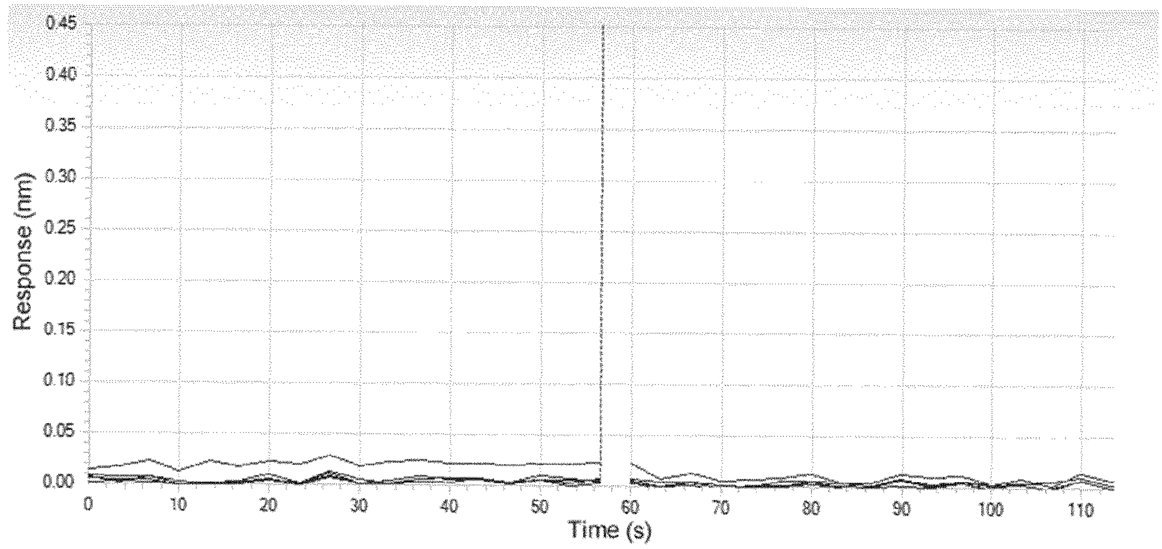


FIG. 23

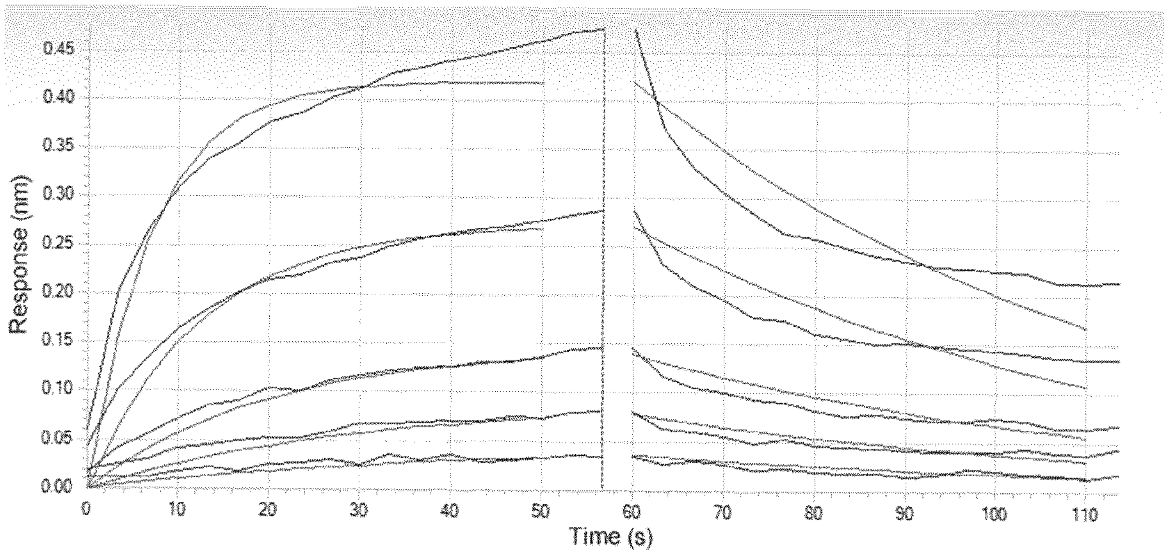


FIG. 24

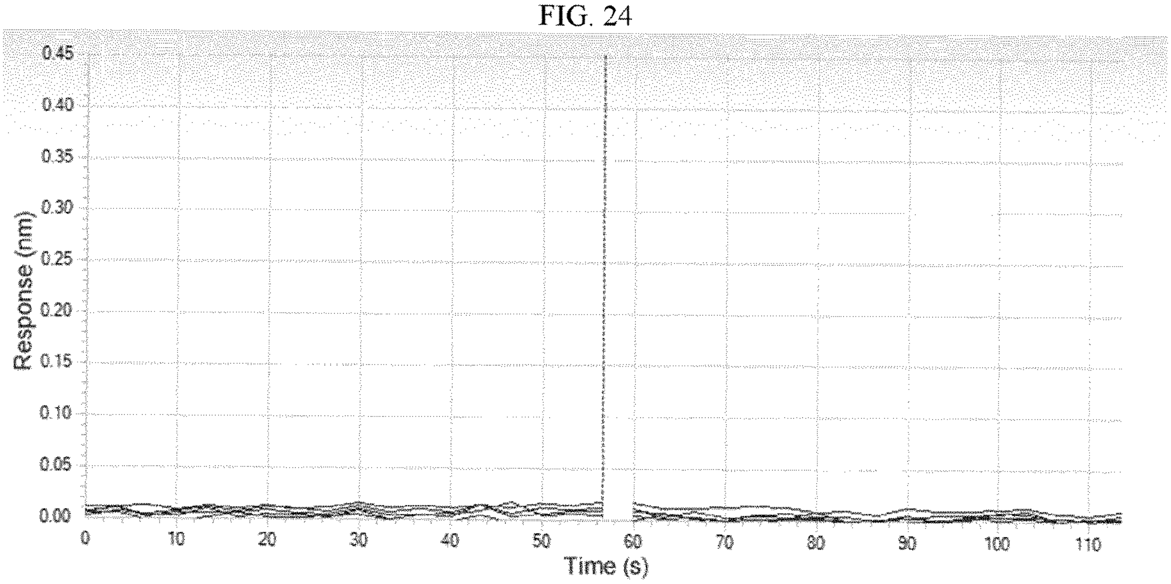


FIG. 25

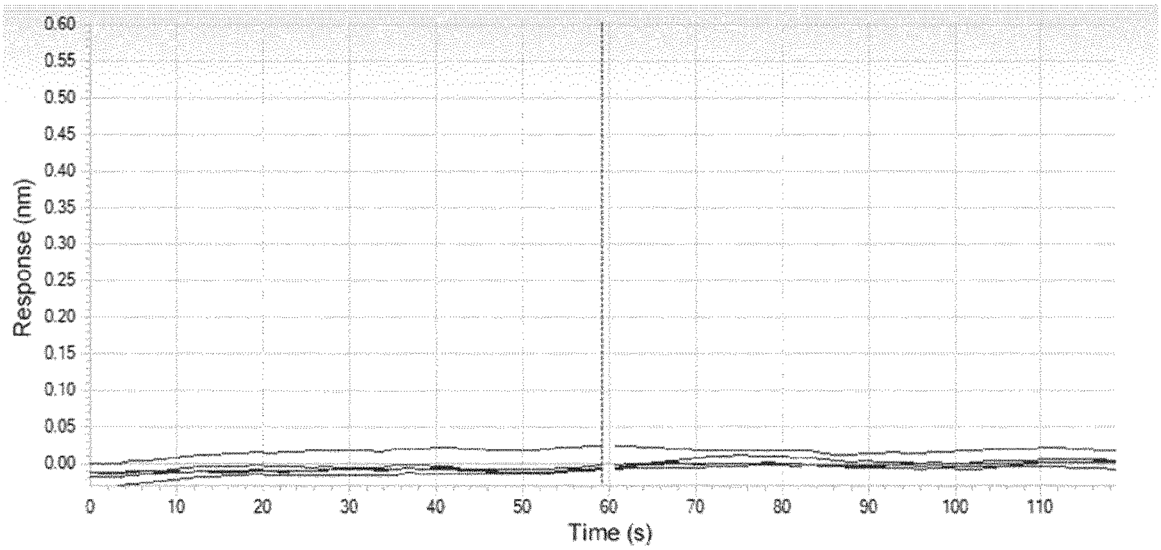

0.60
0.55
0.50
0.45
0.40
0.35
0.30
0.25
0.20
0.15
0.10
0.05
0.00
Response (nm)
0
10
20
30
40
50
60
70
80
90
100
110
Time (s)

FIG. 26

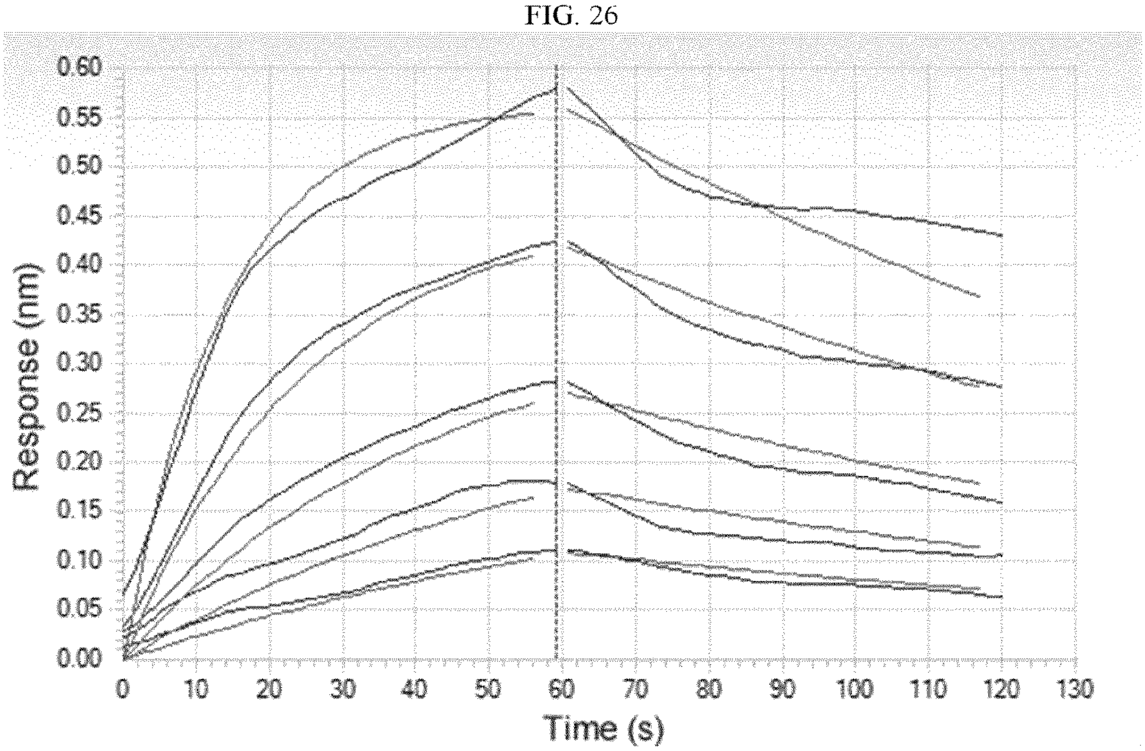

0.60
0.55
0.50
0.45
0.40
0.35
0.30
0.25
0.20
0.15
0.10
0.05
0.00
Response (nm)
0
10
20
30
40
50
60
70
80
90
100
110
120
130
Time (s)

FIG. 27

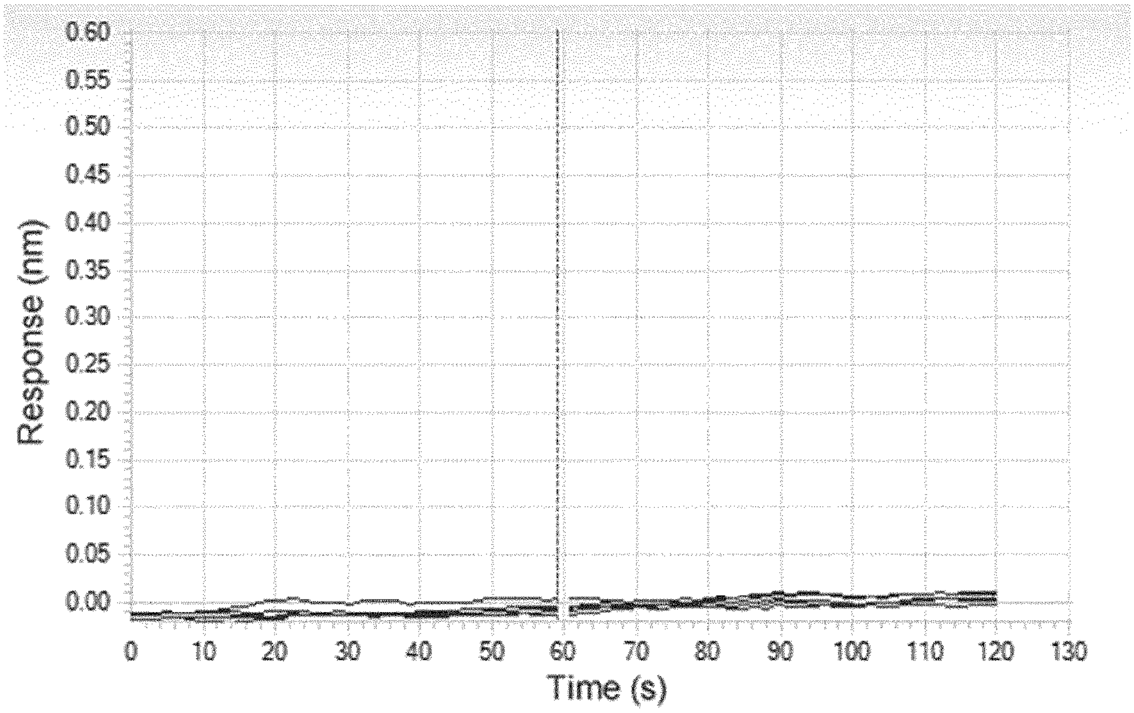
0.60
0.55
0.50
0.45
0.40
0.35
0.30
0.25
0.20
0.15
0.10
0.05
0.00
Response (nm)
0
10
20
30
40
50
60
70
80
90
100
110
120
130
Time (s)

FIG. 28

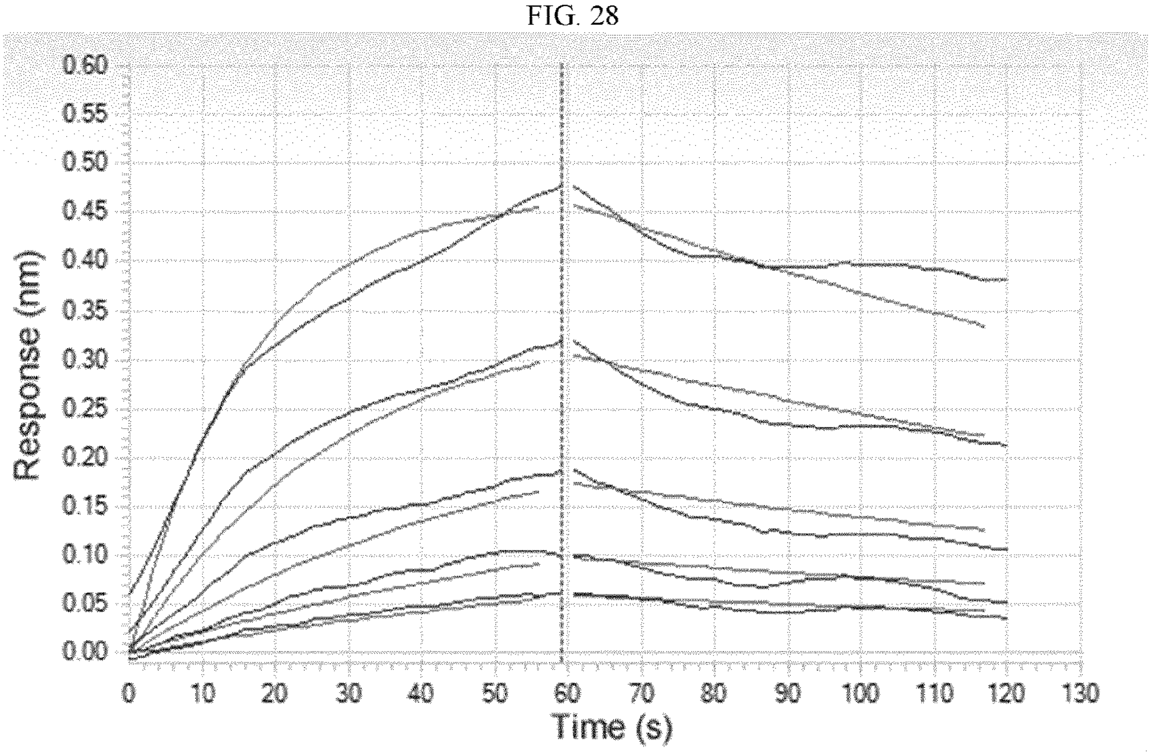
0.60
0.55
0.50
0.45
0.40
0.35
0.30
0.25
0.20
0.15
0.10
0.05
0.00
Response (nm)
0
10
20
30
40
50
60
70
80
90
100
110
120
130
Time (s)

FIG. 29

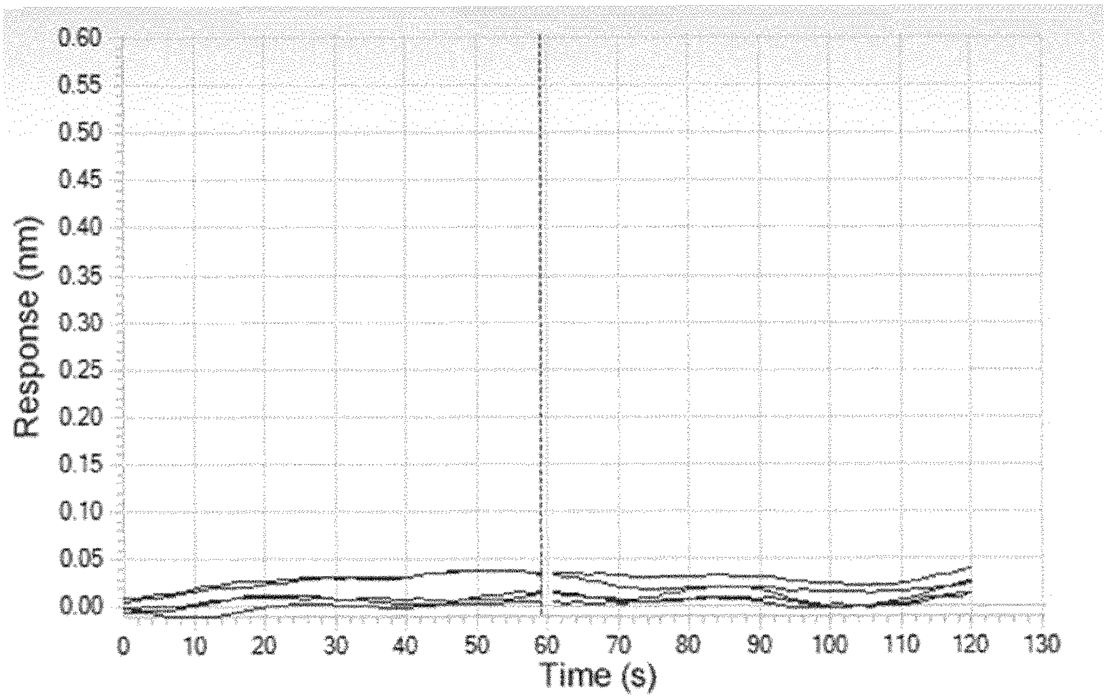
Response (nm)
0.60
0.55
0.50
0.45
0.40
0.35
0.30
0.25
0.20
0.15
0.10
0.05
0.00
0 10 20 30 40 50 60 70 80 90 100 110 120 130
Time (s)

FIG. 30

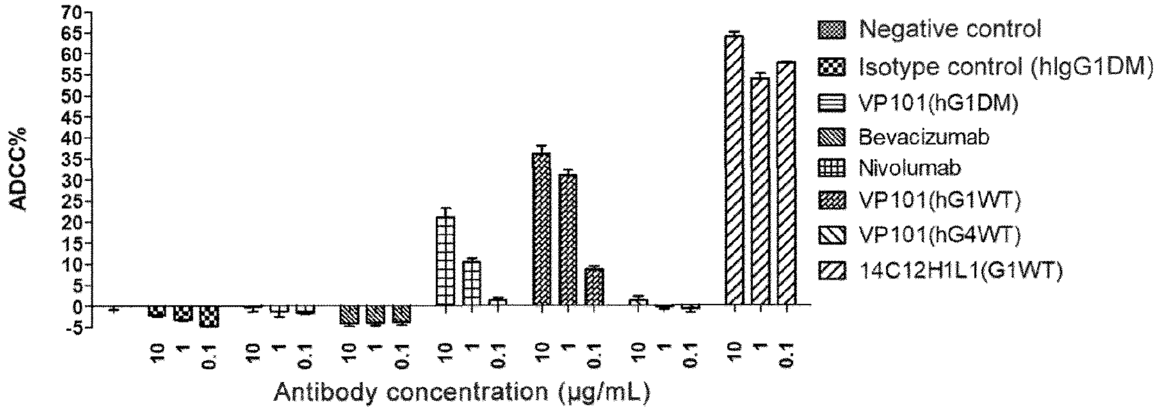
ADCC%
70 65 60 55 50 45 40 35 30 25 20 15 10 5 0 -5
10 1 0.1 10 1 0.1 10 1 0.1 10 1 0.1 10 1 0.1 10 1 0.1 10 1 0.1
Antibody concentration (μg/mL)
Negative control
Isotype control (hIgG1DM)
VP101(hG1DM)
Bevacizumab
Nivolumab
VP101(hG1WT)
VP101(hG4WT)
14C12H1L1(G1WT)

FIG. 31

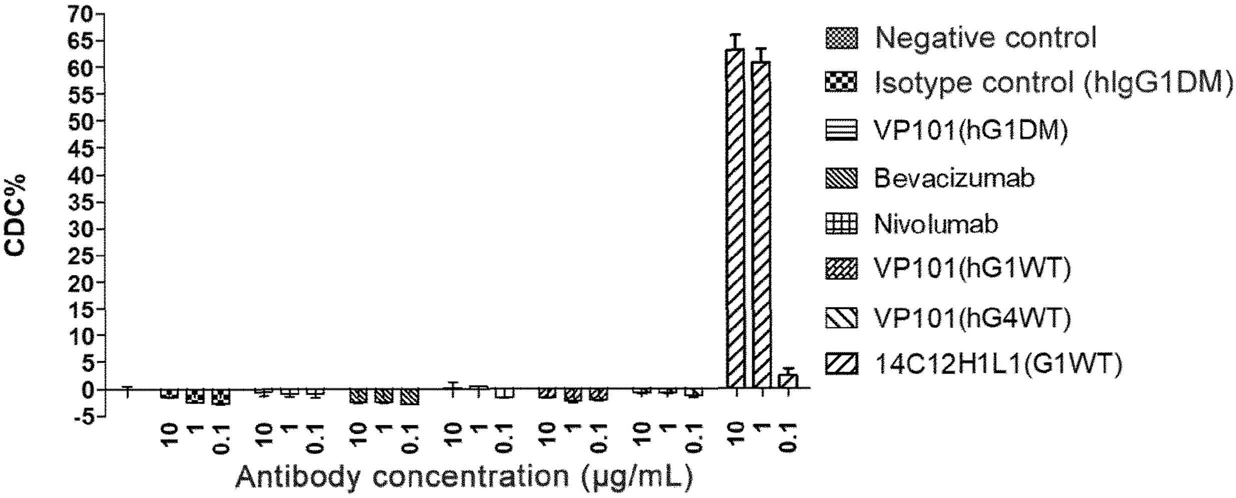


FIG. 32

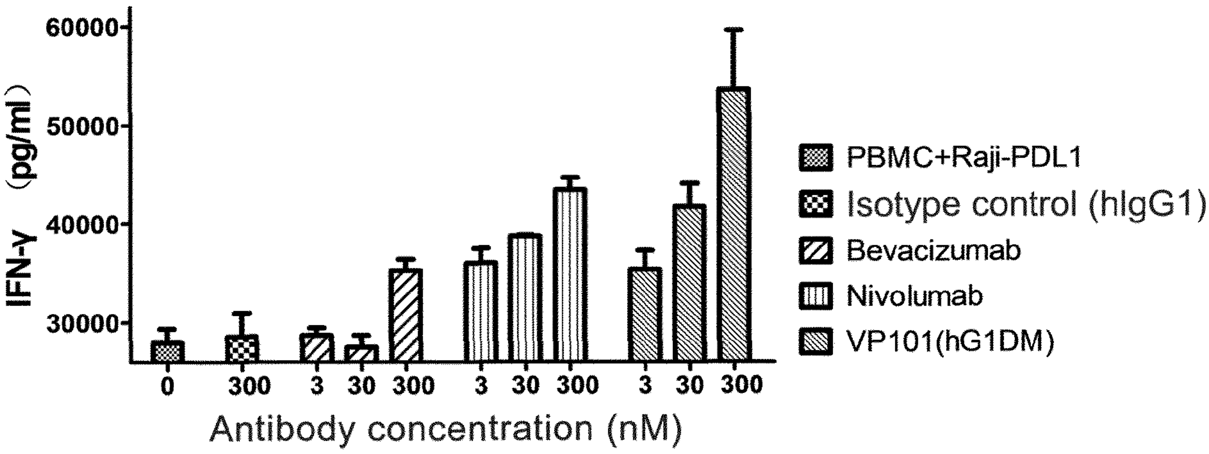


FIG. 33

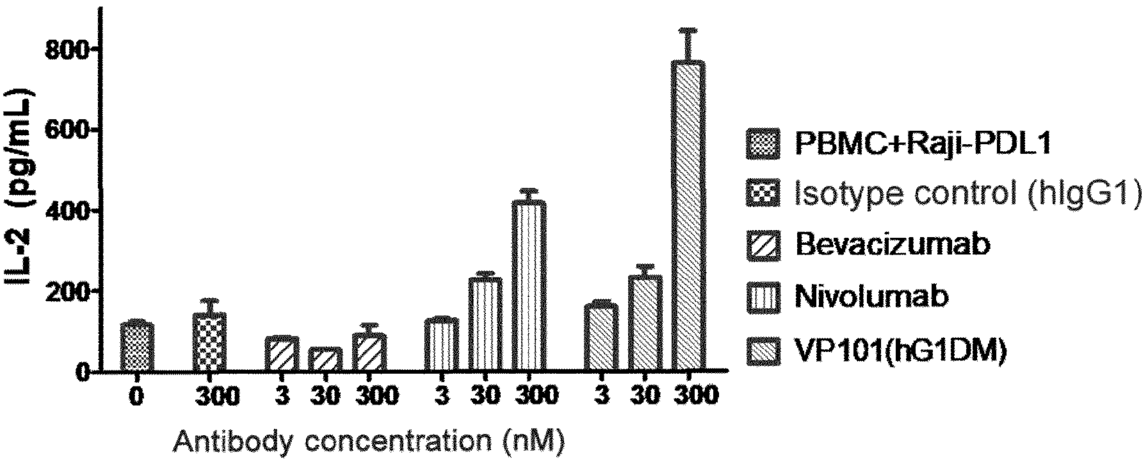


FIG. 34

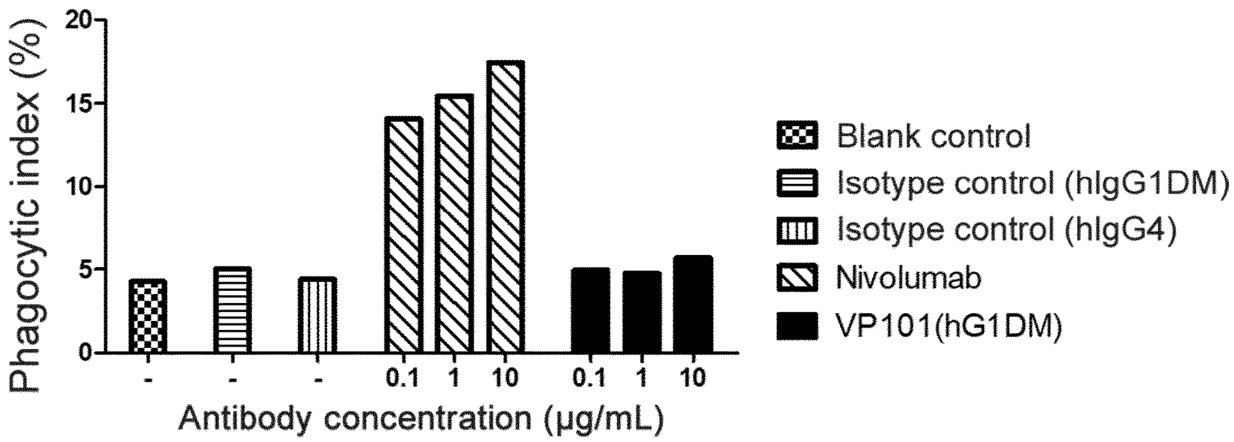
Phagocytic index (%)
20
15
10
5
0
-
-
-
0.1
1
10
0.1
1
10
Antibody concentration (μg/mL)
Blank control
Isotype control (hIgG1DM)
Isotype control (hIgG4)
Nivolumab
VP101(hG1DM)

FIG. 35

European Patent Office / Europäisches Patentamt / Office européen des brevets

## EUROPEAN SEARCH REPORT

Application Number
EP 25 22 2544

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CN 109 053 895 A (AKESO BIOPHARMA INC) 21 December 2018 (2018-12-21)<br>* sequences 15-26 *<br>* page 26; example 1; table 1 * | 1-15 | INV.<br>C07K16/46<br>C12N15/13<br>A61K39/395<br>A61P35/00<br>C07K16/22<br>A61P35/02<br>C07K16/28 |
| A | MARJAN HEZAREH ET AL: "Effector function activities of a panel of mutants of a broadly neutralizing antibody against human immunodeficiency virus type 1", JOURNAL OF VIROLOGY, THE AMERICAN SOCIETY FOR MICROBIOLOGY, US, vol. 75, no. 24, 1 December 2001 (2001-12-01), pages 12161-12168, XP002635226, ISSN: 0022-538X, DOI: 10.1128/JVI.75.24.12161-12168.2001<br>* abstract * | 1-15 | |
| A | WO 2009/058812 A1 (GENENTECH INC [US]; LEBRETON BENEDICTE ANDREE [US] ET AL.) 7 May 2009 (2009-05-07)<br>* figures 2a, b; sequences 11, 12 * | 1-15 | |
| Y | XIN CHEN ET AL: "Fc?R-Binding Is an Important Functional Attribute for Immune Checkpoint Antibodies in Cancer Immunotherapy", FRONTIERS IN IMMUNOLOGY, vol. 10, 1 January 2019 (2019-01-01), page 292, XP055672861, DOI: 10.3389/fimmu.2019.00292<br>* page 2, left-hand column, paragraph 2 *<br>* page 3, right-hand column, paragraph 2 * | 1-15 | TECHNICAL FIELDS SEARCHED (IPC)<br>C07K |

The present search report has been drawn up for all claims

| Place of search | Date of completion of the search | Examiner |
|---|---|---|
| The Hague | 19 May 2026 | Pflug, Alexander |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 25 22 2544

This annex lists the patent family members relating to the patent documents cited in the above-mentioned European search report. The members are as contained in the European Patent Office EDP file on
The European Patent Office is in no way liable for these particulars which are merely given for the purpose of information.

19-05-2026

| Patent document cited in search report | Publication date | Patent family member(s) | Publication date |
|---|---|---|---|
| CN 109053895 A | 21-12-2018 | AU 2019332708 A1 | 15-04-2021 |
| | | AU 2026201467 A1 | 19-03-2026 |
| | | BR 112021003559 A2 | 25-05-2021 |
| | | CA 3110749 A1 | 05-03-2020 |
| | | CN 109053895 A | 21-12-2018 |
| | | DK 3882275 T5 | 28-10-2024 |
| | | EA 202190535 A1 | 20-07-2021 |
| | | EP 3882275 A1 | 22-09-2021 |
| | | EP 4461746 A2 | 13-11-2024 |
| | | ES 2989584 T3 | 27-11-2024 |
| | | FI 3882275 T3 | 25-10-2024 |
| | | HR P20241417 T1 | 03-01-2025 |
| | | HU E068809 T2 | 28-01-2025 |
| | | IL 281116 A | 29-04-2021 |
| | | JP 7374995 B2 | 07-11-2023 |
| | | JP 7589314 B2 | 25-11-2024 |
| | | JP 2021536242 A | 27-12-2021 |
| | | JP 2024023186 A | 21-02-2024 |
| | | JP 2025037880 A | 18-03-2025 |
| | | KR 20210053912 A | 12-05-2021 |
| | | KR 20260036304 A | 16-03-2026 |
| | | LT 3882275 T | 25-10-2024 |
| | | PH 12021550407 A1 | 20-09-2021 |
| | | PL 3882275 T3 | 10-02-2025 |
| | | PT 3882275 T | 15-10-2024 |
| | | RS 66073 B1 | 29-11-2024 |
| | | SG 11202101985T A | 29-04-2021 |
| | | SI 3882275 T1 | 30-04-2025 |
| | | US 2021340239 A1 | 04-11-2021 |
| | | US 2023340097 A1 | 26-10-2023 |
| | | US 2025197487 A1 | 19-06-2025 |
| | | WO 2020043184 A1 | 05-03-2020 |
| | | ZA 202101894 B | 31-08-2022 |
| WO 2009058812 A1 | 07-05-2009 | AR 069097 A1 | 30-12-2009 |
| | | AR 111171 A2 | 12-06-2019 |
| | | AU 2008318865 A1 | 07-05-2009 |
| | | BR PI0817182 A2 | 17-03-2015 |
| | | CA 2703279 A1 | 07-05-2009 |
| | | CL 2008003218 A1 | 06-03-2009 |
| | | CN 101889025 A | 17-11-2010 |
| | | CN 103554215 A | 05-02-2014 |
| | | CN 105315323 A | 10-02-2016 |
| | | CO 6280422 A2 | 20-05-2011 |
| | | CY 1116129 T1 | 08-02-2017 |
| | | DK 2215117 T3 | 02-03-2015 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 25 22 2544

This annex lists the patent family members relating to the patent documents cited in the above-mentioned European search report. The members are as contained in the European Patent Office EDP file on
The European Patent Office is in no way liable for these particulars which are merely given for the purpose of information.

19-05-2026

| Patent document cited in search report | Publication date | Patent family member(s) | Publication date |
|---|---|---|---|
| | | DK 2565206 T3 | 06-06-2016 |
| | | DK 2840090 T3 | 23-04-2018 |
| | | EP 2215117 A1 | 11-08-2010 |
| | | EP 2565206 A2 | 06-03-2013 |
| | | EP 2840090 A1 | 25-02-2015 |
| | | EP 3441402 A1 | 13-02-2019 |
| | | ES 2533266 T3 | 08-04-2015 |
| | | ES 2572958 T3 | 03-06-2016 |
| | | ES 2666170 T3 | 03-05-2018 |
| | | HK 1143821 A1 | 14-01-2011 |
| | | HK 1182401 A1 | 29-11-2013 |
| | | HK 1206753 A1 | 15-01-2016 |
| | | HK 1221234 A1 | 26-05-2017 |
| | | HR P20150282 T1 | 19-06-2015 |
| | | HR P20180943 T1 | 10-08-2018 |
| | | HU E024877 T2 | 29-02-2016 |
| | | HU E027668 T2 | 28-11-2016 |
| | | HU E037409 T2 | 28-08-2018 |
| | | JP 5237382 B2 | 17-07-2013 |
| | | JP 5885864 B2 | 16-03-2016 |
| | | JP 2011502161 A | 20-01-2011 |
| | | JP 2013173747 A | 05-09-2013 |
| | | JP 2015145371 A | 13-08-2015 |
| | | JP 2015155406 A | 27-08-2015 |
| | | JP 2017019790 A | 26-01-2017 |
| | | KR 20100086015 A | 29-07-2010 |
| | | KR 20140015166 A | 06-02-2014 |
| | | KR 20150008503 A | 22-01-2015 |
| | | ME 02101 B | 20-10-2015 |
| | | NZ 584839 A | 27-07-2012 |
| | | PE 20091434 A1 | 17-10-2009 |
| | | PH 12013501128 A1 | 02-12-2015 |
| | | PL 2215117 T3 | 30-06-2015 |
| | | PL 2565206 T3 | 31-08-2017 |
| | | PL 2840090 T3 | 31-07-2018 |
| | | PT 2215117 E | 01-04-2015 |
| | | RU 2010121816 A | 10-12-2011 |
| | | SG 175597 A1 | 28-11-2011 |
| | | SG 10201401690X A | 27-06-2014 |
| | | SI 2215117 T1 | 31-03-2015 |
| | | SI 2840090 T1 | 31-05-2018 |
| | | TW 200927289 A | 01-07-2009 |
| | | TW 201512216 A | 01-04-2015 |
| | | US 2009148435 A1 | 11-06-2009 |
| | | US 2015056196 A1 | 26-02-2015 |
| | | US 2018118781 A1 | 03-05-2018 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO. EP 25 22 2544

This annex lists the patent family members relating to the patent documents cited in the above-mentioned European search report. The members are as contained in the European Patent Office EDP file on
The European Patent Office is in no way liable for these particulars which are merely given for the purpose of information.

19-05-2026

| Patent document cited in search report | Publication date | Patent family member(s) | Publication date |
|---|---|---|---|
| | | WO 2009058812 A1 | 07-05-2009 |
| | | ZA 201002850 B | 27-07-2011 |
| | | ZA 201102169 B | 27-12-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5747654 A
- US 4816567 A
- CN 1259962 A
- CN 106967172 A


### Non-patent literature cited in the description


- **MA LI**. *Chinese Journal of Birth Health and Heredity*, 2016, vol. 24 (5), 146-148
- **ROSKOSKI R JR. et al.** *Crit Rev Oncol Hematol*, 2007, vol. 62 (3), 179-213
- **TAKAHASHI T et al.** *Oncogene*, 1999, vol. 18 (13), 2221-2230
- **DONG HONGCHAO et al.** *Journal of Modern Oncology, Sep.*, 2014, vol. 22 (9), 2231-3
- **HOMET M. B.** ; **PARISI G. et al.** Anti-PD-1 Therapy in Melanoma. *Semin Oncol.*, June 2015, vol. 42 (3), 466-473
- **HELD SA** ; **HEINE A et al.** Advances in immunotherapy of chronic myeloid leukemia CML. *Curr Cancer Drug Targets.*, September 2013, vol. 13 (7), 768-74
- **JOYCE F. LIU et al.** *JAMA Oncol.*, 2019, vol. 5 (12), 1731-1738
- **MANEGOLD C et al.** *J Thorac Oncol.*, February 2017, vol. 12 (2), 194-207
- **DUDEK AZ et al.** *J Clin Oncol.*, 2018, vol. 36 (4558)
- **STEIN S et al.** *J Clin Oncol*, 2018, vol. 36 (15), 4074
- **BENDELL JC et al.** Safety and efficacy of MPDL3280A (anti-PDL1) in combination with bevacizumab (bev) and/or FOLFOX in patients (pts) with metastatic colorectal cancer (mCRC). *Presented at: American Society of Clinical Oncology*
- **HOCHSTER HS et al.** Efficacy and safety of atezolizumab (atezo) and bevacizumab (bev) in a phase Ib study of microsatellite instability (MSI)-high metastatic colorectal cancer (mCRC). *Presented at: American Society of Clinical Oncology Gastrointestinal Cancers Symposium*, 19 January 2017 (673)
- **YUKINORI OZAKI et al.** A multicenter phase II study evaluating the efficacy of nivolumab plus paclitaxel plus bevacizumab triple-combination therapy as a first-line treatment in patients with HER2-negative metastatic breast cancer: WJOG9917B NEWBEAT trial [abstract. *In: Proceedings of the 2019 San Antonio Breast Cancer Symposium*
- Abstract nr PD1-03); the combination of VEGFR2 antibody and PD-1 antibody also showed good anti-tumor effects in adenocarcinoma of the stomach and gastroesophageal junction adenocarcinoma. *Cancer Res*, 2020, vol. 80 (4)
- **HERBST RS et al.** *Lancet Oncol.*, 2019, vol. 20 (8), 1109-1123
- **KRISHNANSU S. et al.** *N Engl J Med.*, 2014, vol. 370, 734-743
- **ANTONARAKIS ES et al.** *J Clin Oncol.*, 10 February 2020, vol. 38 (5), 395-405
- **JOAQUIM BELLMUNT. et al.** *N Engl J Med.*, 2017, vol. 376, 1015-1026
- **KATO K et al.** *Lancet Oncol.*, 2019, vol. 20 (11), 1506-17
- **SCHERPEREEL A et al.** *Lancet Oncol.*, 2019, vol. 20 (2), 239-253
- **MÜLLER D** ; **KONTERMANN RE**. Bispecific antibodies for cancer immunotherapy: current perspectives. *BioDrugs*, 2010, vol. 24, 89-98
- **COLOMA M. J.** ; **MORRISON S. L.** Design and production of novel tetravalent bispecific antibodies. *Nat Biotechnol.*, 1997, vol. 15, 159-163
- **MILLER B. R.** ; **DEMAREST S. J. et al.** Stability engineering of scFvs for the development of bispecific and multivalent antibodies. *Protein Eng Des Sel*, 2010, vol. 23, 549-57
- **FITZGERALD J** ; **LUGOVSKOY A**. Rational engineering of antibody therapeutics targeting multiple oncogene pathways. *MAbs*, 2011, vol. 3, 299-309
- **HOGARTH PM** ; **PIETERSZ GA**. *NATURE REVIEWS DRUG DISCOVERY*, 2012, vol. 11 (4), 311-331
- **RAJAGOPAL**. *Prot. Engin*, 1997, vol. 10, 1453-1459
- **REITER**. *Nat. Biotechnol.*, 1996, vol. 14, 1239-1245
- **REITER**. *Protein Engineering*, 1995, vol. 8, 1323-1331
- **WEBBER**. *Molecular Immunology*, 1995, vol. 32, 249-258
- **REITER**. *Immunity*, 1995, vol. 2, 281-287

- **REITER**. *JBC*, 1994, vol. 269, 18377-18331
- **REITER**. *Inter. J. of Cancer*, 1994, vol. 58, 142-149
- **REITE et al.** *Cancer Res.*, 1994, vol. 54, 2714-2718
- **EHRENMANN, FRANCOIS** ; **QUENTIN KAAS** ; **MARIE-PAULE LEFRANC**. IMGT/3Dstructure-DB and IMGT/DomainGapAlign: a database and a tool for immunoglobulins or antibodies, T cell receptors, MHC, IgSF and MhcSF. *Nucleic acids research*, 2009, D301-D307
- **KABAT**. Sequences of Proteins of Immunological Interest. National Institutes of Health, 1987
- **CHOTHIA** ; **LESK**. *J. Mol. Biol.*, 1987, vol. 196, 901-917
- **CHOTHIA et al.** *Nature*, 1989, vol. 342, 878-883
- Fundamental Immunology. Raven Press, 1989
- **WARD et al.** *Nature*, 1989, vol. 341, 544-546
- **BIRD et al.** *Science*, 1988, vol. 242, 423-426
- **HUSTON et al.** *Proc. Natl. Acad. Sci. USA*, 1988, vol. 85, 5879-5883
- **HOLLIGER et al.** *Proc. Natl. Acad. Sci. USA*, 1993, vol. 90, 6444-6448
- **ALFTHAN et al.** *Protein Eng.*, 1995, vol. 8, 725-731
- **CHOI et al.** *Eur. J. Immunol.*, 2001, vol. 31, 94-106
- **HU et al.** *Cancer Res.*, 1996, vol. 56, 3055-3061
- **KIPRIYANOV et al.** *J. Mol. Biol.*, 1999, vol. 293, 41-56
- **ROOVERS et al.** *Cancer Immunol.*, 2001
- **HOLLIGER P et al.** *Proc. Natl. Acad. Sci. USA*, 1993, vol. 90, 6444-6448
- **POLJAK R. J. et al.** *Structure*, 1994, vol. 2, 1121-1123
- **KOHLER et al.** *Nature*, 1975, vol. 256, 495
- **MORRISON et al.** *Proc. Natl. Acad. Sci. USA*, 1984, vol. 81, 6851-6855
- **JONES et al.** *Nature*, 1986, vol. 321, 522-525
- **REICHMANN et al.** *Nature*, 1988, vol. 332, 323-329
- **PRESTA**. *Curr. Op. Struct. Biol.*, 1992, vol. 2, 593-596
- **CLARK**. *mmunol. Today*, 2000, vol. 21, 397-402
- Epitope Mapping Protocols in Methods in Molecular Biology. 1996, vol. 66
- Remington’s Pharmaceutical Sciences. Mack Publishing Company, 1995
- **BARETTI M et al.** *Pharmacol Ther.*, 2018, vol. 189, 45-62
- **J. SAMBROOK et al.** Guide to Molecular Cloning Experiments. Science Press
- **ACIERNO**. *Affinity maturation increases the stability and plasticity of the Fv domain of anti-protein antibodies*
- **ACIERNO et al.** *J Mol Biol.*, 2007, vol. 374 (1), 130-46